# EUROPEAN PATENT SPECIFICATION

(11) **EP 3 599 286 B1**
(45) Date of publication and mention of the grant of the patent: **07.09.2022**
(21) Application number: 18382566.0
(22) Date of filing: 27.07.2018
(51) Int. Cl.: C12Q 1/6883

(54) **BIOMARKERS FOR DIAGNOSIS AND/OR PROGNOSIS OF FRAILTY**
BIOMARKER ZUR DIAGNOSE UND/ODER PROGNOSE VON GEBRECHLICHKEIT
BIOMARQUEURS POUR LE DIAGNOSTIC ET LE PRONOSTIC DE LA FRAGILITÉ

(43) Date of publication of application: 29.01.2020
(73) Proprietor: Administración General De La Communidad Autónoma De Euskadi, 01010 Vitoria-Gateiz (Álava) (ES)
(72) Inventor: MATHEU FERNÁNDEZ, Ander, 20014 Donostia/ San Sebastian (ES); OTAEGUI BICHOT, David, 20014 Donostia/ San Sebastian (ES); CARRASCO GARCÍA, Estefanía, 20014 Donostia/ San Sebastian (ES); ALBERRO GARITANO, Ainhoa, 20014 Donostia/ San Sebastian (ES); VERGARA MICHELTORENA, Itziar, 20014 Donostia/ San Sebastian (ES); VROTSOU, Kalliopi, 20014 Donostia/ San Sebastian (ES)
(74) Representative: Pons

(56) References cited:
- WO-A1-2011/051402
- EL ASSAR MARIAM ET AL: "Frailty Is Associated With Lower Expression of Genes Involved in Cellular Response to Stress: Results From the Toledo Study for Healthy Aging", JOURNAL OF THE AMERICAN MEDICAL DIRECTORS ASSOCIATION, ELSEVIER, NL, vol. 18, no. 8, 21 June 2017 (2017-06-21), XP085144850, ISSN: 1525-8610, DOI: 10.1016/J.JAMDA.2017.04.019
- Anonymous: "[HuGene-2_0-st] Affymetrix Human Gene 2.0 ST Array [transcript (gene) version]", , 14 February 2013 (2013-02-14), XP055522381, Retrieved from the Internet: URL:https://www.ncbi.nlm.nih.gov/geo/query /acc.cgi?acc=GPL16686 [retrieved on 2018-11-09] -& Anonymous: "Data Sheet GeneChip Human Gene 2.0 ST Array", , 1 January 2015 (2015-01-01), XP055522394, Retrieved from the Internet: URL:https://assets.thermofisher.com/TFS-As sets/LSG/brochures/hugene_2_st_datasheet.p df [retrieved on 2018-11-09]
- Anonymous: "Data Sheet: GeneChip Gene 1.0 ST Array System for Human, Mouse and Rat", , 1 January 2007 (2007-01-01), XP055062792, Retrieved from the Internet: URL:http://media.affymetrix.com/support/te chnical/datasheets/gene_1_0_st_datasheet.p df [retrieved on 2013-05-14]
- QU T ET AL: "Upregulated monocytic expression of CXC chemokine ligand 10 (CXCL-10) and its relationship with serum interleukin-6 levels in the syndrome of frailty", CYTOKINE, ACADEMIC PRESS LTD, PHILADELPHIA, PA, US, vol. 46, no. 3, 1 June 2009 (2009-06-01), pages 319-324, XP026133554, ISSN: 1043-4666, DOI: 10.1016/J.CYTO.2009.02.015 [retrieved on 2009-04-01]
- MICHELLE SHARDELL ET AL: "Plasma Klotho and Frailty in Older Adults: Findings From the InCHIANTI Study", JOURNALS OF GERONTOLOGY, SERIES A, BIOLOGICAL SCIENCES ANDMEDICAL SCIENCES, vol. 00, 19 October 2017 (2017-10-19), pages 1-6, XP055521719, US ISSN: 1079-5006, DOI: 10.1093/gerona/glx202
- IRYNA RUSANOVA ET AL: "Analysis of Plasma MicroRNAs as Predictors and Biomarkers of Aging and Frailty in Humans", OXIDATIVE MEDICINE AND CELLULAR LONGEVITY, vol. 2018, 18 July 2018 (2018-07-18), pages 1-9, XP055521704, US ISSN: 1942-0900, DOI: 10.1155/2018/7671850
- WEI-JU LEE ET AL: "Soluble ICAM-1, Independent of IL-6, Is Associated with Prevalent Frailty in Community-Dwelling Elderly Taiwanese People", PLOS ONE, vol. 11, no. 6, 16 June 2016 (2016-06-16), page e0157877, XP055521724, DOI: 10.1371/journal.pone.0157877
- JUULIA JYLHÄVÄ ET AL: "Identification of a prognostic signature for old-age mortality by integrating genome-wide transcriptomic data with the conventional predictors: the Vitality 90+ Study", BMC MEDICAL GENOMICS, BIOMED CENTRAL LTD, LONDON UK, vol. 7, no. 1, 11 September 2014 (2014-09-11), page 54, XP021198021, ISSN: 1755-8794, DOI: 10.1186/1755-8794-7-54

## Description

The present invention falls within the field of Molecular Biology and Medicine. Specifically, it relates to an *in vitro* method for the diagnosis and/or prognosis of frailty based on biomarkers. More specifically, the present invention relates to EGR1 biomarker and the uses thereof to determine whether a subject suffers from and/or is susceptible to developing frailty.

### BACKGROUND OF THE INVENTION

The ageing population is a success of public health policies and socioeconomic development, and is a challenge for society, which must ensure the well-being and social participation of the elderly population, as well as the provision of services to them, taking into consideration their social and health needs in a sustainable manner. Ageing is accompanied by a series of physiological changes that lead to a progressive loss of adaption to the demands of the environment and an increase in vulnerability. The most acute expression of the problem of the ageing population is the clinical condition of frailty.

Frailty is defined as a geriatric condition or syndrome characterized by the reduction of the functional reserve and adaptive capacity of elderly people. This deficit determines an overall deterioration of health that unfailingly progresses towards dependency. Frailty has proven to be a risk factor that is separate from serious adverse health events, such as institutionalization, hospitalization, falls, disability and dependency. Frail people are six times more likely to become dependent than people who are considered robust of the same age and sex. The prevalence of frailty varies depending on the instrument used to measure it and the field of study, but it ranges from 15 to 25% of the population over the age of 70. Unlike dependency, frailty is reversible and there is an abundance of evidence on the effectiveness of interventions based on muscle strengthening through exercise, dietary improvement or controlling polypharmacy. Therefore, preventing disability by acting on frailty is possible, given that the latter can be detected and is susceptible to intervention. Frailty is separately linked to a higher risk of mortality, dependency, falls and fractures, hospitalization and reduction of quality of life related to health.

The key element of frailty is an increase in vulnerability to low intensity stressors, caused by an alteration in multiple interrelated systems and that leads to a reduction of the homeostatic reserve and the adaptive capacity of the organism that predisposes it to adverse health events and hinders correct day-to-day development due to the loss of functional capacity that frail individuals suffer from.

The identification of frail elderly subjects is highly relevant since it enables, by means of personalized interventions that are adapted to the needs of the subjects and their families, the natural progression of frailty towards dependency to be stopped. Addressing frailty is a key focus in the prevention of dependency. Preventing the development of dependency is as important, if not more important, as addressing it, and delaying the development through early detection of frailty is more effective when it is new and reversible.

In recent years, numerous instruments have been proposed for the identification of frail subjects in various health care areas. Classical approaches for identifying frailty are based on prediction rules, such as the phenotype proposed by Fried, Tilburg frailty indicator or FRAIL tool; on clinical judgement of professionals, such as the Clinical Frailty Scale or Gerontopole Frailty Scale; and lastly, on counting deficits, such as the Frailty Index (Rodriguez-Mañas L, Fried LP. Lancet. 2015;385(9968): e7-e9). However, thus far, none of them have been systematically incorporated into standard practice in the National Health System. There are several reasons for this lack of acceptable tools in clinical environments, among which the following are noteworthy: 1) the poor validation thereof in clinical environments, wherein the prevalence of frailty is much higher than that existing in the population-based studies, 2) the dichotomous characterization (frail vs not frail) of the population, which makes it impossible to monitor and follow-up with the subjects, and 3) the excessive time required by some of them.

In recent years, there have also been different attempts to identify and determine specific molecular biomarkers related to the molecular mechanisms involved in the physiological deterioration that is characteristic of frailty, which entails the development of clinical features and functional deterioration. Thus, some studies have looked at different genes expressed differentially in the frail population, the majority of them being genes involved in inflammation (for example, IL-1, IL-6 and TNF-alpha), oxidative stress (for example, malondialdehyde levels and protein oxidation levels in general), and metabolism (for example, glucose, insulin, sirtuin 1 and sirtuin 3) (Calvani R, et al. SPRINTT Consortium. Aging Clin Exp Res. 2017 Feb;29(1):29-34); which affect different systems of the organism, but the information of which has not been consistently validated and there has been no transfer to clinical practice and national health systems. El Assar, M. et al. (Journal of The American Medical Directors Association, 2017, vol. 18, no. 8) discloses that frailty is associated with lower expression of genes involve in cellular response to stress which genes thus can be used as biomarkers for diagnosing frailty.

Therefore, in order to progress in the screening and diagnosis of frailty in different health care areas, functional and molecular tools, among others, that enable said screening and diagnosis to be simply, objectively and reliably carried out individually or collectively must be made available to health professionals. As such, the importance of generating and validating a tool that is easy to implement both practically and temporarily, which is non-invasive and which can even be routinely included in individuals' medical records, is increasingly necessary. The use of biomarkers through non-invasive tests that enable the fast and easy identification of frail individuals could be said tool.

### DESCRIPTION OF THE INVENTION

The identification of molecular markers of frailty in order to understand the biological base of the frailty substrate and having a simple, fast, cheap, reliable and quantifiable instrument for the suitable identification and detection of the same is currently a field of growing interest with innovative and industrial potential. In this regard, the inventors have identified a biomarker that is useful in the diagnosis and/or prognosis of frailty in a subject. Specifically, the biomarker of the invention relates to the EGR1 gene and/or to the expression product or protein thereof. The invention has beed described in the appended claims.

Therefore, a first aspect of the invention relates to the use of the expression levels of the EGR1 biomarker, hereinafter biomarker of the invention, for the *in vitro* diagnosis and/or prognosis of frailty in a subject.

In a preferred embodiment, the first aspect of the present invention further comprises the use of the expression levels of at least one of the biomarkers selected from the list consisting of: DDX11L1, hsa-miR-454 and/or any combinations thereof.

In another preferred embodiment of the first aspect of the present invention, it further comprises determining the expression levels of at least one of the biomarkers selected from the list consisting of: CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1, and/or any combinations thereof.

The present description discloses determining the expression levels of at least one of the biomarkers selected from the list consisting of: EGR1, DDX11L1, hsa-miR-454, CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, mi-R626, MTRNR2L2, RLN1, TIA1, TRAJ14, TRAJ16, TRAJ48, TRAV16, TRBV3-1, and/or any combinations thereof.

In another more preferred embodiment, the first aspect of the invention further comprises determining the expression levels of the set of biomarkers CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, mi-R626, MTRNR2L2, RLN1, TIA1, TRAJ14, TRAJ16, TRAJ48, TRAV16 and TRBV3-1.

In a preferred embodiment, determining the expression levels of the biomarkers of the invention is carried out *in vitro* in an isolated biological sample of a subject.

An "isolated biological sample" includes, but is not limited to, cells, tissues and/or biological fluids of an organism, selected from among any of the following: blood, serum, plasma, urine and/or tears, by means of any method known by a person skilled in the art. Thus, in preferred embodiment of the method of invention, the isolated biological sample of step (a) is a blood, plasma, serum or urine sample. The preferred isolated biological sample in the present invention is peripheral blood, and/or comprises peripheral blood cells (PBMCs), more preferably mononuclear cells. Thus, in another more preferred embodiment of the method of the invention, the isolated biological sample are peripheral blood mononuclear cells. These cells are isolated by, for example but not limited to, separation by density gradients using Ficoll, which is a density gradient medium based on the principle of differential migration of cells through the media during the centrifugation step of the method. Once isolated, these cells are incubated in controlled conditions, which make the subsequent determinations reliable.

As used in this document, the terms "frailty" or "frailty syndrome" are defined as a geriatric syndrome that reflects the state of reduced physiological reserves and vulnerability to stress factors related to the acceleration of aging. The physiopathology of frailty is rooted in the deregulation of multiple interrelated systems, particularly the immune, hormone and endocrine systems. Frail individuals share a series of clinical attributes that have been measured and categorized in clinical frailty scales. These attributes include, among others, involuntary weight loss, muscle weakness, slow gait speed, fatigue, low physical activity, cognitive impairment and change in mood. On the other hand, an individual that is not considered frail is a "robust" individual, or is a "dependent" individual, which is the stage before frailty.

The term "individual" or "subject", as used in the description, refers to an animal, preferably a mammal, and more preferably to a human being. The term "individual" in this specification, is a synonym for "patient", and does not intend to be limiting in any way, the former being of any age, sex or in any physical condition. The individual can be anyone, an individual that is predisposed to suffering from frailty or an individual that suffers from said illness.

Another aspect of the present invention relates to an *in vitro* method for the diagnosis and/or prognosis of frailty in an individual, hereinafter the method of the invention, which comprises:
(a) quantifying the expression levels of the EGR1 biomarker in an isolated biological sample of said individual, and
(b) comparing the expression levels obtained in step (a) with reference values, wherein an increase in expression levels of the EGR1 biomarker in the sample of the patient with respect to the reference value for said biomarker, is indicative of suffering or being predisposed to suffering from frailty.

In another preferred embodiment of the method of the invention, it further comprises quantifying in step (a) the expression levels of at least one of the biomarkers selected from the list consisting of: DDX11L1, miR-454 and/or any combinations thereof, preferably the set of DDX11L1 and miR-454 biomarkers, wherein a reduction in the expression levels of the DDX11L1 and miR-454 biomarkers, in the sample of the patient with respect to the reference values for said biomarkers, is indicative of suffering or being predisposed to suffering from frailty.

of the present disclosure discloses a method characterized in that it comprises determining the expression levels of at least one of the biomarkers selected from the list consisting of EGR1, DDX11L, miR-454, CISH, DDX11L10, LOC101929775, LOC644172, NSF, TRAJ17, TRAJ19, TRAV8-3 and/or any combinations thereof, preferably, determining the expression levels of the biomarkers CISH, DDX11L10, LOC101929775, LOC644172, NSF, TRAJ17, TRAJ19 and TRAV8-3.

In another preferred embodiment, the method of the invention further comprises quantifying in step (a) the expression levels of at least one of the biomarkers selected from the list consisting of: CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, mi-R626, MTRNR2L2, RLN1, TIA1, TRAJ14, TRAJ16, TRAJ48, TRAV16, TRBV3-1, and/or any combinations thereof, preferably, the set of biomarkers CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, mi-R626, MTRNR2L2, RLN1, TIA1, TRAJ14, TRAJ16, TRAJ48, TRAV16 and TRBV3-1.

The expression levels of all the biomarkers can be simultaneously quantified, or any of said biomarkers, or any combinations thereof, can be independently chosen.

"Diagnosis" is understood as the method through which an illness or disease, preferably frailty, is identified. For the purpose of the present invention, the term diagnosis refers to the capacity to differentiate between individuals affected or not by frailty or frailty syndrome. The term "prognosis" refers to the method by means of which a prediction of what will happen in the development or course of an illness, preferably frailty or frailty syndrome, is established. It is understood as the expected evolution of an illness and refers to the assessment of the probability according to which a subject suffers from an illness as well as the assessment of the onset thereof, state of development, evolution, or regression thereof, and/or the prognosis of the course of the illness in the future. As people skilled in the art will understand, this assessment, although it is preferred for it to be so, cannot normally be correct for 100% of the subjects that are to be diagnosed. However, the term requires a statistically significant part of the subjects to be identified as suffering from the illness or having predisposition to the same. The amount that is statistically significant can be established by a person skilled in the art by using different statistical tools, for example, but not limited to, by determining confidence intervals, determining the significant p value, Student's t test or Fisher's discriminant function, non-parametric Mann-Whitney measurements, Spearman's correlation, logistic regression, linear regression, area under the ROC curve (AUC). Preferably, the confidence intervals are at least 90%, at least 95%, at least 97%, at least 98% or at least 99%. Preferably, the p value is less than 0.1, than 0.05, than 0.01, than 0.005 or than 0.0001. Preferably, the illness can be correctly identified in a differential manner in at least 60%, more preferably in at least 70%, much more preferably in at least 80% or even more preferably in at least 90% of the subjects of a specific analyzed group or population.

The expression levels of the biomarkers, genes and/or proteins described in the present invention provide a specific expression profile. The term "expression level", also referred to as "gene product amount" or "expression product amount" refers to biochemical material, whether RNA or protein, result of the expression of a gene. Sometimes a measurement of the gene product amount is used to deduce how active a gene is. "Gene expression profile" is understood as the gene profile obtained after the quantification of the mRNA and/or protein produced by the genes of interest or biomarkers described in the present invention, in an isolated biological sample. The expression profile of the biomarkers is preferably carried out by determining the level of mRNA derived from the transcription thereof, before the extraction of the total RNA present in the isolated biological sample, which can be carried out by means of protocols known in the state of the art. The detection of the expression product amount of the biomarkers of the present invention can be carried out by any means known in the state of the art. The authors of the present invention have shown that the detection of the amount or concentration of antibodies compared to these biomarkers in a semi-quantitative or quantitative manner enables the diagnosis and/or prognosis of frailty in a subject. The measurement of the amount or concentration of the expression product of these genes, preferably in a semi-quantitative or quantitative manner, can be carried out directly or indirectly. The direct measurement relates to the measurement of the amount or concentration of the expression product of the genes, based on a signal that is obtained directly from the transcripts of said genes, or the proteins, and which is directly correlated to the number of RNA or protein molecules produced by the genes. Said signal, which can also be referred to as intensity signal, can be obtained, for example, by measuring an intensity value of a chemical or physical property of said products. The indirect measurement includes the measurement obtained from a second component or a biological measurement system (for example, the measurement of cellular responses, ligands, "labels" or enzyme reaction product).

Determining the level of mRNA derived from the transcription of the genes used as biological markers in the present invention, can be carried out by, for example, although not limited to, amplification by polymerase chain reaction (PCR), reverse transcription in combination with polymerase chain reaction (RT-PCR), quantitative RT-PCR, reverse transcription in combination with ligase chain reaction (RT-LCR), or any other nucleic acid amplification method; serial analysis of gene expression (SAGE, SuperSAGE); DNA microarrays prepared with oligonucleotides deposited by any mechanism; DNA microarrays prepared with oligonucleotides synthesized in situ by means of photolithography or by any other mechanism; in situ hybridization by using specific probes marked with any labelling method; by means of electrophoresis gels; by means of membrane transfer and hybridization with a specific probe; by nuclear magnetic resonance or any other diagnostic imaging technique using paramagnetic diagnostic nanoparticles or any other type of detectable nanoparticles functionalized with antibodies or by any other means. The quantitative detection of the expression of the genes used as biological markers in the present invention can be carried out more preferably by real-time PCR (RT-PCR or RTqPCR). Real-time detection of the amplified products can be carried out through the use of fluorescent molecules that are interspersed in the doublestranded DNA or through hybridization with different types of probes.

In another preferred embodiment, the gene expression profile could be obtained by means of the detection and/or quantification of the proteins product of the mRNA translation derived from the transcription of genes used as biological markers in the present invention, for example by means of, but not limited to, immunodetection by western blot, cytometry, radioimmunoassay, and radioimmunometric techniques, ELISA (Enzyme Linked ImmunoadSorbent Assay), or any combination thereof.

The term "comparison", as used in the description, refers, but is not limited to, the comparison of the expression levels of the biomarkers of the invention in the biological sample to be analyzed, also called problem biological sample, with the expression levels of one or more reference samples.

The terms "reference value" or "reference amount" as used in the description, refers to the absolute or relative amount of gene and/or protein expression level of the biomarkers of the invention, which enable the individuals suffering from or who will suffer from the illness, that is frail and/or dependent individuals, to be differentiated from individuals that do not suffer or will not suffer from the illness, that is, robust individuals. Said values or amounts can be determined by the method of the present invention from a reference sample that can be analyzed, for example, simultaneously or consecutively, along with the problem biological sample. As such, for example but in a non-limiting manner, the reference sample can be the negative controls, that is, the amounts detected by the method of the invention in samples of individuals that do not suffer from the illness, frailty, that is, robust individuals. Thus, in a preferred embodiment of the method of the invention, the reference value corresponds to expression levels of the biomarkers in an isolated biological sample of robust individuals.

The comparison described in section (b) of the method of the present invention can be manual or computer-assisted.

The terms "polynucleotide" and "nucleic acid" are used interchangeably here, referring to polymeric forms of nucleotides of any length, both ribonucleotides (RNA) and deoxyribonucleotides (DNA). The terms "amino acid sequence", "peptide", "oligopeptide", "polypeptide" and "protein" are used interchangeably here, and refer to a polymeric form of amino acids of any length, which can be coding or noncoding, chemically or biochemically modified.

The characteristics of the biomarkers object of the present invention are described below:
In the context of the present invention, EGR1 (*Early growth response protein 1),* also known as Zif268 (*zinc finger protein 225*), NGFI-A (*nerve growth factor-induced protein A*), AT225, G0S30, KROX-24, TIS8, ZNF225, *Transcription Factor* ETR103, TIS8, is also defined by a nucleotide or polynucleotide sequence in SEQ ID NO: 1 with Gene ID: 1958 in NCBI, which constitutes the coding sequence of the protein in SEQ ID NO: 2 with access number in the UniProt database: P18146, and which would comprise different variants from: a) nucleic acid molecules that code a polypeptide which comprises the amino acid sequence of SEQ ID NO: 2, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 2, and in which the polypeptide encoded by said nucleic acids exhibits the activity and structural characteristics of the EGR1 protein. Among said nucleic acid molecules, is that included in SEQ ID NO: 1.

In the context of the present invention, DDX11L1 or *DEAD*/*H-box helicase 11 like 1,* is also defined by a sequence of nucleotides in SEQ ID NO:3 with Gene ID: 100287102 in NCBI, which constitutes the coding sequence of the protein in SEQ ID NO: 4, with the access number in the UniProt database: B7ZGX0, and that would comprise different variants from: a) nucleic acid molecules that code a polypeptide which comprises the amino acid sequence of SEQ ID NO: 4, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 4, and in which the polypeptide encoded by said nucleic acids exhibits the activity and structural characteristics of the DDX11L1 protein. Among said nucleic acid molecules, is that included in SEQ ID NO: 3.

In the context of the present invention, miR-454 or MicroRNA 454, Hsa-miR-454 is also defined by a nucleotide or polynucleotide sequence in SEQ ID NO: 5, with Gene ID: 768216 in NCBI.

In the context of the present invention, CISH (*Cytokine-inducible SH2-containing protein),* also known as BACTS2, CIS, CIS-1, G18 or SOCS, is also defined by a nucleotide or polynucleotide sequence in SEQ ID NO: 6 with Gene ID: 1154 in NCBI, which constitutes the coding sequence of the protein in SEQ ID NO: 7, with access number of the UniProt database: Q9NSE2, and which comprises different variants from: a) nucleic acid molecules that code a polypeptide which comprises the amino acid sequence of SEQ ID NO: 7, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 7, and in which the polypeptide encoded by said nucleic acids exhibits the activity and structural characteristics of the CISH protein. Among said nucleic acid molecules, is that included in SEQ ID NO: 6.

In the context of the present invention, DDX11L10 (*DEAD*/*H-box helicase 11 like 10),* also known as DDX11L1 or DDX11P, is also defined by a nucleotide or polynucleotide sequence in SEQ ID NO: 8, with Gene ID: 100287029 in NCBI, which constitutes the coding sequence of the protein in SEQ ID NO: 9, with the access number in the UniProt database: B7ZGX5, and that would comprise different variants from: a) nucleic acid molecules that code a polypeptide which comprises the amino acid sequence of SEQ ID NO: 9, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 9, and in which the polypeptide encoded by said nucleic acids exhibits the activity and structural characteristics of the DDX11L10 protein. Among said nucleic acid molecules, is that included in SEQ ID NO: 8.

In the context of the present invention, LOC101929775, also known as *C-Jun-amino-terminal kinase-interacting protein 1-lik*e, is also defined by a nucleotide or polynucleotide sequence in SEQ ID NO: 10, with Gene ID: 101929775 in NCBI.

In the context of the present invention, LOC644172 also known as MAPK8IP1P2 (*Mitogen-Activated Protein Kinase 8 Interacting Protein 1 Pseudogene 2),* is also defined by a nucleotide or polynucleotide sequence in SEQ ID NO: 11, with Gene ID: 644172 in NCBI.

In the context of the present invention, NSF (-*N-ethylmaleimide-sensitive factor*), also known as SKD2 or SEC18, is also defined by a nucleotide or polynucleotide sequence in SEQ ID NO: 12, with Gene ID: 4905 in NCBI, which constitutes the coding sequence of the protein in SEQ ID NO: 13, with the access number in the UniProt database: Q96D47, and that would comprise different variants from: a) nucleic acid molecules that code a polypeptide which comprises the amino acid sequence of SEQ ID NO: 13, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 13, and in which the polypeptide encoded by said nucleic acids exhibits the activity and structural characteristics of the NSF protein. Among said nucleic acid molecules, is that included in SEQ ID NO: 12.

In the context of the present invention, TRAJ17 (*T-cell receptor alpha joining 17*), is also defined by a nucleotide sequence in SEQ ID NO: 14, with Gene ID: 28738 in NCBI, which constitutes the coding sequence of the protein in SEQ ID NO: 15, with the access number in the UniProt database: A0A075B6W8, and that would comprise different variants from: a) nucleic acid molecules that code a polypeptide which comprises the amino acid sequence of SEQ ID NO: 15, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 15, and in which the polypeptide encoded by said nucleic acids exhibits the activity and structural characteristics of the TRAJ17 protein. Among said nucleic acid molecules, is that included in SEQ ID NO: 14.

In the context of the present invention, TRAJ19 (*T cell receptor alpha joining 19 (non-functional)),* is also defined by a nucleotide sequence in SEQ ID NO: 16, with Gene ID: 28736 in NCBI, which constitutes the coding sequence of the protein in SEQ ID NO: 17, with the access number in the UniProt database: A0A075B6Y4, and that would comprise different variants from: a) nucleic acid molecules that code a polypeptide which comprises the amino acid sequence of SEQ ID NO: 17, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 17, and in which the polypeptide encoded by said nucleic acids exhibits the activity and structural characteristics of the TRAJ19 protein. Among said nucleic acid molecules, is that included in SEQ ID NO: 16.

In the context of the present invention, TRAV8-3 (*T cell receptor alpha variable 8-3*)*,* also known as TCRAV1S4, TCRAV8S3 or TRAV83, is also defined by a nucleotide sequence in SEQ ID NO: 18, with Gene ID: 28683 in NCBI, which constitutes the coding sequence of the protein in SEQ ID NO: 19, with the access number in the UniProt database: A0A0A6YYJ7, and that would comprise different variants from: a) nucleic acid molecules that code a polypeptide which comprises the amino acid sequence of SEQ ID NO: 19, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 19, and in which the polypeptide encoded by said nucleic acids exhibits the activity and structural characteristics of the TRAV8-3 protein. Among said nucleic acid molecules, is that included in SEQ ID NO: 18.

In the context of the present invention, CD40L (Ligand CD40), also known as IGM; IMD3; TRAP; gp39; CD154; HIGM1; T-BAM; TNFSF5 and hCD40L, is also defined by a nucleotide sequence in SEQ ID NO: 20, with Gene ID: 959 in NCBI, which constitutes the coding sequence of the protein in SEQ ID NO: 21, with the access number in the UniProt database: Q45QX2, and that would comprise different variants from: a) nucleic acid molecules that code a polypeptide which comprises the amino acid sequence of SEQ ID NO: 21, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 21, and in which the polypeptide encoded by said nucleic acids exhibits the activity and structural characteristics of the CD40L protein. Among said nucleic acid molecules, is that included in SEQ ID NO: 20.

In the context of the present invention, CLDN12 (*Claudin* 12), is also defined by a nucleotide sequence in SEQ ID NO: 22, with Gene ID: 9069 in NCBI, which constitutes the coding sequence of the protein in SEQ ID NO: 23, with the access number in the UniProt database: P56749, and that would comprise different variants from: a) nucleic acid molecules that code a polypeptide which comprises the amino acid sequence of SEQ ID NO: 23, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 23, and in which the polypeptide encoded by said nucleic acids exhibits the activity and structural characteristics of the CLDN12 protein. Among said nucleic acid molecules, is that included in SEQ ID NO: 22.

In the context of the present invention, CNTNAP3 (*Contactin Associated Protein-Like* 3)*,* also known as CASPR3; CNTNAP3A, is also defined by a nucleotide sequence in SEQ ID NO: 24, with Gene ID: 79937 in NCBI, which constitutes the coding sequence of the protein in SEQ ID NO: 25, with the access number in the UniProt database: Q9BZ76, and that would comprise different variants from: a) nucleic acid molecules that code a polypeptide which comprises the amino acid sequence of SEQ ID NO: 25, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 25, and in which the polypeptide encoded by said nucleic acids exhibits the activity and structural characteristics of the CNTNAP3 protein. Among said nucleic acid molecules, is that included in SEQ ID NO: 24.

In the context of the present invention, CNTNAP3B (*Contactin Associated Protein-Like 3B*), also known as CASPR3B, is also defined by a nucleotide sequence in SEQ ID NO: 26, with Gene ID: 728577 in NCBI, which constitutes the coding sequence of the protein in SEQ ID NO: 27, with the access number in the UniProt database: Q96NU0, and that would comprise different variants from: a) nucleic acid molecules that code a polypeptide which comprises the amino acid sequence of SEQ ID NO: 27, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 27, and in which the polypeptide encoded by said nucleic acids exhibits the activity and structural characteristics of the CNTNAP3B protein. Among said nucleic acid molecules, is that included in SEQ ID NO: 26.

In the context of the present invention, CSRNP1 (*Cysteine and Serine Rich Nuclear Protein* 1), also known as AXUD1; URAX1; TAIP-3; CSRNP-1 and FAM130B, is also defined by a nucleotide sequence in SEQ ID NO: 28, with Gene ID: 64651 in NCBI, which constitutes the coding sequence of the protein in SEQ ID NO: 29, with the access number in the UniProt database: Q96S65, and that would comprise different variants from: a) nucleic acid molecules that code a polypeptide which comprises the amino acid sequence of SEQ ID NO: 29, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 29, and in which the polypeptide encoded by said nucleic acids exhibits the activity and structural characteristics of the CSRNP1 protein. Among said nucleic acid molecules, is that included in SEQ ID NO: 28.

In the context of the present invention, CTSLP8 (*Cathepsin L Pseudogene 8*), also known as CTSL1P8, is also defined by a nucleotide or polynucleotide sequence in SEQ ID NO: 30, with Gene ID: 1518 in NCBI.

In the context of the present invention, CXCL8 (*C-X-C motif chemokine ligand 8*), also known as IL8; NAF; GCP1; LECT; LUCT; NAP1; GCP-1; LYNAP; MDNCF; MONAP and NAP-1, is also defined by a nucleotide sequence in SEQ ID NO: 31, with Gene ID: 3576 in NCBI, which constitutes the coding sequence of the protein in SEQ ID NO: 32, with the access number in the UniProt database: P10145, and that would comprise different variants from: a) nucleic acid molecules that code a polypeptide which comprises the amino acid sequence of SEQ ID NO: 32, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 32, and in which the polypeptide encoded by said nucleic acids exhibits the activity and structural characteristics of the CXCL8 protein. Among said nucleic acid molecules, is that included in SEQ ID NO: 31.

In the context of the present invention, G0S2 (*G0*/*G1 Switch 2),* is also defined by a nucleotide sequence in SEQ ID NO: 33, with Gene ID: 50486 in NCBI, which constitutes the coding sequence of the protein in SEQ ID NO: 34, with the access number in the UniProt database: P27469, and that would comprise different variants from: a) nucleic acid molecules that code a polypeptide which comprises the amino acid sequence of SEQ ID NO: 34, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 34, and in which the polypeptide encoded by said nucleic acids exhibits the activity and structural characteristics of the G0S2 protein. Among said nucleic acid molecules, is that included in SEQ ID NO: 33.

In the context of the present invention, GCNT4 (*Glucosaminyl (N-acetyl) Transferase 4, Core 2),* also known as C2GNT3, is also defined by a nucleotide sequence in SEQ ID NO: 35, with Gene ID: 51301 in NCBI, which constitutes the coding sequence of the protein in SEQ ID NO: 36, with the access number in the UniProt database: Q9P109, and that would comprise different variants from: a) nucleic acid molecules that code a polypeptide which comprises the amino acid sequence of SEQ ID NO: 36, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 36, and in which the polypeptide encoded by said nucleic acids exhibits the activity and structural characteristics of the GCNT4 protein. Among said nucleic acid molecules, is that included in SEQ ID NO: 35.

In the context of the present invention, GJB6 (*Gap Junction Protein beta 6*), also known as ED2; EDH; HED; CX30; HED2; DFNA3; ECTD2; DFNA3B and DFNB1B, is also defined by a nucleotide sequence in SEQ ID NO: 37, with Gene ID: 10804 in NCBI, which constitutes the coding sequence of the protein in SEQ ID NO: 38, with the access number in the UniProt database: O95452, and that would comprise different variants from: a) nucleic acid molecules that code a polypeptide which comprises the amino acid sequence of SEQ ID NO: 38, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 38, and in which the polypeptide encoded by said nucleic acids exhibits the activity and structural characteristics of the GJB6 protein. Among said nucleic acid molecules, is that included in SEQ ID NO: 37.

In the context of the present invention, IGHV2-26 (*Immunoglobulin Heavy Variable 2-26)*, also known as VH and IGHV226, is also defined by a nucleotide sequence in SEQ ID NO: 39, with Gene ID: 28455 in NCBI, which constitutes the coding sequence of the protein in SEQ ID NO: 40, with the access number in the UniProt database: A0A0B4J1V2, and that would comprise different variants from: a) nucleic acid molecules that code a polypeptide which comprises the amino acid sequence of SEQ ID NO: 40, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 40, and in which the polypeptide encoded by said nucleic acids exhibits the activity and structural characteristics of the IGHV2-26 protein. Among said nucleic acid molecules, is that included in SEQ ID NO: 39.

In the context of the present invention, LOC100505530 is also defined by a nucleotide or polynucleotide sequence in SEQ ID NO: 41, with Gene ID: 100505530 in NCBI.

In the context of the present invention, LOC105378916 is also defined by a nucleotide or polynucleotide sequence in SEQ ID NO: 42, with Gene ID: 105378916 in NCBI.

In the context of the present invention, miR-3941, microRNA 3941, MIR3941 or Hsa-miR-3941 is also defined by a nucleotide or polynucleotide sequence in SEQ ID NO: 43, with Gene ID: 100500866 in NCBI.

In the context of the present invention, miR-487A, microRNA 487A, MIR487A or Hsa-miR-487A is also defined by a nucleotide or polynucleotide sequence in SEQ ID NO: 44, with Gene ID: 619555 in NCBI.

In the context of the present invention, miR-626, microRNA 626, MIR626 or Hsa-miR-626 is also defined by a nucleotide or polynucleotide sequence in SEQ ID NO: 45, with Gene ID: 693211 in NCBI.

In the context of the present invention, MTRNR2L2 (*MT-RNR2-iike 2),* also known as HN2, is also defined by a nucleotide or polynucleotide sequence in SEQ ID NO: 46, with Gene ID: 100462981 in NCBI, which constitutes the coding sequence of the protein in SEQ ID NO: 47, with the access number in the UniProt database: P0CJ69, and that would comprise different variants from: a) nucleic acid molecules that code a polypeptide which comprises the amino acid sequence of SEQ ID NO: 47, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 47, and in which the polypeptide encoded by said nucleic acids exhibits the activity and structural characteristics of the MTRNR2L2 protein. Among said nucleic acid molecules, is that included in SEQ ID NO: 46.

In the context of the present invention, RLN1 (*Relaxin 1),* also known as H1; H1RLX; RLXH1; bA12D24.3.1 and bA12D24.3.2, is also defined by a nucleotide or polynucleotide sequence in SEQ ID NO: 48, with Gene ID: 6013 in NCBI, which constitutes the coding sequence of the protein in SEQ ID NO: 49, with the access number in the UniProt database: P04808, and that would comprise different variants from: a) nucleic acid molecules that code a polypeptide which comprises the amino acid sequence of SEQ ID NO: 49, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 47, and in which the polypeptide encoded by said nucleic acids exhibits the activity and structural characteristics of the RNL1 protein. Among said nucleic acid molecules, is that included in SEQ ID NO: 48.

In the context of the present invention, TIA1 (*TIA1 cytotoxic granule associated RNA binding protein),* also known as WDM and TIA-1, is also defined by a nucleotide or polynucleotide sequence in SEQ ID NO: 50, with Gene ID: 7072 in NCBI, which constitutes the coding sequence of the protein in SEQ ID NO: 51, with the access number in the UniProt database: P31483, and that would comprise different variants from: a) nucleic acid molecules that code a polypeptide which comprises the amino acid sequence of SEQ ID NO: 51, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 51, and in which the polypeptide encoded by said nucleic acids exhibits the activity and structural characteristics of the TIA1 protein. Among said nucleic acid molecules, is that included in SEQ ID NO: 50.

In the context of the present invention, TRAJ14 *(T cell receptor alpha joining 14),* is also defined by a nucleotide or polynucleotide sequence in SEQ ID NO: 52, with Gene ID: 28741 in NCBI, which constitutes the coding sequence of the protein in SEQ ID NO: 53, with the access number in the UniProt database: A0N4Z3, and that would comprise different variants from: a) nucleic acid molecules that code a polypeptide which comprises the amino acid sequence of SEQ ID NO: 53, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 53, and in which the polypeptide encoded by said nucleic acids exhibits the activity and structural characteristics of the TRAJ14 protein. Among said nucleic acid molecules, is that included in SEQ ID NO: 52.

In the context of the present invention, TRAJ16 (*T cell receptor alpha joining 16),* is also defined by a nucleotide or polynucleotide sequence in SEQ ID NO: 54, with Gene ID: 28739 in NCBI, which constitutes the coding sequence of the protein in SEQ ID NO: 55, with the access number in the UniProt database: A0A75B6V0, and that would comprise different variants from: a) nucleic acid molecules that code a polypeptide which comprises the amino acid sequence of SEQ ID NO: 55, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 55, and in which the polypeptide encoded by said nucleic acids exhibits the activity and structural characteristics of the TRAJ16 protein. Among said nucleic acid molecules, is that included in SEQ ID NO: 54.

In the context of the present invention, TRAJ48 *(T cell receptor alpha joining 48),* is also defined by a nucleotide or polynucleotide sequence in SEQ ID NO: 56, with Gene ID: 28707 in NCBI, which constitutes the coding sequence of the protein in SEQ ID NO: 57, with the access number in the UniProt database: A0A075B6V3, and that would comprise different variants from: a) nucleic acid molecules that code a polypeptide which comprises the amino acid sequence of SEQ ID NO: 57, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 57, and in which the polypeptide encoded by said nucleic acids exhibits the activity and structural characteristics of the TRAJ48 protein. Among said nucleic acid molecules, is that included in SEQ ID NO: 56.

In the context of the present invention, TRAV16 *(T cell receptor alpha variable 16),* also known as TCRAV9S1 and TCRAV16S1, is also defined by a nucleotide or polynucleotide sequence in SEQ ID NO: 58, with Gene ID: 28667 in NCBI, which constitutes the coding sequence of the protein in SEQ ID NO: 59, with the access number in the UniProt database: A0A0A6YYK6, and that would comprise different variants from: a) nucleic acid molecules that code a polypeptide which comprises the amino acid sequence of SEQ ID NO: 59, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 59, and in which the polypeptide encoded by said nucleic acids exhibits the activity and structural characteristics of the TRAV16 protein. Among said nucleic acid molecules, is that included in SEQ ID NO: 58.

In the context of the present invention, TRBV3-1 *(T cell receptor alpha variable 3-1*)*,* also known as TRBV31; TCRBV3S1 and TCRBV9S1A1T, is also defined by a nucleotide or polynucleotide sequence in SEQ ID NO: 60, with Gene ID: 28619 in NCBI, which constitutes the coding sequence of the protein in SEQ ID NO: 61, with the access number in the UniProt database: A0A576, and that would comprise different variants from: a) nucleic acid molecules that code a polypeptide which comprises the amino acid sequence of SEQ ID NO: 61, b) nucleic acid molecules whose complementary chain hybrids with the polynucleotide sequence of a), c) nucleic acid molecules whose sequence differs from a) and/or b) due to the degeneracy of the genetic code, d) nucleic acid molecules codifying a polypeptide comprising the amino acid sequence with an identity of at least 80%, 90%, 95%, 98% or 99% with the SEQ ID NO: 61, and in which the polypeptide encoded by said nucleic acids exhibits the activity and structural characteristics of the TRBV3-1 protein. Among said nucleic acid molecules, is that included in SEQ ID NO: 60.

The measurement of the activities of the biomarkers of the invention can be carried out by any means known in the state of the art. In addition, the presence of polymorphisms or mutations can be determined by DNA sequencing.

In the sense used in this description, the term "variant" refers to a protein that is substantially equivalent to any of the biomarkers of the invention. In general, a variant includes additions, deletions or substitutions of amino acids and/or nucleotides. The term "variant" also includes the proteins resulting from post-translational modifications such as, for example, but not limited to, phosphorylation, glycosylation, SUMOylation, methylation or acylation.

As used here, a biomarker is "substantially equivalent" to the biomarkers of the invention when the amino acid and/or nucleotide sequence thereof has a good alignment with the amino acid and/or nucleotides of the biomarkers of the invention, that is, when the amino acid and/or nucleotide sequence thereof has a level of identity with respect to the amino acid and/or nucleotide sequence of the biomarkers of the invention, of at least 50%, typically of at least 80%, advantageously of at least 85%, preferably of at least 90%, more preferably of at least 95%, and even more preferably of at least 99%. The sequences equivalent to the biomarkers of the invention can be easily identified by a person skilled in the art, for example, with the help of a computer program suitable for comparing sequences. The expression "functionally equivalent", as used here, means that the proteins or the fragment(s) of the protein(s) in question essentially keep(s) the biological or immunological properties described in this document. Said capacity can be determined by means of conventional methods.

The term "identity", as used in this specification, refers to the proportion of nucleotides or amino acids that are identical between two nucleotide or amino acid sequences that are compared. The methods for comparing sequences are known in the state of the art, and include, although are not limited to the same, the GAG program, including GAP (Devereuxef al., Nucleic Acids Research 12: 287 (1984) Genetics Computer Group University of Wisconsin, Madison, (WI); BLAST, BLASTP or BLASTN, and FASTA (Altschul et al., 1999. J. Mol. Biol. 215: 403-41).

The diagnosis and prognosis methods described in this document are designed to capture the relationship between the expression level of the biomarkers described in the present document and the risk of suffering from frailty. Once an individual has been diagnosed by means of the method described in this document, this individual can be treated or an intervention can be carried out in accordance with the therapeutic or intervention regime that is considered suitable depending on the status of the illness characterized by the patient.

Another aspect of the present invention relates to the use of a kit or device, hereinafter use of the kit of the invention, which comprises primers, probes and/or antibodies capable of detecting the expression levels of the EGR1 biomarker in an isolated biological sample, for the *in vitro* diagnosis and/ prognosis of frailty in an individual.

The present disclosure discloses a kit which comprises the elements necessary to quantify the expression levels of EGR1 biomarker, or a kit further comprising the elements necessary to quantify the expression levels of the DDX11L1, miR-454 biomarkers and/or any combinations thereof. More preferably, the kit comprises the elements necessary to quantify the expression levels of the EGR1, DDX11L1 and miR-454 biomarkers, even more preferably, the kit further comprises the elements necessary to quantify the expression levels of the biomarkers selected from the list consisting of: CISH, DDX11L10, LOC101929775, LOC644172, NSF, TRAJ17, TRAJ19, TRAV8-3 or any combinations thereof, or in addition, the biomarkers selected from the list consisting of: CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1, or any combinations thereof. The elements necessary to quantify the expression levels of these biomarkers are selected from the list consisting of: primers, probes and/or antibodies.

In another preferred embodiment, the use of the kit of the invention further comprises primers, probes and/or antibodies capable of detecting the expression levels of at least one of the biomarkers selected from the list consisting of: DDX11L1, miR-454, CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 and TRBV3-1, and/or any combinations thereof.

In a preferred embodiment, the isolated sample is peripheral blood, and even more preferably are peripheral blood mononuclear cells.

Said kit or device can contain all the reagents necessary to determine the biomarkers of the invention by means of any of the methods that exist in the state of the art and/or described in this document. The kit can also include, without any type of limitation, buffers, agents for preventing contamination, gene and/or protein degradation inhibitors, etc. Furthermore, the kit can include all the media and containers necessary to initiate and optimize the same. Preferably, the kit further comprises the instructions to carry out any of the methods of the invention.

The kit or device is a parts kit, which comprises a component A, formed by a device for collecting the sample of step a), and a component B, formed by the elements necessary to carry out the quantitative analysis of the sample.

Throughout the description, the word "comprises" and its variants are not intended to exclude other technical characteristics, additives, components or steps. For those skilled in the art, other objects, advantages and characteristics of the invention may be deduced from both the description and the practical use of the invention. The following examples and drawings are provided by way of illustration, and are not meant to limit the present invention.

### BRIEF DESCRIPTION OF THE FIGURES

**FIG. 1****.** Graph showing the prevalence of frailty in the sample of analyzed patients (n=26), observing a significant increase in the number of frail women with respect to men in the analyzed sample (p<0.001).
**FIG. 2****.** Data in BOX-PLOT format of the Time Up and Go test (TUG) (**A**) where a statistical significance of p<0.001 between men and women is observed; and (**B**) Gait speed (GS) where a statistical significance of p=0.711 between men and women is observed.
**FIG. 3****.** (**A**) Hierarchical grouping of the expression of biomarkers analyzed in the present invention and differentially expressed between the groups of robust and frail subjects according to all the Tests studied. (**B**) Sensitivity and specificity values of each classifier in the sample of patients analyzed. (**C**) ROC curve obtained with the set of 35 biomarkers of the invention by means of the Compound Covariate Predictor classifier.

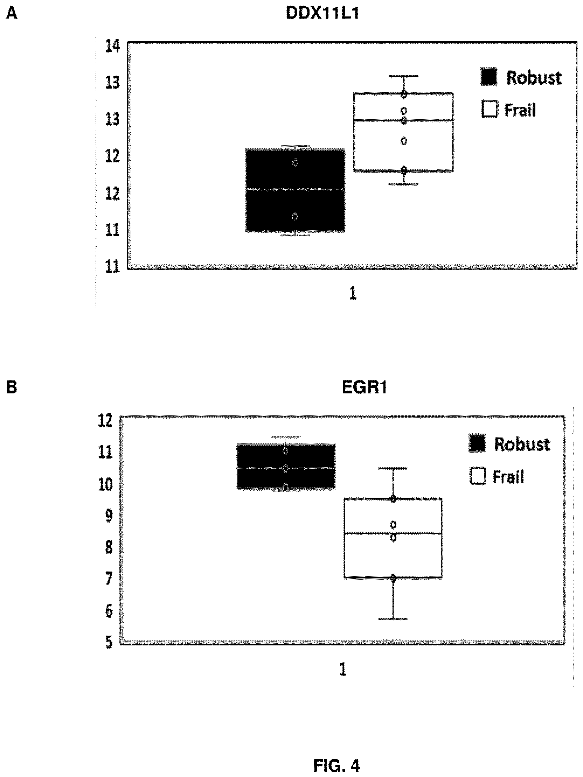

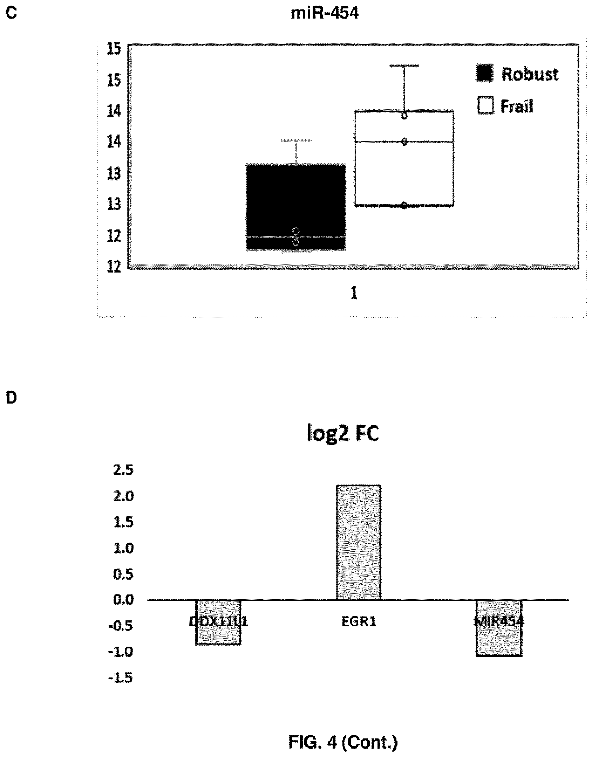

**FIG. 4****.** Analysis of the gene expression of the DDX11L1 (**A**), EGR1 (**B**) and hsa-miR-454 (**C**) biomarkers between robust individuals (black block) and frail individuals (white block) in the set of subjects of the original cohort (n=26). The cycle difference (Ct) between the biomarkers and the reference gene used as a control is shown, meaning a greater value in the number of cycles of said markers entails a lower expression of the same. (**D**) This figure shows the number of times the expression of the biomarker is higher (in the case of EGR1) or lower (in DDX11L1 and hsa-miR-454) in the frail population with respect to the robust group.
**FIG. 5****.** Analysis of the gene expression of the EGR1 biomarker in the validation population (n=182) obtained according to each of the tests indicated. The black block represents robust individuals and the white block represents frail individuals. For the Carlson test, the grey block represents dependent individuals.
**FIG. 6****.** EGR1 biomarker expression in the sub-cohort of 58 individuals where the individuals have been identified as Robust or Frail in all the tests carried out.
**FIG. 7****.** ROC curve of the EGR1 biomarker in the original cohort (n=25) (**A**) and in the validation cohort (n=58) by means of qPCR (classification ends) (**B**).
**FIG. 8****.** ROC curve of the three genes in the sub-cohort of 58 samples selected from the validation cohort (n=182), including the combination of the EGR1, DDX11L1 and hsa-miR-454 biomarkers.
**FIG 9****.** Gene expression of the EGR1, DDX11L1 and hsa-miR-454 biomarkers in cultured fibroblasts obtained from healthy subjects (robust) that have kept their robust state (robust cell) or have changed to frail by means of serial passages (frail cell).

### EXAMPLES

The invention is illustrated below by means of tests carried out by the inventors which reveal the effectiveness of the product of the invention.

### Example 1. Identification of frailty biomarkers

The Primary Care Research Unit has formed a cohort of 1000 subjects from the community of the Guipúzcoa province (Spain). Specifically, the subjects come from the OSI Bidasoa and OSI Donostialdea and were invited to participate by their reference primary care professionals and, after giving their informed consent, they were asked to a single face-to-face interview in which a proposed base assessment that includes the collection of socio-demographic and health status data (KOS), taking anthropometric measurements, the Tilburg and Barthel Test questionnaires, and functional execution tests, such as Time Up and Go and Gait Speed, were carried out. Furthermore, information regarding present comorbidity, chronic prescriptions, as well as the occurrence of falls and the use of health services was obtained based on their medical records. The results obtained confirmed that 56% of the subjects are women and the average age of the cohort is 83 years old.

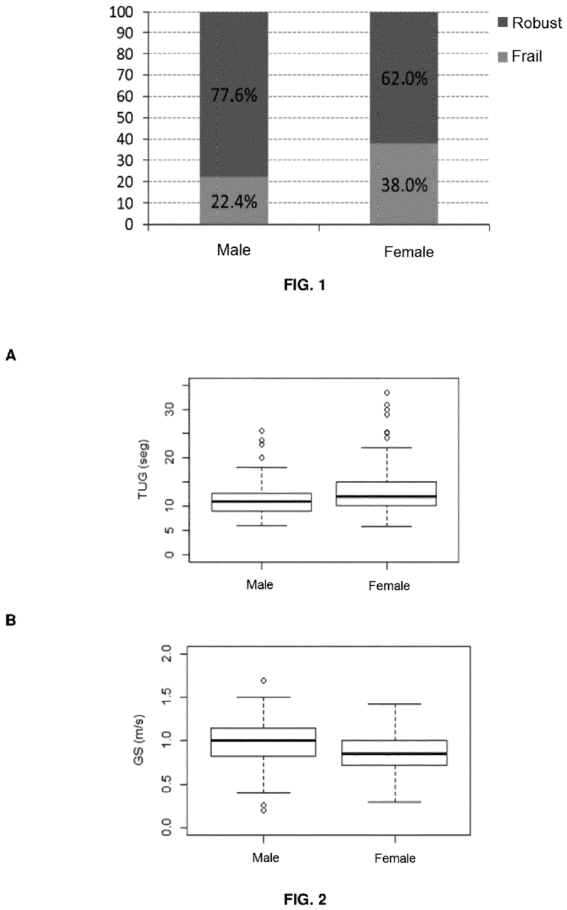

Frailty is a very prevalent condition in the sample studied, although it varies depending on the identification instrument, tool or test used. As such, considering frailty based on the Tilburg questionnaire, the prevalence is at 30%, although as observed in FIG. 1, this varies according to sex, showing 22.4% frailty for men with respect to 38.0% frailty shown for women (p<0.001). The result obtained in the functional execution tests, Time Up and Go and Gait Speed, for the sample of individuals analyzed, indicated that the percentage of frail individuals is higher (50% in TUG) than that observed with the Tilburg Test (38.46%), as can be seen in **FIG. 2**. Moreover, these studies also indicate that there is a high percentage of subjects with a risk of suffering from adverse events such as falls or hospitalizations, with significant differences when the sex is considered, especially in the case of the Time Up and Go test (**FIG. 2A**). Lastly, the Barthel Test enabled the individuals to be grouped into robust, frail and dependent.

A group of individuals described as robust and frail, according to Tilburg, were randomly selected keeping the representativeness in terms of age, sex and age range proposed for the analysis of the complete transcriptome. A total of 26 subjects were selected, whose grouping into population groups, based on the tests carried out, is detailed in **Table 1**.

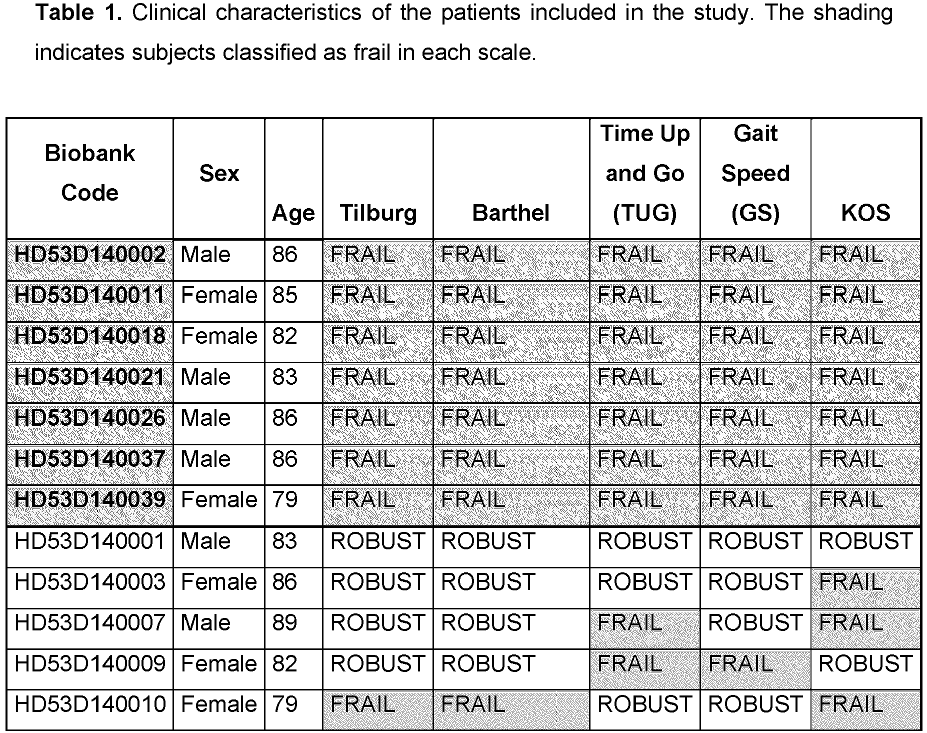

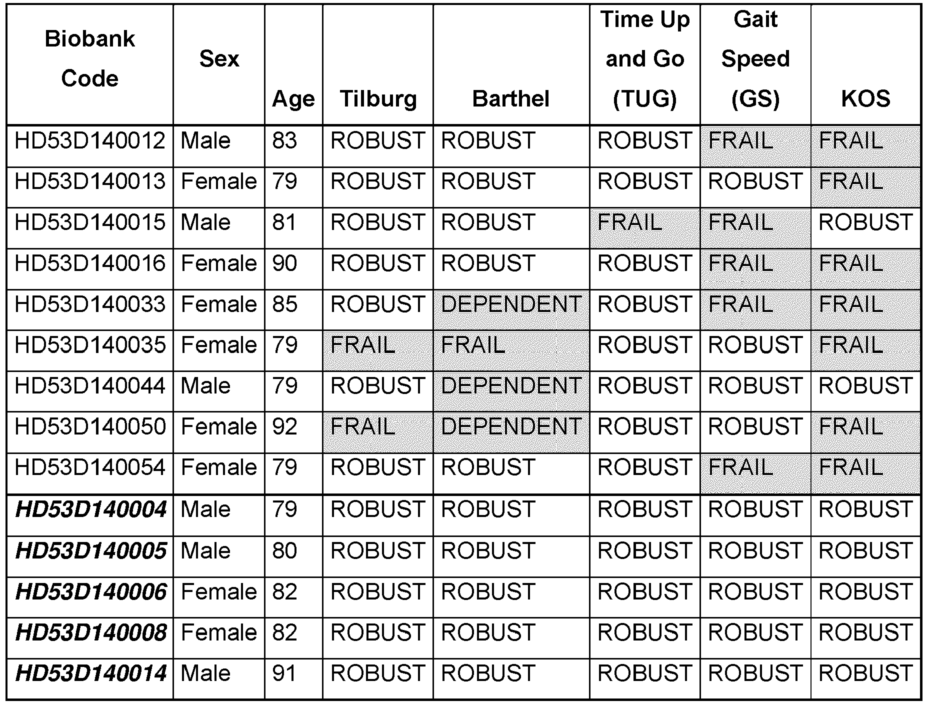

A comparison was made of the different classification tools used in the classification of the 26 subjects described in **Table 1**. The results show that the grouping into robust, frail and dependent varied based on the functional tool or test used and the number of individuals in each population group are summarized in **Table 2**. Despite the differences obtained, it has been possible to see that there are subjects that are considered frail or robust individuals in all the classification scales or tests analyzed (**Table 1**).

**Table 2. Classification of the 26 individuals of Table 1 depending on the scale or tool used.**

| | **Tilburg** | **TUG** | **GS** | **KOS** | **(Barthel)** |
|---|---|---|---|---|---|
| **Robust** | 16 | 16 | 13 | 9 | 14 |
| **Frail** | 10 | 10 | 13 | 17 | 7 **Dependents** |
| | | | | | 5 **Frail** |

The transcriptomic study is then carried out of the different population groups characterized in the cohort studied (**Table 1**). Firstly, blood was extracted from all the participants in the study (n=26) to search for biomarkers by means of expression arrays. RNA was isolated from the blood cells (peripheral blood mononuclear cells - PBMCs) by means of a RNeasy Mini kit (Qiagen). The amount of RNA was quantified per sample by characterization by absorbance (Nanodrop) and 2 µg were aliquoted per individual.

HuGene-2_0-st-v1 expression arrays (Affymetrix) were used for the transcriptomic analysis, analyzing the expression of 48226 transcripts present in the isolated RNA of the blood sample extracted from the subjects included in this study (n=26). After undergoing the Affymetrix quality controls through the Expression console software, the data were standardized by means of RMA (Robust Median Analysis) implemented in R-Bioconductor. In addition, a quality control was carried out of the samples and of the data obtained with the internal controls of the array itself and the use of studies by means of hierarchical clustering, principal component analysis (PCA) and with the search for possible technical outliers.

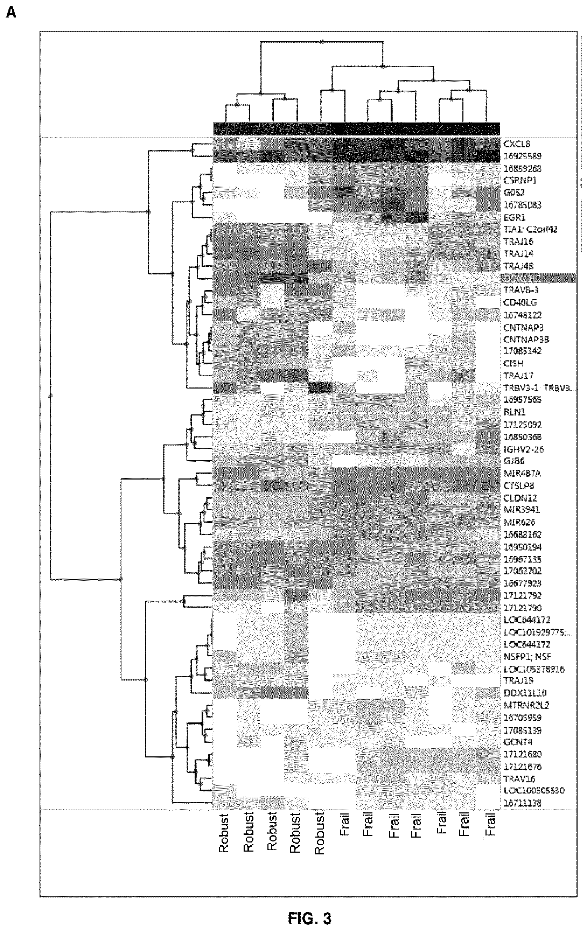

Data analysis was carried out on 26 subjects, comparing the molecular pattern presented by the robust individuals with respect to the molecular pattern of frail individuals, by using the scales and tests described to group robust and frail indicated in Table 1. The molecular pattern of frail individuals varies and is very heterogeneous depending on whether the analysis is carried out based on the Tilburg Test, on the Time Up and Go or on the Gait Speed test (Fig. 3A).

Then, the samples of the individuals that are frail or robust in the five scales or tools used were selected, which enables clear differences in the molecular pattern of the robust and frail groups to be observed. Moreover, the sensitivity and specificity values for the frail individuals were specifically determined by means of different statistical classifiers, such as *Compound Covariate Predictor* (CCP), *Diagonal linear Discriminant Analysis* (DLDA), *1-Nearest Neighbor* (1NN), *3-Nearest Neighbors* (3NN), *Nearest Centroid* (NC), *Support Vector Machine* (SVM) and *Bayesian Compound Covariate Predictor* (BCCP).

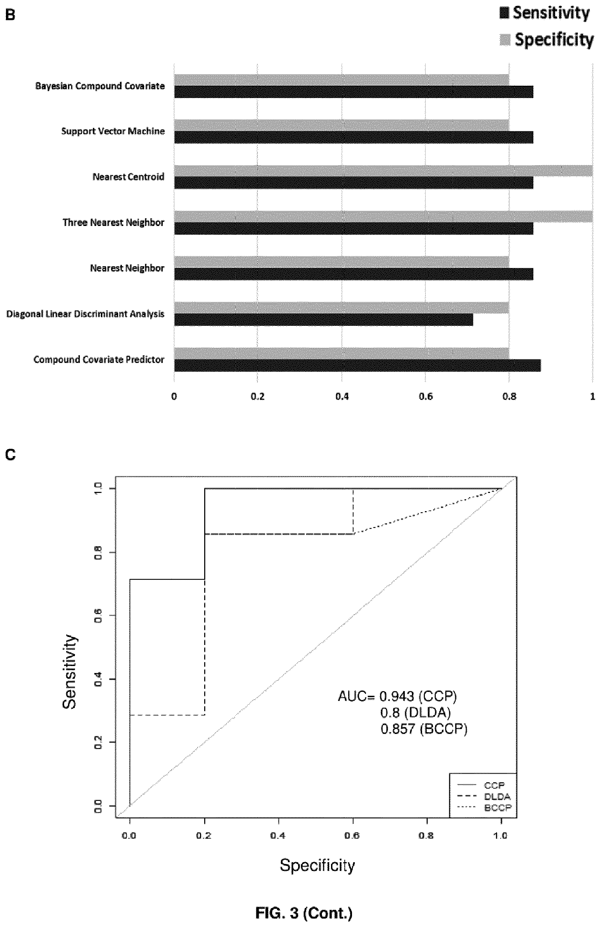

The results obtained with the different aforementioned classifiers have enabled a set of 35 biomarkers (Table 3) to be identified, which makes it possible to differentiate between robust and frail individuals, with a promising ROC curve (area under the curve: 0.943) (FIG. 3C). The analyses carried out for the selection of this set of 35 genes show a sensitivity between 0.714 and 0.857 and a specificity between 0.8 and 1 (FIG. 3B). The best results (sensitivity 0.857 and specificity 1) are obtained with the 3NN and NC classifiers.

Table 3 includes the list of the 35 biomarkers identified as potential frailty biomarkers, along with the statistical value thereof.

**Table 3. Frailty biomarkers analyzed in the present invention.**

| **Biomarker** | **Entrez Gene ID (SEQ ID NO:)** | **Uniprot Accession (SEQ ID NO:)** | ***Frail Avg (log2)*** | ***Robust Avg (log2)*** | ***Fold Change*** | **P-value** |
|---|---|---|---|---|---|---|
| **EGR1** | 1958 (SEQ ID NO: 1) | P18146 (SEQ ID NO: 2) | 8.08 | 6.66 | 2.66 | 0.0014 |
| **DDX11L1** | 100287102 (SEQ ID NO: 3) | B7ZGX0 (SEQ ID NO: 4) | 7.73 | 9.32 | -3.01 | 0.0039 |
| **Hsa_miR-454** | 768216 (SEQ ID NO: 5) | N/A | 3.13 | 3.94 | -1.63 | 0.0004 |
| **CISH** | 1154 (SEQ ID NO: 6) | Q9NSE2 (SEQ ID NO: 7) | 6.82 | 7.99 | -2.25 | 0.0033 |
| **DDX11L10** | 100287029 (SEQ ID NO: 8) | B7ZGX5 (SEQ ID NO: 9) | 6.25 | 8.28 | -4.11 | 0.0013 |
| **LOC101929775** | 101929775 (SEQ ID NO: 10) | N/A | 5.82 | 7.22 | -2.62 | 0.0004 |
| **LOC644172** | 644172 (SEQ ID NO: 11) | N/A | 5.82 | 7.22 | -2.62 | 0.0004 |
| **NSF** | 4905 (SEQ ID NO: 12) | Q96D47 (SEQ ID NO: 13) | 5.58 | 7.43 | -3.59 | 0.0002 |
| **TRAJ17** | 28738 (SEQ ID NO: 14) | A0A075B6W8 (SEQ ID NO: 15) | 7.18 | 8.57 | -2.62 | 0.0017 |
| **TRAJ19** | 28736 (SEQ ID NO: 16) | A0A075B6Y4 (SEQ ID NO: 17) | 6.52 | 7.6 | -2.11 | 0.0037 |
| **TRAV8-3** | 28683 (SEQ ID NO: 18) | A0A0A6YYJ7 (SEQ ID NO: 19) | 7.42 | 8.86 | -2.71 | 0.0004 |
| CD40LG | 959 (SEQ ID NO: 20) | Q45QX2 (SEQ ID NO: 21) | 7.14 | 8.15 | -2.01 | 0.0098 |
| **CLDN12** | 9069 (SEQ ID NO: 22) | P56749 (SEQ ID NO: 23) | 3.13 | 4.51 | -2.6 | 0.0151 |
| **CNTNAP3** | 79937 (SEQ ID NO: 24) | Q9BZ76 (SEQ ID NO: 25) | 6.8 | 8.18 | -2.6 | 0.0479 |
| **CNTNAP3B** | 728577 (SEQ ID NO: 26) | Q96NU0 (SEQ ID NO: 27) | 6.95 | 8.21 | -2.39 | 0.0310 |
| **CSRNP1** | 64651 (SEQ ID NO: 28) | Q96S65 (SEQ ID NO: 29) | 8.01 | 6.94 | 2.1 | 0.0307 |
| **CTSLP8** | 1518 (SEQ ID NO: 30) | N/A | 2.14 | 3.25 | -2.16 | 0.0260 |
| **CXCL8** | 3576 (SEQ ID NO: 31) | P10145 (SEQ ID NO: 32) | 10.17 | 9.06 | 2.15 | 0.0190 |
| **G0S2** | 50486 (SEQ ID NO: 33) | P27469 (SEQ ID NO: 34) | 8.89 | 7.46 | 2.69 | 0.0318 |
| **GCNT4** | 51301 (SEQ ID NO: 35) | Q9P109 (SEQ ID NO: 36) | 5.8 | 6.82 | -2.02 | 0.0217 |
| **GJB6** | 10804 (SEQ ID NO: 37) | 095452 (SEQ ID NO: 38) | 5.18 | 4.09 | 2.12 | 0.0057 |
| **IGHV2-26** | 28455 (SEQ ID NO: 39) | A0A0B4J1V2 (SEQ ID NO: 40) | 3.63 | 4.88 | -2.38 | 0.0291 |
| **LOC100505530** | 100505530 (SEQ ID NO: 41) | N/A | 5.35 | 6.64 | -2.45 | 0.0224 |
| **LOC105378916** | 105378916 (SEQ ID NO: 42) | N/A | 6.36 | 7.72 | -2.56 | 0.0333 |
| **miR-3941** | 100500866 (SEQ ID NO: 43) | N/A | 3.16 | 4.43 | -2.4 | 0.0059 |
| **miR-487A** | 619555 (SEQ ID NO: 44) | N/A | 2.42 | 3.62 | -2.29 | 0.0275 |
| **miR-626** | 693211 (SEQ ID NO: 45) | N/A | 2.96 | 4.04 | -2.11 | 0.0244 |
| **MTRNR2L2** | 100462981 (SEQ ID NO: 46) | P0CJ69 (SEQ ID NO: 47) | 5.58 | 6.84 | -2.39 | 0.0334 |
| **RLN1** | 6013 (SEQ ID NO: 48) | P04808 (SEQ ID NO: 49) | 4.65 | 5.71 | -2.08 | 0.0092 |
| **TIA1** | 7072 (SEQ ID NO: 50) | P31483 (SEQ ID NO: 51) | 7.46 | 8.49 | -2.05 | 0.0462 |
| **TRAJ14** | 28741 (SEQ ID NO: 52) | A0N4Z3 (SEQ ID NO: 53) | 8.14 | 9.17 | -2.04 | 0.0128 |
| **TRAJ16** | 28739 (SEQ ID NO: 54) | A0A75B6V0 (SEQ ID NO: 55) | 7.77 | 8.78 | -2.01 | 0.0300 |
| **TRAJ48** | 28707 (SEQ ID NO: 56) | A0A075B6V3 (SEQ ID NO: 57) | 7.83 | 8.9 | -2.09 | 0.0185 |
| **TRAV16** | 28667 (SEQ ID NO: 58) | A0A0A6YYK6 (SEQ ID NO: 59) | 5 | 6.58 | -2.98 | 0.0172 |
| **TRBV3-1** | 28619 (SEQ ID NO: 60) | A0A576 (SEQ ID NO: 61) | 7.21 | 8.39 | -2.26 | 0.0058 |

Entrez gene ID: Identifying code of the gene in the NCBI database; Uniprot AC: Identifying code of the protein coded by the gene in the Uniprot database; N/A: the transcript does not code for protein; *Frail-Robust Average* (log2): values of the Frail and Robust group in log2; *Fold Change:* Relationship between the frail and robust values; p-value: statistical significance obtained in the statistical study.

Therefore, the analysis of the samples of the individuals that are frail or robust in the five scales or tools used enables the results obtained to be differentiated, providing the method described and the results obtained in the present invention with effectiveness and robustness that has not been shown in any study known in the state of the art until now, wherein the results obtained come from one or two functional scales or tools to identify frailty. The analyses of the Principle Component Analysis (PCA) show that the scales and tools used to assess frailty (clinical test), despite the heterogeneity thereof, can be classification tools that enable the results thereof to be correlated with different molecular expression patterns.

The following step was to obtain a sufficiently robust profile that can be used in everyday clinical practice and, to do so, the minimum molecular pattern was sought, based on the 35 biomarkers identified and mentioned above **(table** 3), that enables frail individuals to be identified with a high specificity and sensitivity. To do so, a statistical analysis was carried out by means of the T-test (Fc>|2| and p-value<0.05), identifying the EGR1 biomarker as the most reproducible and solid candidate for identifying frail individuals. In the data obtained from the arrays, EGR1 shows an area under the curve of 0.831 (p=0.009), with a sensitivity of 100% and a specificity of 87.5% for a cut-off point established at 6.18.

Taking into account the data shown in Table 3, wherein it can be seen that, in addition to EGR1, there are other biomarkers that also individually have good data for identifying frail individuals, although not as good as those of EGR1. As such, the inventors show that the combination of three biomarkers, EGR1, DDS and miR454, has a synergistic effect in the identification of frail individuals, providing a value under the curve of 0.868 (p=0.004), with a sensitivity of 100% and a specificity of 94.1%.

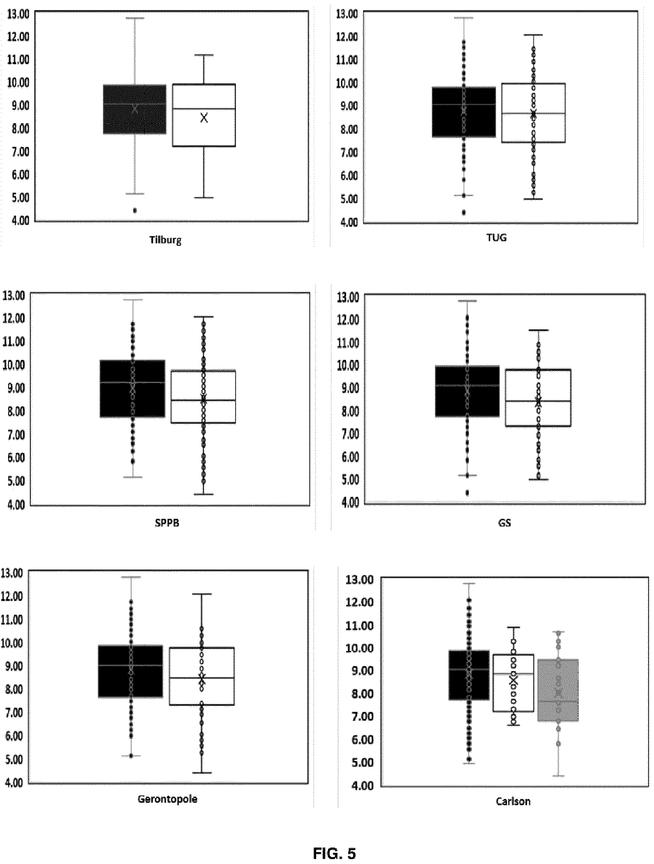

The three genes identified in the transcriptomic analysis, and selected from the set of 35 genes differentially expressed between frail and robust individuals, were then validated. First, the expression thereof in the samples of robust and frail individuals used in the original microarray was analyzed by means of quantitative PCR (RTqPCR). This analysis confirmed the result obtained in the microarray, it being observed that the expression levels of the EGR1 gene (SEQ ID NO: 1) increased, while those of the DDX11L1 gene (SEQ ID NO: 3), and those of the miRNA hsa-miR-454 (SEQ ID NO: 5) reduced in frail individuals compared to robust individuals **(****FIG. 5****).**

Once the EGR1 gene (SEQ ID NO: 1), which can be used in combination with the DDX11L1 gene (SEQ ID NO: 3) and with miRNA hsa-miR-454 (SEQ ID NO: 5), is identified as a frailty biomarker, the EGR1 biomarker was identified, once again by means of qPCR technique, and the difference of said biomarker between frail and robust individuals was greater in a wider population series (n=172). The RTqPCRs have been carried out with predesigned probes for the EGR gene of the company Qiagen (for EGR1 (QT00999964), using B2M (QT00088935) as an endogenous gene. The clinical characteristics of this validation series are summarized in **Table 4.**

**Table 4. Summary of demographic characteristics of the validation cohort.**

| | **Male** | **Female** | **Total** | **N total** |
|---|---|---|---|---|
| **n** | 81 (47.09%) | 91 (52.91%) | | 172 |
| **Average age** | 80.22 | 80.00 | 80.10 | 172 |
| **% Frail TUG** | 32.10% | 40.66% | 36.63% | 172 |
| **% Frail Tilburg** | 19.23% | 31.82% | 25.90% | 172 |
| **% Frail GS** | 20.99% | 35.16% | 28.49% | 172 |
| **% Frail all** | 3.70% | 6.59% | 36.69% | 9 |
| **% Robust all** | 35.80% | 21.98% | 36.90% | 49 |

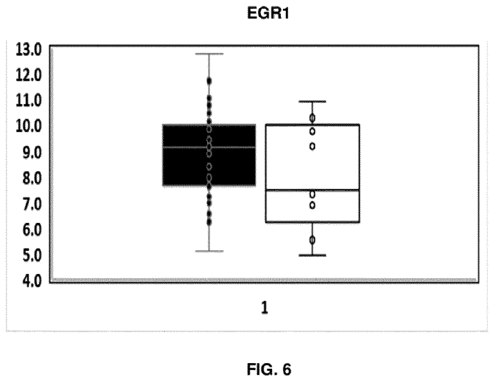

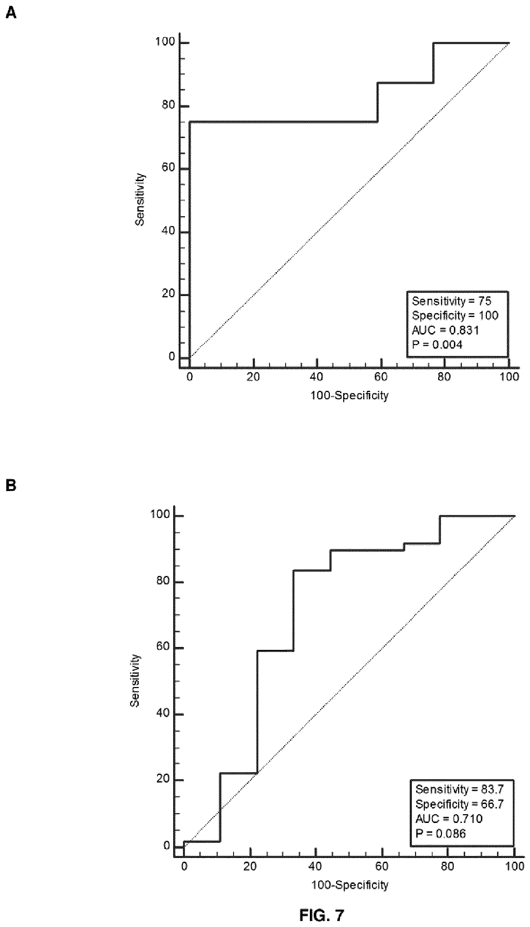

The results obtained in this validation cohort for the frailty biomarker EGR1 (SEQ ID NO: 1) confirm the data obtained previously, showing that said biomarker has an altered expression, which is significantly increased in frail individuals with respect to the expression of said biomarkers in robust individuals (p=0.0260). It must be noted that the expression levels of the EGR1 biomarkers (SEQ ID NO: 1) are increased (in a range of 1.07 to 1.43) in frail individuals using all the functional tests and scales analyzed, which include the Tilburg Test, TUG and GS, SPPB, Gerontopole and Carlson Test (FIG. 6), but above all when the frail and robust individuals are selected according to the tests and scales studied in the validation cohort (FIG. 7). According to the above diagram, there are 58 individuals in the validation cohort that share classification in all the scales. In this subcohort, EGR1 functions best as a biomarker (FC:1.84) (FIG. 7).

These data confirm that EGR1 is capable of identifying and grouping frail individuals using the majority of the functional tests and scales studied, but above all when the frail individuals are selected according to all the tests and scales.

The analysis carried out for the EGR1 biomarker in the cohorts analyzed, both the original cohort and the validation cohort, show a ROC curve with a sensitivity of between 0.75 and 0.83 and a specificity between 0.66 and 1 (**FIG. 7**).

These results consolidate the usefulness of the analysis of the expression levels of the EGR1 biomarker to differentiate the frail subjects or that will suffer from frailty from robust subjects.

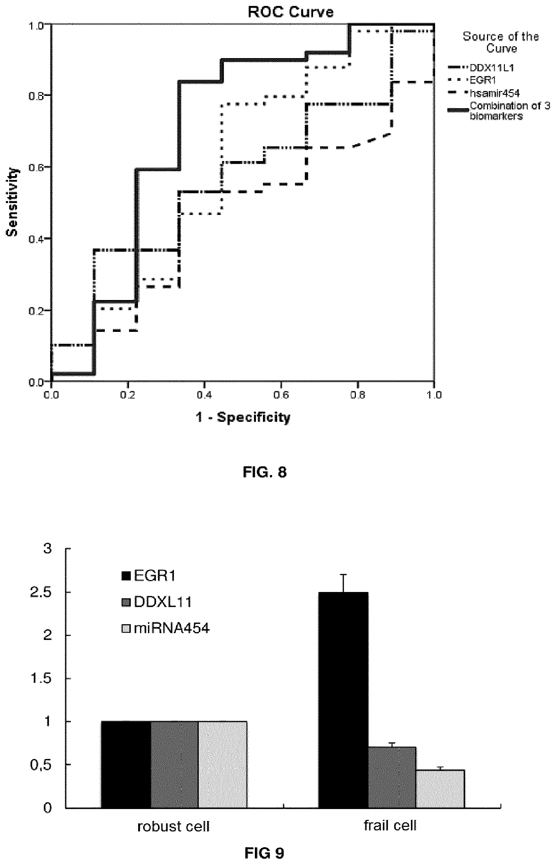

Lastly, according to the analysis diagram used in the arrays, the impact of the EGR1 (SEQ ID NO: 1) and DDX11L1 (SEQ ID NO: 3) genes and miRNA hsa-miR-454 (SEQ ID NO: 5), as a group molecular pattern that enables frail individuals to be identified sensitively and specifically using each of said biomarkers individually, was analyzed. In order to carry out said analysis, the ROC curve of each of the biomarkers was individually analyzed, as was the ROC curve for the combination of the three biomarkers, in the validation cohort of robust and frail individuals (n=58). The results show, as mentioned above, that the best results with regards to specificity and sensitivity are shown by the EGR1 biomarker individually, with respect to the other biomarkers (DDX11L1 and miR-454) individually. However, as seen in FIG. 8, there is synergy with regards to the sensitivity and specificity in the identification of frail individuals when the three EGR1, DDXL11 and miR-454 biomarkers are combined (**FIG. 8**).

All the results shown herein prove the usefulness of the EGR1 biomarker in the *in vitro* diagnosis and/or prognosis of frailty in a subject, which when used in combination with the DDX11L1 and miR-454 biomarkers, show a greater specificity and sensitivity in the identification of frail individuals.

### Example 2. Analysis of the expression of frailty biomarkers of the invention in fibroblast cultures.

Maintenance by means of serial passages of cell cultures, especially fibroblasts, creates stress in the cells that is similar to the molecular and physiological level that aging and development of frailty entails. Taking this into account, the expression of frailty biomarkers, EGR1, DDX11L1 and hsa-miR-454, in *in vitro* fibroblast cultures obtained from samples of adult subjects aged between 30 and 50 years old were analyzed.

The fibroblasts were isolated from the skin of said individuals and the cell cultures were created through the methods of mechanical and enzymatic cell decomposition, with trypsin. The isolated fibroblasts were maintained in culture during a number of passages (each passage is 7 days of maintenance in culture) until they are well established and grown. The serial passage tests *(in vitro* aging) with said fibroblasts were initiated in early stages when the cells are still robust and they have been cultivated by means of serial passages in cell culture conditions in incubators with 20% oxygen, being similar to the aging and frailty conditions observed in individuals between the ages of 50 and 70, until high passages are reach wherein the cells provide frailty characteristics.

After said time has passed, said cultures were collected and the expression of the biomarkers of the invention in said cells was analyzed. The results of the *in vitro* fibroblasts show that there is also a differentiated expression of the biomarker of the invention, EGR1 (SEQ ID NO: 1), in the robust fibroblast cultures (early passage) used as a control with respect to the frail fibroblast cultures (serial culture). Specifically, the results show that the expression of said biomarker is altered in the frail cells, a significant increase of 2.49 time in the expression of the biomarker with respect to robust cells (p=0.018) being observed (**FIG. 9**).

Additionally, the expression of the DDXL11 and hsa-miR-454 biomarkers in the robust fibroblast cultures (early passage) used as a control with respect to the frail fibroblast cultures (serial culture) was also analyzed. The results show that the expression of said biomarkers are altered in the frail cells, a significant decrease of 0.70 and 0.44 times the expression levels of the DDX11L1 (SEQ ID NO: 3) (p=0.0038) and hsa-miR-454 (SEQ ID NO: 5) (p=0.0001) biomarkers, respectively, with respect to the robust cells was observed (**FIG. 9**).

Said *in vitro* results correlate with the results obtained in the samples of subjects diagnosed as frail using the biomarkers of the present invention.

## Claims

1. Use of the expression levels of the EGR1 biomarker, for the *in vitro* diagnosis and/or prognosis of frailty in a subject.

2. The use according to claim 1, further comprising the expression levels of at least one of the biomarkers selected from the list consisting of: DDX11L1, miR-454 and/or any combinations thereof.

3. The use according to claim 1 or 2, further comprising the expression levels of at least one of the biomarkers selected from the list consisting of: CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1, and/or any combinations thereof.

4. The use according to any of claims 1 to 3, further comprising the expression levels of the biomarkers CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 and TRBV3-1.

5. An *in vitro* method for the diagnosis and/or prognosis of frailty in an individual comprising:
(a) quantifying the expression levels of the EGR1 biomarker in an isolated biological sample of said individual, and
(b) comparing the expression levels obtained in step (a) with reference values,
wherein an increase in expression levels of the EGR1 biomarker in the sample of the patient with respect to the reference values for said biomarker, is indicative of suffering or being predisposed to suffering from frailty.

6. The *in vitro* method according to claim 5, further comprising quantifying in step (a) the expression levels of at least one of the biomarkers selected from the list consisting of: DDX11L1, miR-454, and/or any combinations thereof, preferably the set of DDX11L1 and miR-454 biomarkers, wherein a reduction in the expression levels of the DDX11L1 and miR-454 biomarkers, in the sample of the patient with respect to the reference values for said biomarkers, is indicative of suffering or being predisposed to suffering from frailty.

7. The *in vitro* method according to claims 5 or 6, further comprising quantifying in step (a) the expression levels of at least one of the biomarkers selected from the list consisting of: CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1, and/or any combinations thereof, preferably the set of biomarkers CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 and TRBV3-1.

8. The *in vitro* method according to any of claims 5 to 7, wherein the reference value corresponds to expression levels of the biomarkers in an isolated biological sample of robust individuals.

9. The *in vitro* method according to any of claims 5 to 8, wherein the isolated biological sample of step (a) is a blood, plasma, serum or urine sample.

10. The *in vitro* method according to claim 9, wherein the isolated biological sample are peripheral blood mononuclear cells.

11. A use of the kit or device comprising primers, probes and/or antibodies capable of detecting the expression levels of the EGR1 biomarkers in an isolated biological sample, for the *in vitro* diagnosis and/or prognosis of frailty in an individual.

12. Use of the kit or device according to claim 11, wherein the kit or device further comprises primers, probes and/or antibodies capable of detecting the expression levels of at least one of the biomarkers selected from the list consisting of: DDX11L1, miR-454, CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 and TRBV3-1, and/or any combinations thereof.

## Patentansprüche

1. Verwendung der Expressionsniveaus des EGR1-Biomarkers, für die *in-vitro* Diagnose und/oder Prognose von Gebrechlichkeit bei einem Subjekt.

2. Verwendung nach Anspruch 1, ferner umfassend die Expressionsniveaus von mindestens einem der Biomarker, ausgewählt aus der Liste, bestehend aus: DDX11L1, miR-454 und/oder beliebige Kombinationen davon.

3. Verwendung nach Anspruch 1 oder 2, ferner umfassend die Expressionsniveaus von mindestens einem der Biomarker, ausgewählt aus der Liste, bestehend aus: CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1 und/oder beliebige Kombinationen davon.

4. Verwendung nach einem der Ansprüche 1 bis 3, ferner umfassend die Expressionsniveaus der Biomarker CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 und TRBV3-1.

5. Ein *in-vitro* Verfahren zur Diagnose und/oder Prognose von Gebrechlichkeit bei einem Individuum, umfassend:
(a) Quantifizieren der Expressionsniveaus des EGR1-Biomarkers in einer isolierten biologischen Probe des Individuums und
(b) Vergleichen der in Schritt (a) erhaltenen Expressionsniveaus mit Referenzwerten,
wobei ein Anstieg der Expressionsniveaus des EGR1-Biomarkers in der Probe des Patienten in Bezug auf die Referenzwerte für den Biomarker darauf hindeutet, dass er an Gebrechlichkeit leidet oder dafür prädisponiert ist.

6. Das *in-vitro* Verfahren nach Anspruch 5, ferner umfassend in Schritt (a) das Quantifizieren der Expressionsniveaus von mindestens einem der Biomarker, ausgewählt aus der Liste, bestehend aus: DDX11L1, miR-454 und/oder beliebige Kombinationen davon, vorzugsweise der Satz von DDX11 L1und miR-454-Biomarkern, wobei eine Verringerung der Expressionsniveaus der DDX11L1- und miR-454-Biomarker in der Probe des Patienten in Bezug auf die Referenzwerte für diese Biomarker anzeigt, dass man an Gebrechlichkeit leidet oder dafür prädisponiert ist.

7. Das *in-vitro* Verfahren nach Anspruch 5 oder 6, ferner umfassend in Schritt (a) das Quantifizieren der Expressionsniveaus von mindestens einem der Biomarker, ausgewählt aus der Liste, bestehend aus: CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1 und/oder beliebige Kombinationen davon, vorzugsweise der Satz von Biomarkern CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 and TRBV3-1.

8. Das *in-vitro* Verfahren nach einem der Ansprüche 5 bis 7, wobei der Referenzwert Expressionsniveaus der Biomarker in einer isolierten biologischen Probe robuster Individuen entspricht.

9. Das *in-vitro* Verfahren nach einem der Ansprüche 5 bis 8, wobei die isolierte biologische Probe aus Schritt (a) eine Blut-, Plasma-, Serum- oder Urinprobe ist.

10. Das *in-vitro* Verfahren nach Anspruch 9, wobei die isolierte biologische Probe periphere mononukleäre Blutzellen sind.

11. Eine Verwendung des Kits oder der Vorrichtung, umfassend Primer, Sonden und/oder Antikörper, die in der Lage sind, die Expressionsniveaus der EGR1-Biomarker in einer isolierten biologischen Probe nachzuweisen, für die *in-vitro* Diagnose und/oder Prognose von Gebrechlichkeit bei einem Individuum.

12. Verwendung des Kits oder der Vorrichtung nach Anspruch 11, wobei das Kit oder die Vorrichtung ferner Primer, Sonden und/oder Antikörper umfasst, die in der Lage sind, die Expressionsniveaus von mindestens einem der Biomarker nachzuweisen, ausgewählt aus der Liste, bestehend aus: DDX11L1, miR-454, CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 and TRBV3-1 und/oder beliebige Kombinationen davon.

## Revendications

1. Utilisation des niveaux d'expression du biomarqueur EGR1, pour le diagnostic et/ou le pronostic *in vitro* de fragilité chez un sujet.

2. Utilisation selon la revendication 1, comprenant en outre les niveaux d'expression d'au moins l'un des biomarqueurs choisis dans la liste constituée de : DDX11L1, miR-454 et/ou n'importe quelles combinaisons de ceux-ci.

3. Utilisation selon la revendication 1 ou 2, comprenant en outre les niveaux d'expression d'au moins l'un des biomarqueurs choisis dans la liste constituée de : CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1 et/ou n'importe quelles combinaisons de ceux-ci.

4. Utilisation selon l'une quelconque des revendications 1 à 3, comprenant en outre les niveaux d'expression des biomarqueurs CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 et TRBV3-1.

5. Procédé *in vitro* pour le diagnostic et/ou le pronostic de fragilité chez un individu comprenant :
(a) la quantification des niveaux d'expression du biomarqueur EGR1 dans un échantillon biologique isolé dudit individu et
(b) la comparaison des niveaux d'expression obtenus à l'étape (a) avec des valeurs de référence,
dans lequel une augmentation des niveaux d'expression du biomarqueur EGR1 dans l'échantillon du patient, par rapport aux valeurs de référence pour ledit biomarqueur, indique qu'il souffre ou qu'il est prédisposé à souffrir de fragilité.

6. Procédé *in vitro* selon la revendication 5, comprenant en outre la quantification à l'étape (a) des niveaux d'expression d'au moins l'un des biomarqueurs choisis dans la liste constituée de : DDX11L1, miR-454 et/ou n'importe quelles combinaisons de ceux-ci, de préférence de l'ensemble des biomarqueurs DDX11L1 et miR-454, dans lequel une réduction des niveaux d'expression des biomarqueurs DDX11L1 et miR-454, dans l'échantillon du patient par rapport aux valeurs de référence pour lesdits biomarqueurs, indique qu'il souffre ou qu'il est prédisposé à souffrir de fragilité.

7. Procédé *in vitro* selon les revendications 5 ou 6, comprenant en outre la quantification à l'étape (a) des niveaux d'expression d'au moins l'un des biomarqueurs choisis dans la liste constituée de: CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16, TRBV3-1 et/ou n'importe quelles combinaisons de ceux-ci, de préférence de l'ensemble des biomarqueurs CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 et TRBV3-1.

8. Procédé *in vitro* selon l'une quelconque des revendications 5 à 7, dans lequel la valeur de référence correspond à des niveaux d'expression des biomarqueurs dans un échantillon biologique isolé d'individus robustes.

9. Procédé *in vitro* selon l'une quelconque des revendications 5 à 8, dans lequel l'échantillon biologique isolé de l'étape (a) est un échantillon de sang, de plasma, de sérum ou d'urine.

10. Procédé *in vitro* selon la revendication 9, dans lequel l'échantillon biologique isolé est des cellules mononucléaires du sang périphérique.

11. Utilisation du kit ou du dispositif comprenant des amorces, des sondes et/ou des anticorps capables de détecter les niveaux d'expression des biomarqueurs EGR1 dans un échantillon biologique isolé, pour le diagnostic et/ou le pronostic *in vitro* de fragilité chez un individu.

12. Utilisation du kit ou du dispositif selon la revendication 11, dans lequel le kit ou le dispositif comprend en outre des amorces, des sondes et/ou des anticorps capables de détecter les niveaux d'expression d'au moins l'un des biomarqueurs choisis dans la liste constituée de : DDX11L1, miR-454, CISH, DDX11L10, LOC101929775, LOC644172, NSF, TRAJ17, TRAJ19, TRAV8-3, CD40LG, CLDN12, CNTNAP3, CNTNAP3B, CSRNP1, CTSLP8, CXCL8, G0S2, GCNT4, GJB6, IGHV2-26, LOC100505530, LOC105378916, miR-3941, miR-487A, miR-626, MTRNR2L2, RLN1, TIA1, TRAJ14, TRAJ16, TRAJ48, TRAV16 et TRBV3-1 et/ou n'importe quelles combinaisons de ceux-ci.


### SEQUENCE LISTING

<110> Administración General de la Comunidad Autónoma de Euskadi.
<120> Biomarkers for the diagnosis and/or prognosis of frailty
<130> EP3347.5
<160> 61
<170> PatentIn version 3.5
<210> 1
   <211> 3136
   <212> DNA
   <213> Homo sapiens
<400> 1
   gcgcagaact tggggagccg ccgccgccat ccgccgccgc agccagcttc cgccgccgca      60
   ggaccggccc ctgccccagc ctccgcagcc gcggcgcgtc cacgcccgcc cgcgcccagg     120
   gcgagtcggg gtcgccgcct gcacgcttct cagtgttccc cgcgccccgc atgtaacccg     180
   gccaggcccc cgcaactgtg tcccctgcag ctccagcccc gggctgcacc cccccgcccc     240
   gacaccagct ctccagcctg ctcgtccagg atggccgcgg ccaaggccga gatgcagctg     300
   atgtccccgc tgcagatctc tgacccgttc ggatcctttc ctcactcgcc caccatggac     360
   aactacccta agctggagga gatgatgctg ctgagcaacg gggctcccca gttcctcggc     420
   gccgccgggg ccccagaggg cagcggcagc aacagcagca gcagcagcag cgggggcggt     480
   ggaggcggcg gggcggcagc caacagcagc agcagcagca gcaccttcaa ccctcaggcg     540
   gacacgggcg agcagcccta cgagcacctg accgcagagt cttttcctga catctctctg     600
   aacaacgaga aggtgctggt ggagaccagt taccccagcc aaaccactcg actgcccccc     660
   atcacctata ctggccgctt ttccctggag cctgcaccca acagtggcaa caccttgtgg     720
   cccgagcccc tcttcagctt ggtcagtggc ctagtgagca tgaccaaccc accggcctcc     780
   tcgtcctcag caccatctcc agcggcctcc tccgcctccg cctcccagag cccacccctg     840
   agctgcgcag tgccatccaa cgacagcagt cccatttact cagcggcacc caccttcccc     900
   acgccgaaca ctgacatttt ccctgagcca caaagccagg ccttcccggg ctcggcaggg     960
   acagcgctcc agtacccgcc tcctgcctac cctgccgcca agggtggctt ccaggttccc    1020
   atgatccccg actacctgtt tccacagcag cagggggatc tgggcctggg caccccagac    1080
   cagaagccct tccagggcct ggagagccgc acccagcagc cttcgctaac ccctctgtct    1140
   actattaagg cctttgccac tcagtcgggc tcccaggacc tgaaggccct caataccagc    1200
   taccagtccc agctcatcaa acccagccgc atgcgcaagt accccaaccg gcccagcaag    1260
   acgccccccc acgaacgccc ttacgcttgc ccagtggagt cctgtgatcg ccgcttctcc    1320
   cgctccgacg agctcacccg ccacatccgc atccacacag gccagaagcc cttccagtgc    1380
   cgcatctgca tgcgcaactt cagccgcagc gaccacctca ccacccacat ccgcacccac     1440
   acaggcgaaa agcccttcgc ctgcgacatc tgtggaagaa agtttgccag gagcgatgaa     1500
   cgcaagaggc ataccaagat ccacttgcgg cagaaggaca agaaagcaga caaaagtgtt     1560
   gtggcctctt cggccacctc ctctctctct tcctacccgt ccccggttgc tacctcttac     1620
   ccgtccccgg ttactacctc ttatccatcc ccggccacca cctcataccc atcccctgtg     1680
   cccacctcct tctcctctcc cggctcctcg acctacccat cccctgtgca cagtggcttc     1740
   ccctccccgt cggtggccac cacgtactcc tctgttcccc ctgctttccc ggcccaggtc     1800
   agcagcttcc cttcctcagc tgtcaccaac tccttcagcg cctccacagg gctttcggac     1860
   atgacagcaa ccttttctcc caggacaatt gaaatttgct aaagggaaag gggaaagaaa     1920
   gggaaaaggg agaaaaagaa acacaagaga cttaaaggac aggaggagga gatggccata     1980
   ggagaggagg gttcctctta ggtcagatgg aggttctcag agccaagtcc tccctctcta     2040
   ctggagtgga aggtctattg gccaacaatc ctttctgccc acttcccctt ccccaattac     2100
   tattcccttt gacttcagct gcctgaaaca gccatgtcca agttcttcac ctctatccaa     2160
   agaacttgat ttgcatggat tttggataaa tcatttcagt atcatctcca tcatatgcct     2220
   gaccccttgc tcccttcaat gctagaaaat cgagttggca aaatggggtt tgggcccctc      2280
   agagccctgc cctgcaccct tgtacagtgt ctgtgccatg gatttcgttt ttcttggggt     2340
   actcttgatg tgaagataat ttgcatattc tattgtatta tttggagtta ggtcctcact     2400
   tgggggaaaa aaaaaaaaga aaagccaagc aaaccaatgg tgatcctcta ttttgtgatg     2460
   atgctgtgac aataagtttg aacctttttt tttgaaacag cagtcccagt attctcagag     2520
   catgtgtcag agtgttgttc cgttaacctt tttgtaaata ctgcttgacc gtactctcac     2580
   atgtggcaaa atatggtttg gtttttcttt tttttttttt ttgaaagtgt tttttcttcg     2640
   tccttttggt ttaaaaagtt tcacgtcttg gtgccttttg tgtgatgcgc cttgctgatg     2700
   gcttgacatg tgcaattgtg agggacatgc tcacctctag ccttaagggg ggcagggagt     2760
   gatgatttgg gggaggcttt gggagcaaaa taaggaagag ggctgagctg agcttcggtt     2820
   ctccagaatg taagaaaaca aaatctaaaa caaaatctga actctcaaaa gtctattttt     2880
   ttaactgaaa atgtaaattt ataaatatat tcaggagttg gaatgttgta gttacctact     2940
   gagtaggcgg cgatttttgt atgttatgaa catgcagttc attattttgt ggttctattt     3000
   tactttgtac ttgtgtttgc ttaaacaaag tgactgtttg gcttataaac acattgaatg     3060
   cgctttattg cccatgggat atgtggtgta tatccttcca aaaaattaaa acgaaaataa     3120
   agtagctgcg attggg                                                      3136
<210> 2
   <211> 543
   <212> **PRT**
   <213> Homo sapiens
<400> 2
   Met Ala Ala Ala Lys Ala Glu Met Gln Leu Met Ser Pro Leu Gln Ile
   1               5                   10                  15
   Ser Asp Pro Phe Gly Ser Phe Pro His Ser Pro Thr Met Asp Asn Tyr
               20                  25                  30
   Pro Lys Leu Glu Glu Met Met Leu Leu Ser Asn Gly Ala Pro Gln Phe
           35                  40                  45
   Leu Gly Ala Ala Gly Ala Pro Glu Gly Ser Gly Ser Asn Ser Ser Ser
       50                  55                  60
   Ser Ser Ser Gly Gly Gly Gly Gly Gly Gly Gly Gly Ser Asn Ser Ser
   65                  70                  75                  80
   Ser Ser Ser Ser Thr Phe Asn Pro Gln Ala Asp Thr Gly Glu Gln Pro
                   85                  90                  95
   Tyr Glu His Leu Thr Ala Glu Ser Phe Pro Asp Ile Ser Leu Asn Asn
               100                 105                 110
   Glu Lys Val Leu Val Glu Thr Ser Tyr Pro Ser Gln Thr Thr Arg Leu
           115                 120                 125
   Pro Pro Ile Thr Tyr Thr Gly Arg Phe Ser Leu Glu Pro Ala Pro Asn
       130                 135                 140
   Ser Gly Asn Thr Leu Trp Pro Glu Pro Leu Phe Ser Leu Val Ser Gly
   145                 150                 155                 160
   Leu Val Ser Met Thr Asn Pro Pro Ala Ser Ser Ser Ser Ala Pro Ser
                   165                 170                 175
   Pro Ala Ala Ser Ser Ala Ser Ala Ser Gln Ser Pro Pro Leu Ser Cys
               180                 185                 190
   Ala Val Pro Ser Asn Asp Ser Ser Pro Ile Tyr Ser Ala Ala Pro Thr
           195                 200                 205
   Phe Pro Thr Pro Asn Thr Asp Ile Phe Pro Glu Pro Gln Ser Gln Ala
       210                 215                 220
   Phe Pro Gly Ser Ala Gly Thr Ala Leu Gln Tyr Pro Pro Pro Ala Tyr
   225                 230                 235                 240
   Pro Ala Ala Lys Gly Gly Phe Gln Val Pro Met Ile Pro Asp Tyr Leu
                   245                 250                 255
   Phe Pro Gln Gln Gln Gly Asp Leu Gly Leu Gly Thr Pro Asp Gln Lys
               260                 265                 270
   Pro Phe Gln Gly Leu Glu Ser Arg Thr Gln Gln Pro Ser Leu Thr Pro
           275                 280                 285
   Leu Ser Thr Ile Lys Ala Phe Ala Thr Gln Ser Gly Ser Gln Asp Leu
       290                 295                 300
   Lys Ala Leu Asn Thr Ser Tyr Gln Ser Gln Leu Ile Lys Pro Ser Arg
   305                 310                 315                 320
   Met Arg Lys Tyr Pro Asn Arg Pro Ser Lys Thr Pro Pro His Glu Arg
                   325                 330                 335
   Pro Tyr Ala Cys Pro Val Glu Ser Cys Asp Arg Arg Phe Ser Arg Ser
               340                 345                 350
   Asp Glu Leu Thr Arg His Ile Arg Ile His Thr Gly Gln Lys Pro Phe
           355                 360                 365
   Gln Cys Arg Ile Cys Met Arg Asn Phe Ser Arg Ser Asp His Leu Thr
       370                 375                 380
   Thr His Ile Arg Thr His Thr Gly Glu Lys Pro Phe Ala Cys Asp Ile
   385                 390                 395                 400
   Cys Gly Arg Lys Phe Ala Arg Ser Asp Glu Arg Lys Arg His Thr Lys
                   405                 410                 415
   Ile His Leu Arg Gln Lys Asp Lys Lys Ala Asp Lys Ser Val Val Ala
               420                 425                 430
   Ser Ser Ala Thr Ser Ser Leu Ser Ser Tyr Pro Ser Pro Val Ala Thr
           435                 440                 445
   Ser Tyr Pro Ser Pro Val Thr Thr Ser Tyr Pro Ser Pro Ala Thr Thr
       450                 455                 460
   Ser Tyr Pro Ser Pro Val Pro Thr Ser Phe Ser Ser Pro Gly Ser Ser
   465                 470                 475                 480
   Thr Tyr Pro Ser Pro Val His Ser Gly Phe Pro Ser Pro Ser Val Ala
                   485                 490                 495
   Thr Thr Tyr Ser Ser Val Pro Pro Ala Phe Pro Ala Gln Val Ser Ser
               500                 505                 510
   Phe Pro Ser Ser Ala Val Thr Asn Ser Phe Ser Ala Ser Thr Gly Leu
           515                 520                 525
   Ser Asp Met Thr Ala Thr Phe Ser Pro Arg Thr Ile Glu Ile Cys
       530                 535                 540
<210> 3
   <211> 1652
   <212> **DNA**
   <213> Homo sapiens
<400> 3
   cttgccgtca gccttttctt tgacctcttc tttctgttca tgtgtatttg ctgtctctta      60
   gcccagactt cccgtgtcct ttccaccggg cctttgagag gtcacagggt cttgatgctg     120
   tggtcttcat ctgcaggtgt ctgacttcca gcaactgctg gcctgtgcca gggtgcaagc     180
   tgagcactgg agtggagttt tcctgtggag aggagccatg cctagagtgg gatgggccat     240
   tgttcatctt ctggcccctg ttgtctgcat gtaacttaat accacaacca ggcatagggg     300
   aaagattgga ggaaagatga gtgagagcat caacttctct cacaacctag gccagtgtgt     360
   ggtgatgcca ggcatgccct tccccagcat caggtctcca gagctgcaga agacgacggc     420
   cgacttggat cacactcttg tgagtgtccc cagtgttgca gaggcagggc catcaggcac     480
   caaagggatt ctgccagcat agtgctcctg gaccagtgat acacccggca ccctgtcctg     540
   gacacgctgt tggcctggat ctgagccctg gtggaggtca aagccacctt tggttctgcc     600
   attgctgctg tgtggaagtt cactcctgcc ttttcctttc cctagagcct ccaccacccc     660
   gagatcacat ttctcactgc cttttgtctg cccagtttca ccagaagtag gcctcttcct     720
   gacaggcagc tgcaccactg cctggcgctg tgcccttcct ttgctctgcc cgctggagac     780
   ggtgtttgtc atgggcctgg tctgcaggga tcctgctaca aaggtgaaac ccaggagagt     840
   gtggagtcca gagtgttgcc aggacccagg cacaggcatt agtgcccgtt ggagaaaaca     900
   ggggaatccc gaagaaatgg tgggtcctgg ccatccgtga gatcttccca gggcagctcc     960
   cctctgtgga atccaatctg tcttccatcc tgcgtggccg agggccaggc ttctcactgg    1020
   gcctctgcag gaggctgcca tttgtcctgc ccaccttctt agaagcgaga cggagcagac    1080
   ccatctgcta ctgccctttc tataataact aaagttagct gccctggact attcaccccc    1140
   tagtctcaat ttaagaagat ccccatggcc acagggcccc tgcctggggg cttgtcacct    1200
   cccccacctt cttcctgagt cattcctgca gccttgctcc ctaacctgcc ccacagcctt    1260
   gcctggattt ctatctccct ggcttggtgc cagttcctcc aagtcgatgg cacctccctc    1320
   cctctcaacc acttgagcaa actccaagac atcttctacc ccaacaccag caattgtgcc    1380
   aagggccatt aggctctcag catgactatt tttagagacc ccgtgtctgt cactgaaacc    1440
   ttttttgtgg gagactattc ctcccatctg caacagctgc ccctgctgac tgcccttctc    1500
   tcctccctct catcccagag aaacaggtca gctgggagct tctgccccca ctgcctaggg    1560
   accaacaggg gcaggaggca gtcactgacc ccgagacgtt tgcatcctgc acagctagag    1620
   atcctttatt aaaagcacac tgttggtttc tg                                  1652
<210> 4
   <211> 76
   <212> **PRT**
   <213> Homo sapiens
<400> 4
   Met Thr Ile Phe Arg Asp Pro Val Ser Val Thr Glu Thr Phe Phe Val
   1               5                   10                  15
   Gly Asp Tyr Ser Ser His Leu Gln Gln Leu Pro Leu Leu Thr Ala Leu
               20                  25                  30
   Leu Ser Ser Leu Ser Ser Gln Arg Asn Arg Ser Ala Gly Ser Phe Cys
           35                  40                  45
   Pro His Cys Leu Gly Thr Asn Arg Gly Arg Arg Gln Ser Leu Thr Pro
       50                  55                  60
   Arg Arg Leu His Pro Ala Gln Leu Glu Ile Leu Tyr
   65                  70                  75
<210> 5
   <211> 115
   <212> DNA
   <213> Homo sapiens
<400> 5
   tctgtttatc accagatcct agaaccctat caatattgtc tctgctgtgt aaatagttct      60
   gagtagtgca atattgctta tagggttttg gtgtttggaa agaacaatgg gcagg          115
<210> 6
   <211> 2285
   <212> DNA
   <213> Homo sapiens
<400> 6
   cccgctcgtc tggcccgccc ccctcgccct cgcaggcggt ccgcgccact gcctctcagt      60
   ccctgtcctg cgcccgcgcg ccccgggagc ctacccagca cgcgctccgc gcccactggt     120
   tccctccagc cgccgccgtc cagccgagtc cccactccgg agtcgccgct gccgcgggga 180
   catggtcctc tgcgttcagg ggtgagcacc cccttgtaag ctcagggcta ctgttgggtg 240
   tcagggaaca aagttttaga ctgctgcgct ccaaagcggg cacacacatg tacctagaac 300
   acaccagcca ctgtccccac catgatgatg acacagccat ggacacaccc ctgcccagac 360
   ctcgtccttt gctggctgtg gagcggactg ggcagcggcc cctgtgggcc ccgtccctgg 420
   aactgcccaa gccagtcatg cagcccttgc ctgctggggc cttcctcgag gaggtggcag 480
   agggtacccc agcccagaca gagagtgagc caaaggtgct ggacccagag gaggatctgc 540
   tgtgcatagc caagaccttc tcctaccttc gggaatctgg ctggtattgg ggttccatta 600
   cggccagcga ggcccgacaa cacctgcaga agatgccaga aggcacgttc ttagtacgtg 660
   acagcacgca ccccagctac ctgttcacgc tgtcagtgaa aaccactcgt ggccccacca 720
   atgtacgcat tgagtatgcc gactccagct tccgtctgga ctccaactgc ttgtccaggc 780
   cacgcatcct ggcctttccg gatgtggtca gccttgtgca gcactatgtg gcctcctgca 840
   ctgctgatac ccgaagcgac agccccgatc ctgctcccac cccggccctg cctatgccta 900
   aggaggatgc gcctagtgac ccagcactgc ctgctcctcc accagccact gctgtacacc 960
   taaaactggt gcagcccttt gtacgcagaa gcagtgcccg cagcctgcaa cacctgtgcc 1020
   gccttgtcat caaccgtctg gtggccgacg tggactgcct gccactgccc cggcgcatgg 1080
   ccgactacct ccgacagtac cccttccagc tctgactgta cggggcaatc tgcccaccct 1140
   cacccagtcg caccctggag gggacatcag ccccagctgg acttgggccc ccactgtccc 1200
   tcctccaggc atcctggtgc ctgcatacct ctggcagctg gcccaggaag agccagcaag 1260
   agcaaggcat gggagagggg aggtgtcaca caacttggag gtaaatgccc ccaggccgca 1320
   tgtggcttca ttatactgag ccatgtgtca gaggatgggg agacaggcag gaccttgtct 1380
   cacctgtggg ctgggcccag acctccactc gcttgcctgc cctggccacc tgaactgtat 1440
   gggcactctc agccctggtt tttcaatccc cagggtcggg taggacccct actggcagcc 1500
   agcctctgtt tctgggagga tgacatgcag aggaactgag atcgacagtg actagtgacc 1560
   ccttgttgag gggtaagcca ggctagggga ctgcacaatt atacactatt tatttattta 1620
   ttctccttgg ggttggtgtc aggggcgagc caaccccacc tctatgccct gagccctggt 1680
   agtccagaga ccccaactct gccctggctt ctctggttct tccctgtgga aagcccatcc 1740
   tgagacatct tgctggaacc aaggcaatcc tggatgtcct ggtactgacc cacccgtctg 1800
   tgaatgtgtc cactctcttc tgcccccagc catatttggg gaggatggac aactacaata 1860
   ggtaagaaaa tgcagccgga gcctcagtcc ccagcagagc ctgtgtctca ccccctcaca 1920
   ggacagagct gtatctgcat agagctggtc tcactgtggc gcaggccccg gggggagtgc 1980
   ctgtgctgtc aggaagaggg ggtgctggtt tgagggccgc cactgcagtt ctgctaggtc 2040
   tgcttcctgc ccaggaaggt gcctgcacat gagaggagag aaatacacgt ctgataagac 2100
   ttcatgaaat aataattata gcaaagaaca gtttggtggt cttttctctt ccactgattt 2160
   ttctgtaatg acattatacc tttattacct ctttatttta ttacctctat aataaaatga 2220
   tacctttcat gtacagagcc ctcaatgggg ccgattttca gttgtgtaaa aaaaaaaaaa 2280
   aaaaa 2285
<210> 7
   <211> 258
   <212> PRT
   <213> Homo sapiens
<400> 7
   Met Val Leu Cys Val Gln Gly Pro Arg Pro Leu Leu Ala Val Glu Arg
   1               5                   10                  15
   Thr Gly Gln Arg Pro Leu Trp Ala Pro Ser Leu Glu Leu Pro Lys Pro
               20                  25                  30
   Val Met Gln Pro Leu Pro Ala Gly Ala Phe Leu Glu Glu Val Ala Glu
           35                  40                  45
   Gly Thr Pro Ala Gln Thr Glu Ser Glu Pro Lys Val Leu Asp Pro Glu
       50                  55                  60
   Glu Asp Leu Leu Cys Ile Ala Lys Thr Phe Ser Tyr Leu Arg Glu Ser
   65                  70                  75                  80
   Gly Trp Tyr Trp Gly Ser Ile Thr Ala Ser Glu Ala Arg Gln His Leu
                   85                  90                  95
   Gln Lys Met Pro Glu Gly Thr Phe Leu Val Arg Asp Ser Thr His Pro
               100                 105                 110
   Ser Tyr Leu Phe Thr Leu Ser Val Lys Thr Thr Arg Gly Pro Thr Asn
           115                 120                 125
   Val Arg Ile Glu Tyr Ala Asp Ser Ser Phe Arg Leu Asp Ser Asn Cys
       130                 135                 140
   Leu Ser Arg Pro Arg Ile Leu Ala Phe Pro Asp Val Val Ser Leu Val
   145                 150                 155                 160
   Gln His Tyr Val Ala Ser Cys Thr Ala Asp Thr Arg Ser Asp Ser Pro
                   165                 170                 175
   Asp Pro Ala Pro Thr Pro Ala Leu Pro Met Pro Lys Glu Asp Ala Pro
               180                 185                 190
   Ser Asp Pro Ala Leu Pro Ala Pro Pro Pro Ala Thr Ala Val His Leu
           195                 200                 205
   Lys Leu Val Gln Pro Phe Val Arg Arg Ser Ser Ala Arg Ser Leu Gln
       210                 215                 220
   His Leu Cys Arg Leu Val Ile Asn Arg Leu Val Ala Asp Val Asp Cys
   225                 230                 235                 240
   Leu Pro Leu Pro Arg Arg Met Ala Asp Tyr Leu Arg Gln Tyr Pro Phe
                   245                 250                 255
   Gln Leu
<210> 8
   <211> 1652
   <212> **DNA**
   <213> Homo sapiens
<400> 8
   cttgccgtca gccttttctt tgacctcttc tttctcttca tgtgtatttg ctgtctctta      60
   gcccagactt cccgtgtcct ttccaccggg cctttgagag gtcacagggt cttgatgctg     120
   tggtcttgat ctgcaggtgt ctgacttcca gcaactgctg gcctgtgcca gggtgcaagc     180
   tgagcactgg agtggagttt tcctgtggag aggagccatg cctagagtgg gatgggccat     240
   tgttcatctt ctggcccctg ttgtctgcat gtaacttaat accacaacca ggcatacggg     300
   aaagattgga ggaaagatga gtgagagcat caacttctct gacaacctag gccagtgtgt     360
   ggtgatgcca ggcatgccct tccccagcat caggtctcca gagctgcaga agacgacggc     420
   cgacttggat cacactcttg tgagtgtccc cagtgttgca gaggcagggc catcaggcac     480
   caaagggatt ctgccagcat agtgctcctg gaccagtgat acacccggca ccctgtcctg     540
   gacaggctgt tggcctgaat ctgagccctc gtggaggtca aagcaacctt tggttctgcc     600
   attgctgctg tgtggaagtt cactcctgcc ttttcctttc cctagagcct ccaccacccc     660
   gagatcacat ttctcactgc cttttgtctg cccagtttca ccagaagtag gcctcttcct     720
   gacaggcagc tgcaccactg cctggcgctg cgcccttcct ttgctctgcc cgctggagac     780
   ggtgtttgtc atgggcctgg tctgcaggga tcctgctaca aaggtgaaac ccaggagagt     840
   gtggaatcca gagtgttgcc aggacccagg cacaggcatt agtgcccgtt ggagaaaaca     900
   ggggaatccc gaagaaatgg tgggtcctgg ccatccgtga gatcttccca gggcagctcc     960
   cctctgtgga atccaatctg tcttccatcc tgcgtggccg agggccaggc ttctcactgg 1020
   gcctctgcag gaggctgcca tttgtcctgc ccaccttctt agaagcgaga cggagcagac 1080
   ccatctgcta ctgccctttc tataataact aaagttagct gccctggact attcaccccc 1140
   tagtctccat ttaaaaagat ccccatggcc acagggcccc tgcctggggg cttgtcacct 1200
   cccccacctt cttcctgagt cactcctgca gccttgctcc ctaacctgcc ccacagcctt 1260
   gcctggattt ctatctccct ggcttggtgc cagttcctcc aagtcgatgg cacctccctc 1320
   cctctcaacc acgtgagcaa actccaagac atcttctacc ccaacaccag caattgtgcc 1380
   aagggccatt aggctctcag catgactatt tttagagacc ccgtgtctgt cactgaaacc 1440
   ttttttgtgg gagactattc ctcccatctg caacagctgc ccctgctgac tgcccttctc 1500
   tcctccctct catcccagag aaacaggtca gctgggagct tctgccccca ctgcctaggg 1560
   accaacaggg gcaggaggca gtcactgacc ccgagacgtt tgcatcctgc acagctagag 1620
   gtcctttatt aaaagcacac tgttggtttc tg 1652
<210> 9
   <211> 76
   <212> **PRT**
   <213> Homo sapiens
<400> 9
   Met Thr Ile Phe Arg Asp Pro Val Ser Val Thr Glu Thr Phe Phe Val
   1               5                   10                  15
   Gly Asp Tyr Ser Ser His Leu Gln Gln Leu Pro Leu Leu Thr Ala Leu
               20                  25                  30
   Leu Ser Ser Leu Ser Ser Gln Arg Asn Arg Ser Ala Gly Ser Phe Cys
           35                  40                  45
   Pro His Cys Leu Gly Thr Asn Arg Gly Arg Arg Gln Ser Leu Thr Pro
       50                  55                  60
   Arg Arg Leu His Pro Ala Gln Leu Glu Val Leu Tyr
   65                  70                  75
<210> 10
   <211> 3975
   <212> DNA
   <213> Homo sapiens
<400> 10
   aagggacatg aagagaataa agaaagaaag aagaaaagaa aagaaaaaag aaaagaaaag 60
   ggagggctct gcgggtggcg gcggcgcggg gagccggttg caggccgaga tgctgcagat 120
   ggacctgatc gacgcgacgg gggacactcc cggggccgag gacgacgagg aggacgacga 180
   cgaggagcgc gcggcccggc ggccgggagc ggggccgccc aaggccgagt ccggccagga 240
   gccggcgtcc cgcggccagg gccagagcca aggccagagc cagggcccgg gcagcgggga 300
   cacgtaccgg cccaagcggc ccaccacgct caacctcttt ccgcaggtgc agttgtctca 360
   ggacacactg aataataatt ctctgggcaa aaagtacagt tggcaggatc gggtgtctcg 420
   atcatcctca cccctgaaga caggggagca gacaccaccg catgaaccca tttgcctgag 480
   cgatgagctg cccccccaga gcagccccgc ccccaccaca gatcgaggca cctccaccaa 540
   cagcccacgc tgctggtaga tgagcacgcg cagctggagc tggtgagcct gcggccgtgc 600
   ttcggagact acagtgacga gagtgactcg gccatcgtct acgacaactg tgtctccgtc 660
   tcctcgccct atgagtcagc catcggagag gaatatgagg aggcctcccg gccccagcct 720
   cctgcctgcc tctccaagga ctccacgcct gacgaacccg acgtccattt ctccaagaag 780
   ttcctgaaca tcttcatgag tggccgctcc cgctcctcca gtgccgagtc cttcgggctg 840
   ttctcctgca tcatcaaccg ggaggagcag gagcagaccc accggaccat attcaggttt 900
   gtgcctcgac acgaagacga acctgagctg gaagtggatg accctctgct agtggagctc 960
   caggctgaag actactggta cgaggcctac aacatgcgca ctggtgcccg gggcttcttt 1020
   actgcctatt acgccatcga agtcaccaag gagcccgagc acatggcagc cctggctaaa 1080
   aacagtgact gggtggacca gttccgggtg aagttcctgg gctcagtcca ggttccctat 1140
   cacaagggcg atgtcgtcct ctctgccgct atgcaaaaga ttgccaccac ccgccggcta 1200
   accgtgcact ttaacccgcc ctccagctgt gtcctggaga tcagcgtgcg gggtgtgaag 1260
   ataggtgtca aggccgatga ctcccaggag gccaagggga ataaatgtag ccactttttc 1320
   cagttaaaaa acatctcttt ccgcggatat catccaaaga acaacaagta ctttgggttc 1380
   atcaccaagc acctcgccga ccaccggttt gcctgccacg tctttgtgtc tgaagactcc 1440
   accaaagccc tggcagagtc cgtggggaga gcattccagc agtttcacaa gcagtttgtg 1500
   gagtacacct gccccacaga agatatctac ctggagtagc cgcgcagccc cgccctctgc 1560
   gtcccccggc cctcaggcca gtgccaggac agctggctgc tgacaggatg tggcactgct 1620
   tgaggagggg cacctgccac cgccagggga tgaggaagtg ggggccgctg gctcagggta 1680
   ggggagggtg gggcaatggg gacaggcaaa tgcagtttat tgtaatatat gggattagat 1740
   tcatctatgg agggcagagt gggctgcctg gggattggga gggacagggc ttggggagca 1800
   ggtctctggc agagaaggat gtccgttcca ggagcacacg gccctgcccc atcctgggcc 1860
   atacctcccc tgccagggct cgggtgctct ggctcctgcc ttgatgaagc ccatgtcctg 1920
   ccttgatgaa gcctgtgcta cctgcaagtg cccgccctgc ccctgtccca acacccaccg 1980
   aagagccctg agctcaggct gagcccagcc acctcccaag gactttccag tgaggaaatg 2040
   gcaacacgtg gaagtgaagt ccctgttctc agctctgtca tctgcggggc ttctgggtgg 2100
   ctcctgccac cgacctcact ggcatgctag cctgtggcag gcctaggacc tcagcgggga 2160
   ggaggagctg ccgcaaggcc ctatctcagc aggagaggga ggcttcctga ctgacacagg 2220
   ctagccccat cttggtcctg tcaccctggc cccaactatt aaagtgccat ttcctgtcaa 2280
   aaaaaaaaaa aaaaaaagaa aagggaggga gggagggagg gaggcaggaa ggaaggaaga 2340
   gagggaggga gggaagggag gaagaaaggg aaagaaggga gggagggaaa tagaaagaaa 2400
   gaaggaaaga aaaagaaaga aagagaaata aaataaaaat taaaaaacca taaggttaaa 2460
   gtaaaccctt tttcttcata cagattaaaa cacatgactt caaattacag ctttgcttct 2520
   taatagcttg gtgatgtagg atgttatgta acctctctgt gcctcagttt cctcatttat 2580
   aaaatagggc aataataata tctagcccat aaggcattgt gaggattaaa tgtgaaatgc 2640
   tgatcacaaa tacctagcag cccaatagat actcactgta ataattatta tttttataat 2700
   ttctgcaaaa gtatggtgat gattcttggg ttaacctaaa ggcagatttt cttttatttc 2760
   ttcctgtttc ttttcttttc cttgttcact ttaaagaatt aaaaagaaaa ttgattccag 2820
   cattttggaa taaaaatttg catcaaaaag aatttattca ttttattgac atacaaataa 2880
   aatgtcattt gtttattcaa taaacattta ttaaatgtct ggtaaatttc agacatcatg 2940
   ccaggcacag ggatgacaat gacaataaga tgtggtctct gccctcaggg agctgatagt 3000
   ccaggagact gacaagtaga caggtgatta catgcaatgt aacaaaggct atgatgtcat 3060
   acaagaagac aagtgggagt atgtgatggg agtatggttt tgaccagttc ctcctcttag 3120
   atttatccct ttttctttgg ctataaagca aaagaattgg tcctgttttt ttttcttaac 3180
   tttgcaaatt aaaccataaa ttttaataac tttataaaga taaaaggcaa gcggtcagat 3240
   tcagtggctc acacctataa tcccaacact ttgggaggcc gaggcaggtg gataacctga 3300
   ggtcaggagt tcgagaccag cctggccaac atcgtgaaac cctgtctcta ctaaaaatac 3360
   aaaaattagc tagttgtggt ggcaggcacc tgtaatccca gctactcagg aggctgaggc 3420
   aggagaatcg cttgaacctg ggaggcggag tttgcagtga gatgagatgg agccattgct 3480
   ctccagcctg ggctacagag caagactctg tctcaaaaca aaacaaaaca aaacaaaaag 3540
   atgagcaact tgaattatgg aggacactag aaatagtgtt tcctacagaa tcagggcttc 3600
   ctaccaacat agtcacttct agggttttcg acctgaaaag ttctgtggca tattgtttct 3660
   ttgctatcca cttttttttc cctgtttttc cccctctttc tctcctctac tttatctcct 3720
   agagatctag gtagttccca aaggaataat gctttacgga gtctaatgtt gatttattag 3780
   gtaaaaacag aaaatgactt ttttttttac ccacaagttc cataccaaaa aatgaatgta 3840
   aacttcttat gcagtttcac acattgaaaa tgcaggttat tttaattcca ttgcattttt 3900
   cagaattctc aatcgcaatc ctctgacaac tgttgaagat ccgtatctct ttaaattact 3960
   ggcattaaaa tatct 3975
<210> 11
   <211> 1762
   <212> **DNA**
   <213> Homo sapiens
<400> 11
   cacgctgctg gtagatgagc acgcgcagct ggagccggtg agcctgcggc cgtgcttcgg      60
   agactacagt gacgagagtg actcggccat cgtctatgac aactgtgcct ccgtctcctc     120
   gccctatgag tcagccatcg gagaggaata tgaggaggcc tcccggcccc agcctcctgc     180
   ctgcctctcc aaggactcca cgcctgacga acccgacgtc catttctcca agaagttcct     240
   gaacatcttc atgagtggcc gctcccgctc ctccagtgcc gagtccttcg ggctgttctc     300
   ctgcatcatc aaccgggagg agcaggagca gacccaccgg accatattca ggtttgtgcc     360
   tcgacacgaa gacgaacctg agctggaagt ggatgaccct ctgctagtgg agctccaggc     420
   tgaagactac tggtacgagg cctacaacat gcgcactggt gcccggggct tctttactgc     480
   ctattacgcc atcgaagtca ccaaggagcc cgagcacatg gcagccctgg ctaaaaacag     540
   tgactgggtg gaccagttcc gggtgaagtt cctgggctca gtccaggttc cctatcacaa     600
   gggcgatgtc gtcctctctg ccgctatgca aaagattgcc accacccgcc ggctaaccgt     660
   gcactttaac ccgccctcca gctgtgtcct ggagatcagc gtgcggggtg tgaagatagg     720
   tgtcaaggcc gatgactccc aggaggccaa ggggaataaa tgtagccact ttttccagtt      780
   aaaaaacatc tctttccgcg gatatcatcc aaagaacaac aagtactttg ggttcatcac     840
   caagcacctc gccgaccacc ggtttgcctg ccacgtcttt gtgtctgaag actccaccaa     900
   agccctggca gagtccgtgg ggagagcatt ccagcagttt cacaagcagt ttgtggagta     960
   cacctgcccc acagaagata tctacctgga gtagccgcgc agccccgccc tctgcgtccc    1020
   ccggccctca ggccagtgcc aggacagctg gctgctgaca ggatgtggca ctgcttgagg    1080
   aggggcacct gccaccgcca ggggatgagg aagtgggggc cgctggctca gggtagggga    1140
   gggtggggca atggggagag gcaaatgcag tttattgtaa tatatgggat tagattcatc    1200
   tatggagggc agagtgggct gcctggggat tgggagggac agggcttggg gagcaggtct    1260
   ctggcagaga aggatgtccg ttccaggagc acacggccct gccccatcct gggccatacc    1320
   tcccctgcca gggctcgggt gctctggctc ctgccttgat gaagcccatg tcctgccttg    1380
   atgaagcctg tgccacctgc aagtgcccgc cctgccccgt cccaacaccc accgaagaac    1440
   cctgagctca ggctgagccc agccacctcc caaggacttt ccagtgagga aatggcaaca    1500
   catggaagtg aagtccctgt tctcagctct gtcatctgcg gggcttctgg gtggctcctg    1560
   ccaccgacct cactggcatg ctagcctgtg gcaggcctag gacctcagcg gggaggagga    1620
   gctgccgcaa ggccctatcc cagcaggaga gggaggcttc ctgactgaca caggctagcc    1680
   ccatcttggt cctgtcaccc tggccccaac tattaaagtg ccatttcctg tcaaaaaaaa    1740
   aaaaaaaaaa aagaaaagaa aa                                             1762
<210> 12
   <211> 3995
   <212> DNA
   <213> Homo sapiens
<400> 12
   ggcggttgca cgcctgcgcg agggcgggcg gcgggggcgc gcgcgcggcg ggggcgggct      60
   ttgccgagcg cagagctgca gccgccgagc cggacgtgtc cgcgaagatg gcgggccgga     120
   gcatgcaagc ggcaagatgt cctacagatg aattatcttt aaccaattgt gcagttgtga     180
   atgaaaagga tttccagtct ggccagcatg tgattgtgag gacctctccc aatcacaggt     240
   acacatttac actgaagaca catccatcgg tggttccagg gagcattgca ttcagtttac     300
   ctcagagaaa atgggctggg ctttctattg ggcaagaaat agaagtctcc ttatatacat     360
   ttgacaaagc caaacagtgt attggcacaa tgaccatcga gattgatttc ctgcagaaaa     420
   aaagcattga ctccaaccct tatgacaccg acaagatggc agcagaattt attcagcaat     480
   tcaacaacca ggccttctca gtgggacaac agcttgtctt tagcttcaat gaaaagcttt     540
   ttggcttact ggtgaaggac attgaagcca tggatcctag catcctgaag ggagagcctg     600
   cgacagggaa aaggcagaag attgaagtag gactggttgt tggaaacagt caagttgcat     660
   ttgaaaaagc agaaaattcg tcacttaatc ttattggcaa agctaaaacc aaggaaaatc     720
   gccaatcaat tatcaatcct gactggaact ttgaaaaaat gggaatagga ggtctagaca     780
   aggaattttc agatattttc cgacgagcat ttgcttcccg agtatttcct ccagagattg     840
   tggagcagat gggttgtaaa catgttaaag gcatcctgtt atatggaccc ccaggttgtg     900
   gtaagactct cttggctcga cagattggca agatgttgaa tgcaagagag cccaaagtgg     960
   tcaatgggcc agaaatcctt aacaaatatg tgggagaatc agaggctaac attcgcaaac    1020
   tttttgctga tgctgaagag gagcaaagga ggcttggtgc taacagtggt ttgcacatca    1080
   tcatctttga tgaaattgat gccatctgca agcagagagg gagcatggct ggtagcacgg    1140
   gagttcatga cactgttgtc aaccagttgc tgtccaaaat tgatggcgtg gagcagctaa    1200
   acaacatcct agtcattgga atgaccaata gaccagatct gatagatgag gctcttctta    1260
   gacctggaag actggaagtt aaaatggaga taggcttgcc agatgagaaa ggccgactac    1320
   agattcttca catccacaca gcaagaatga gagggcatca gttactctct gctgatgtag    1380
   acattaaaga actggccgtg gagaccaaga atttcagtgg tgctgaattg gagggtctgg    1440
   tgcgagcagc ccagtccact gctatgaata gacacataaa ggccagtact aaagtggaag    1500
   tggacatgga gaaagcagaa agcctgcaag tgacgagagg agacttcctt gcttctttgg     1560
   agaatgatat caaaccagcc tttggcacaa accaagaaga ttatgcaagt tacattatga     1620
   acggtatcat caaatggggt gacccagtta ctcgagttct agatgatggg gagctgctgg     1680
   tgcagcagac taagaacagt gaccgcacac cattggtcag cgtgcttctg gaaggccctc     1740
   ctcacagtgg gaagactgct ttagctgcaa aaattgcaga ggaatccaac ttcccgttca     1800
   tcaagatctg ttctcctgat aaaatgattg gcttttctga aacagccaaa tgtcaggcca     1860
   tgaagaagat ctttgatgat gcgtacaaat cccagctcag ttgtgtggtt gtggatgaca     1920
   ttgagagatt gcttgattac gtccctattg gccctcgatt ttcaaatctt gtattacagg     1980
   ctcttctcgt tttactgaaa aaggcacctc ctcagggccg caagcttctt atcattggga     2040
   ccactagccg caaagatgtc cttcaggaga tggaaatgct taacgctttc agcaccacca     2100
   tccacgtgcc caacattgcc acaggagagc agctgttgga agctttggag cttttgggca     2160
   acttcaagga taaggaacgc accacaattg cacagcaagt caaagggaag aaggtctgga     2220
   taggaatcaa gaagttacta atgctgatcg agatgtccct acagatggat cctgaatacc     2280
   gtgtgagaaa attcttggcc ctcttaagag aagaaggagc tagccccctt gattttgatt     2340
   gaaaatgaac tatttgaaac acacagtgac caagggaagt gaccaaggtg aagatggcct     2400
   aggatcttca ctgtcttact caagatactg gactaagtgg aacgttctct accttcaaca     2460
   tgtgctcgct ctgcatgatt agtgcaataa aactcccttc cttatgcata ctgagatagc     2520
   ttagtgtctc gtggaaggtg tcaatttggt ttagaatgct gcgcttacct tcccatgcag     2580
   gctaaagtga ttccttcttg ctcagtccct ctgggtggga accatccagt acttgtggac     2640
   actacacgtt tcaacctctc tactagcacc atcacccttg aaaactctca gtcagtgtca     2700
   tgaatgttgc atgacaacag ttggccgatt agaaggcaga ctttctacat gcaaatctgg     2760
   cttagtaaat cgaggtgtgg gccagagatc ctctgacagc tgtcctgagc taacactaaa     2820
   agtcactggg tatttggtta aaggtctccc acaagactgg tattctcttt gcctgaagaa     2880
   acaaggcatt gaatctctaa aatgctgttc tcaatcattg tcagagatgt tttcaagttg     2940
   cagtcagaag atctttctta atagaaagtc agatgactac cgtgttggtt gtgacttccc     3000
   cttaagtata actaatttgc tctgtggtaa gagatatgct cattattacc acttagaaga     3060
   tgttgttaaa aacatgtgaa agataggtat ggaaaaagca tacaccccca aacagaaagg     3120
   agttattaaa gtaatttaca aacctctcag cactaattag tgtccaactc caagtgggtc     3180
   aattccttag tataatatta aggcttacta gtatcactgc tttttcctta gcttaatgac     3240
   ttacttagaa tttatccttt attttaaatg atctgtacta tctagtgtct aaaacactat     3300
   tctccagaaa aatcaatcat tttctagccc tctccctcag tcctttattg tccattccaa     3360
   tacattgaac acatttcctt taccctccac acacttcttc caaaaggaag cacccgttga     3420
   gtccttttga gggtgatttg tcttacaact gactgactta gcaggaattt aattaggtca     3480
   tatttggtga tgagacttat ggagtgtgcc tctctctccc aactgctgct taaaatgcaa     3540
   ggacaagcaa ttagaagcca tcctaaggtg cttacctcac acgccaccca tgaggcttgt     3600
   ggccacagtg gcacttgggt gtggctcctc tgttatttgt cctcatgtga gaaagcagat     3660
   catctccaaa tcttgccatt tgtatacttt tggtggagac ttggatgtca tatcttcttt     3720
   gttttgggtt ttcttcccta gcttattttg tggcttttaa agaagtggat tgtattgtga     3780
   gatcctgtga ttcctggtgg ccagtatcct ggattcctct aagatcttgc ctctttcctc     3840
   ctcatgaaag cagcacacat tgtgttaact tatgtctctt gttaaatgag cttaatgtct     3900
   ttgtgttttg tccaaaactg tattgaaaaa atattgttta atgcaaatga aggaatgcaa     3960
   taaagagtaa atatacttga aaaaaaaaaa aaaaa                                3995
<210> 13
   <211> 326
   <212> PRT
   <213> Homo sapiens
<400> 13
   Leu Leu Ser Ala Asp Val Asp Ile Lys Glu Leu Ala Val Glu Thr Lys
   1               5                   10                  15
   Asn Phe Ser Gly Ala Glu Leu Glu Gly Leu Val Arg Ala Ala Gln Ser
               20                  25                  30
   Thr Ala Met Asn Arg His Ile Lys Ala Ser Thr Lys Val Glu Val Asp
           35                  40                  45
   Met Glu Lys Ala Glu Ser Leu Gln Val Thr Arg Gly Asp Phe Leu Ala
       50                  55                  60
   Ser Leu Glu Asn Asp Ile Lys Pro Ala Phe Gly Thr Asn Gln Glu Asp
   65                  70                  75                  80
   Tyr Ala Ser Tyr Ile Met Asn Gly Ile Ile Lys Trp Gly Asp Pro Val
                   85                  90                  95
   Thr Arg Val Leu Asp Asp Gly Glu Leu Leu Val Gln Gln Thr Lys Asn
               100                 105                 110
   Ser Asp Arg Thr Pro Leu Val Ser Val Leu Leu Glu Gly Pro Pro His
           115                 120                 125
   Ser Gly Lys Thr Ala Leu Ala Ala Lys Ile Ala Glu Glu Ser Asn Phe
       130                 135                 140
   Pro Phe Ile Lys Ile Cys Ser Pro Asp Lys Met Ile Gly Phe Ser Glu
   145                 150                 155                 160
   Thr Ala Lys Cys Gln Ala Met Lys Lys Ile Phe Asp Asp Ala Tyr Lys
                   165                 170                 175
   Ser Gln Leu Ser Cys Val Val Val Asp Asp Ile Glu Arg Leu Leu Asp
               180                 185                 190
   Tyr Val Pro Ile Gly Pro Arg Phe Ser Asn Leu Val Leu Gln Ala Leu
           195                 200                 205
   Leu Val Leu Leu Lys Lys Ala Pro Pro Gln Gly Arg Lys Leu Leu Ile
       210                 215                 220
   Ile Gly Thr Thr Ser Arg Lys Asp Val Leu Gln Glu Met Glu Met Leu
   225                 230                 235                 240
   Asn Ala Phe Ser Thr Thr Ile His Val Pro Asn Ile Ala Thr Gly Glu
                   245                 250                 255
   Gln Leu Leu Glu Ala Leu Glu Leu Leu Gly Asn Phe Lys Asp Lys Glu
               260                 265                 270
   Arg Thr Thr Ile Ala Gln Gln Val Lys Gly Lys Lys Val Trp Ile Gly
           275                 280                 285
   Ile Lys Lys Leu Leu Met Leu Ile Glu Met Ser Leu Gln Met Asp Pro
       290                 295                 300
   Glu Tyr Arg Val Arg Lys Phe Leu Ala Leu Leu Arg Glu Glu Gly Ala
   305                 310                 315                 320
   Ser Pro Leu Asp Phe Asp
                   325
<210> 14
   <211> 1590
   <212> **DNA**
   <213> Homo sapiens
<400> **14**
   ccggtgtcgg gaagcaccag tgccctgagg aagggccatt tccaaaagcc ctgtgctgac        60
   acagggttgc tggttcctct tcaagagccc actctctggg gtggggccat atctccagca       120
   gaggtgggct ggaaaggacc cccccaatcc cgcccgccgt gagcttagct ggagccatgg       180
   cctctgcacc catctcgatg cttgcgatgc tcttcacatt gagtgggctg agagctcagt       240
   cagtggctca gccggaagat caggtcaacg ttgctgaagg gaatcctctg actgtgaaat       300
   gcacctattc agtctctgga aacccttatc ttttttggta tgttcaatac cccaaccgag        360
   gcctccagtt ccttctgaaa tacatcacag gggataacct ggttaaaggc agctatggct       420
   ttgaagctga atttaacaag agccaaacct ccttccacct gaagaaacca tctgcccttg       480
   tgagcgactc cgctttgtac ttctgtgctg tgagagacgc caaagctgca ggcaacaagc       540
   taacttttgg aggaggaacc agggtgctag ttaaaccaaa tatccagaac cctgaccctg       600
   ccgtgtacca gctgagagac tctaaatcca gtgacaagtc tgtctgccta ttcaccgatt       660
   ttgattctca aacaaatgtg tcacaaagta aggattctga tgtgtatatc acagacaaaa       720
   ctgtgctaga catgaggtct atggacttca agagcaacag tgctgtggcc tggagcaaca       780
   aatctgactt tgcatgtgca aacgccttca acaacagcat tattccagaa gacaccttct       840
   tccccagccc agaaagttcc tgtgatgtca agctggtcga gaaaagcttt gaaacagata       900
   cgaacctaaa ctttcaaaac ctgtcagtga ttgggttccg aatcctcctc ctgaaagtgg       960
   ccgggtttaa tctgctcatg acgctgcggc tgtggtccag ctgagatctg caagattgta      1020
   agacagcctg tgctccctcg ctccttcctc tgcattgccc ctcttctccc tctccaaaca      1080
   gagggaactc tcctaccccc aaggaggtga aagctgctac cacctctgtg cccccccggc      1140
   aatgccacca actggatcct acccgaattt atgattaaga ttgctgaaga gctgccaaac      1200
   actgctgcca ccccctctgt tcccttattg ctgcttgtca ctgcctgaca ttcacggcag      1260
   aggcaaggct gctgcagcct cccctggctg tgcacattcc ctcctgctcc ccagagactg      1320
   cctccgccat cccacagatg atggatcttc agtgggttct cttgggctct aggtcctgca      1380
   gaatgttgtg aggggtttat ttttttttaa tagtgttcat aaagaaatac atagtattct      1440
   tcttctcaag acgtgggggg aaattatctc attatcgagg ccctgctatg ctgtgtatct      1500
   gggcgtgttg tatgtcctgc tgccgatgcc ttcattaaaa tgatttggaa gagcaaaaaa      1560
   aaaaaaaaaa aaaaaaaaaa aaaaaaaaaa                                       1590
<210> 15
   <211> 20
   <212> PRT
   <213> Homo sapiens
<400> 15
   Ile Lys Ala Ala Gly Asn Lys Leu Thr Phe Gly Gly Gly Thr Arg Val
   1               5                   10                  15
   Leu Val Lys Pro
               20
<210> 16
   <211> 1001
   <212> **DNA**
   <213> Homo sapiens
<400> 16
   aggcacttct atgccatttt agctattaga ttgaatcacc tctgggctcc ctttcaaatc      60
   taaatgctaa gatcccatga ctcaagcctg gaggattaat aggtgagctc agacttgttt     120
   cttctatttt tactattttg atagccaaag aaataattca tcaaattatt cataagctaa     180
   agcctacttg ggatttttac acctagaaga tggtggggta tgatttccca gtgcagtaaa     240
   tgagaaaaca ataggagaca tcaaggagga aaaaaagaag gaagagataa agggaatgtc     300
   ttaagggagg ctcagaggtt gaatgaagga aatgaggtga ttttgcagag gacagatgtg     360
   gctatcaaag attttacaat ttcacctttg gaaagggatc caaacataat gtcactccaa     420
   gtaagtgagc agccttttgt actcgaaaat agggccaggg gagcaaagtt tcttccaatt     480
   taaacacact caaaaggatg tgtaattgct ttctgatggg agggaactct gaaggtagaa     540
   agactattgt taccacagat cacttgtccc tggagatata gcatctgagg actgactgtc     600
   tagcttagac tgctgtctgc acaagaagaa gacttacaga tgcttaagga ggatggaggc     660
   aaattttcaa ccctgtactc caaagctgag gggagagggg atgggaaaca ttagggctgg     720
   gttcatgtaa aggggaccag cattgtgccg acagaggctc aaccctgggg aggctatact     780
   ttggaagagg aactcagttg actgtctggc ctggtgagtg agtcgctttc tattccagga     840
   aaatattact gtggagaaat taaaagggga gatgaattaa ctcctttagt cttgaaacta     900
   aagagatatg tgtacttccc ctcctggagg accccagtcc ctaggtagat taggacgagg     960
   aagcagaggg agacaaaggc gatggaaagt cccttttagg a                        1001
<210> 17
   <211> 19
   <212> **PRT**
   <213> Homo sapiens
<400> 17
   Tyr Gln Arg Phe Tyr Asn Phe Thr Phe Gly Lys Gly Ser Lys His Asn
   1               5                   10                  15
   Val Thr Pro
<210> 18
   <211> 570
   <212> DNA
   <213> Homo sapiens
<400> 18
   tcagtttctt cttcctgcag ctggttgagt tctttccaga caaagacaag tgacaagaat      60
   tagaggttta aaaagcaacc agattcatct cagcagcttt tgtagtttta aataagcaag     120
   gagtttctcc agcgaaactt cctcacacct cttggtcttg gtctcttcag acactttcct     180
   tcctgttctc tggagatctt gcagaaaaga gcctgcagtg tttcccttgc tcagccatgc     240
   tcctggagct tatcccactg ctggggatac attttgtcct gagaactgcc agagcccagt     300
   cagtgaccca gcctgacatc cacatcactg tctctgaagg agcctcactg gagttgagat     360
   gtaactattc ctatggggca acaccttatc tcttctggta tgtccagtcc cccggccaag     420
   gcctccagct gctcctgaag tacttttcag gagacactct ggttcaaggc attaaaggct     480
   ttgaggctga atttaagagg agtcaatctt ccttcaacct gaggaaaccc tctgtgcatt     540
   ggagtgatgc tgctgagtac ttctgtgctg                                      570
<210> 19
   <211> 113
   <212> **PRT**
   <213> Homo sapiens
<400> 19
   Met Leu Leu Glu Leu Ile Pro Leu Leu Gly Ile His Phe Val Leu Arg
   1               5                   10                  15
   Thr Ala Arg Ala Gln Ser Val Thr Gln Pro Asp Ile His Ile Thr Val
               20                  25                  30
   Ser Glu Gly Ala Ser Leu Glu Leu Arg Cys Asn Tyr Ser Tyr Gly Ala
           35                  40                  45
   Thr Pro Tyr Leu Phe Trp Tyr Val Gln Ser Pro Gly Gln Gly Leu Gln
       50                  55                  60
   Leu Leu Leu Lys Tyr Phe Ser Gly Asp Thr Leu Val Gln Gly Ile Lys
   65                  70                  75                  80
   Gly Phe Glu Ala Glu Phe Lys Arg Ser Gln Ser Ser Phe Asn Leu Arg
                   85                  90                  95
   Lys Pro Ser Val His Trp Ser Asp Ala Ala Glu Tyr Phe Cys Ala Val
               100                 105                 110
   Gly
<210> 20
   <211> 1834
   <212> **DNA**
   <213> Homo sapiens
<400> 20
   actttgacag tcttctcatg ctgcctctgc caccttctct gccagaagat accatttcaa        60
   ctttaacaca gcatgatcga aacatacaac caaacttctc cccgatctgc ggccactgga       120
   ctgcccatca gcatgaaaat ttttatgtat ttacttactg tttttcttat cacccagatg       180
   attgggtcag cactttttgc tgtgtatctt catagaaggt tggacaagat agaagatgaa       240
   aggaatcttc atgaagattt tgtattcatg aaaacgatac agagatgcaa cacaggagaa       300
   agatccttat ccttactgaa ctgtgaggag attaaaagcc agtttgaagg ctttgtgaag       360
   gatataatgt taaacaaaga ggagacgaag aaagaaaaca gctttgaaat gcaaaaaggt       420
   gatcagaatc ctcaaattgc ggcacatgtc ataagtgagg ccagcagtaa aacaacatct       480
   gtgttacagt gggctgaaaa aggatactac accatgagca acaacttggt aaccctggaa       540
   aatgggaaac agctgaccgt taaaagacaa ggactctatt atatctatgc ccaagtcacc       600
   ttctgttcca atcgggaagc ttcgagtcaa gctccattta tagccagcct ctgcctaaag       660
   tcccccggta gattcgagag aatcttactc agagctgcaa atacccacag ttccgccaaa       720
   ccttgcgggc aacaatccat tcacttggga ggagtatttg aattgcaacc aggtgcttcg       780
   gtgtttgtca atgtgactga tccaagccaa gtgagccatg gcactggctt cacgtccttt       840
   ggcttactca aactctgaac agtgtcacct tgcaggctgt ggtggagctg acgctgggag       900
   tcttcataat acagcacagc ggttaagccc accccctgtt aactgcctat ttataaccct       960
   aggatcctcc ttatggagaa ctatttatta tacactccaa ggcatgtaga actgtaataa      1020
   gtgaattaca ggtcacatga aaccaaaacg ggccctgctc cataagagct tatatatctg      1080
   aagcagcaac cccactgatg cagacatcca gagagtccta tgaaaagaca aggccattat      1140
   gcacaggttg aattctgagt aaacagcaga taacttgcca agttcagttt tgtttctttg      1200
   cgtgcagtgt ctttccatgg ataatgcatt tgatttatca gtgaagatgc agaagggaaa      1260
   tggggagcct cagctcacat tcagttatgg ttgactctgg gttcctatgg ccttgttgga      1320
   gggggccagg ctctagaacg tctaacacag tggagaaccg aaaccccccc cccccccccg      1380
   ccaccctctc ggacagttat tcattctctt tcaatctctc tctctccatc tctctctttc      1440
   agtctctctc tctcaacctc tttcttccaa tctctctttc tcaatctctc tgtttccctt      1500
   tgtcagtctc ttccctcccc cagtctctct tctcaatccc cctttctaac acacacacac      1560
   acacacacac acacacacac acacacacac acacacacac agagtcaggc cgttgctagt      1620
   cagttctctt ctttccaccc tgtccctatc tctaccacta tagatgaggg tgaggagtag      1680
   ggagtgcagc cctgagcctg cccactcctc attacgaaat gactgtattt aaaggaaatc      1740
   tattgtatct acctgcagtc tccattgttt ccagagtgaa cttgtaatta tcttgttatt      1800
   tattttttga ataataaaga cctcttaaca ttaa                                 1834
<210> 21
   <211> 124
   <212> **PRT**
   <213> Homo sapiens
<400> 21
   Leu Gln Trp Ala Glu Lys Gly Tyr Tyr Thr Met Ser Asn Asn Leu Val
   1               5                   10                  15
   Thr Leu Glu Asn Gly Lys Gln Leu Thr Val Lys Arg Gln Val Leu Tyr
               20                  25                  30
   Tyr Ile Tyr Ala Gln Val Thr Phe Cys Ser Asn Arg Glu Ala Ser Ser
           35                  40                  45
   Gln Ala Pro Phe Ile Ala Ser Leu Cys Leu Lys Ser Pro Gly Arg Phe
       50                  55                  60
   Glu Arg Ile Leu Leu Arg Ala Ala Asn Thr His Ser Ser Ala Lys Pro
   65                  70                  75                  80
   Cys Gly Gln Gln Ser Ile His Leu Gly Gly Val Phe Glu Leu Gln Pro
                   85                  90                  95
   Gly Ala Ser Val Phe Val Asn Val Thr Asp Pro Ser Gln Val Ser His
               100                 105                 110
   Gly Thr Gly Phe Thr Ser Phe Gly Leu Leu Lys Leu
           115                 120
<210> 22
   <211> 3684
   <212> DNA
   <213> Homo sapiens
<400> 22
   aggatgttgc ctgcggctgg cggcccagtg gattctggga attgtagtcc cagccatcca        60
   gggcattgcc gttcagggcc acgggaaaac ctgactgcgc tcccagaagc ctccggtgta       120
   cctcgctggg aacgcacttc ctgggacgct gagagggaga cgctccaaga ggctcctcag       180
   tgtgggcgag taaaatgccc tgcgtgtgag aagcaggctc agattattgc tactctgtat       240
   tcagatcttc atgtgtctct tctaagctga ataaagctgt tttggaactg tcaggtacct       300
   caaagcatga agaaaacgag gcacagggaa gttctgaatc tggcccaagt ctgactgaca       360
   gtactccaca agcttgcctg ccatgggctg tcgggatgtc cacgcagcca cagtcctttc       420
   cttcctgtgt ggaatcgcct cagtagcagg cctctttgca gggactctgc ttcccaactg       480
   gagaaaatta cgattgatca cattcaacag aaacgagaag aacctgactg tttacacagg     540
   cctgtgggtg aaatgtgccc ggtatgacgg gagcagtgac tgcctgatgt acgacactac     600
   ttggtactca tcagttgacc agctggacct gcgtgtcctc cagtttgccc tacccctcag     660
   catgctgatc gccatgggtg ccctgctgct ctgcctgatt ggaatgtgca acactgcctt     720
   caggtcctcg gtgcccaaca tcaaactggc caagtgtctg gtcaatagtg caggttgcca     780
   cctggtggct gggctgctat ttttcctggc aggtactgtg agcctctccc catctatctg     840
   ggtcatcttt tataacatcc atctgaacaa gaagtttgag ccagtctttt catttgacta     900
   tgcagtgtat gtcactattg ctagtgctgg gggcctgttt atgacttccc ttatactatt     960
   tatttggtat tgtacatgca aatctttgcc ttctcctttc tggcaaccat tgtactccca    1020
   tccacccagt atgcatactt actcacagcc ctattcagca cgctctcgcc tctctgccat    1080
   tgaaattgac attccagtag tttcacacac cacttaatgg ggaaatagtt aattgttaaa    1140
   gaaaacttct tgtagcctca cattcccctt gtgcaaagag ctcttttgga cctacataca    1200
   ttttcctttg tttttgacca atcaatgaag ccaaatttat atgtcctagt agaatgaagt    1260
   gctgctagtt tttatgagaa gtatattata ttaaatgtga attttttaaa ttttgcttct    1320
   tatactggaa ggaattttag ccttcatatt gatatctaat taattattta agtggaagag    1380
   gcctgcatca caattgaggt aatgtagagc aacatgttaa agaatgatgg ttagcagaag    1440
   ctgttgtata caatcttcat gaaaatttca gtgtgtattt ttctttttct ataatacctt    1500
   taactgcaaa gaaaaggcag tttcaaatat aagaaattta tttcaggtaa gggtaatatt    1560
   ttaatagtag tcaataatct agcttaaggc tgtaactctt ctatcggggc taattgtatg    1620
   aataggtgtc agtatgttga agattacttt cttttgtgac tttcttctac ctcatgccac    1680
   tgtttaaaag taaaacgtat tttaatgatg ttagaataag actaccattc taaatatcac    1740
   ctacttatga ataacatgta ataattttta acattaatga ttccataaat tgtattattg    1800
   ggattagaat gtgctttatg acaggttagt gtttcctctg aggcagaaaa ctcttttttg    1860
   gagatatctt ccatcaagca gtactcgtgc ccatatacaa tctcttagtg gctaggagaa    1920
   ataaataaaa gggccataat ggtttgttct ctttcagaca taatttagta ggggacaaga    1980
   agtctgttct tcagtgagta cactagagat ttactctggt gactgccttt tgagttatgg    2040
   gtgaagtaag gtatggcttt accataacct tgattcattc acccttgatt catttctcgc    2100
   ccccgtcact gattatttcc ttgagcatat atctctgcct aacactttag taggtgctat    2160
   agaggataca tgaaaagtat gagatctggt tccatccagt aagacatttt aatagagaag    2220
   atcaaaatgt tacctggcag ttggggaata atctgacttc gttggcagtt ggccttaact    2280
   tcttaatcat tgatccagga atatttcaac cagagacaca actttctggc agacagacaa    2340
   attgtacaac accaacaata tcctggacct tgaaattctg tttacttcag tccattgtat     2400
   cctttaaggc acctgtgcta gcctagattt tgtaataaca ctgatttatg agaatggaca     2460
   aaagtggtag ggaaattgtt ccctctccac ttctgaaagt atgatgatgt attaaggatg     2520
   gaggagttat taaaaatgtc tcttctgatg aggtaacaat tagatgaaac catgttaaag     2580
   ctgagatgaa cacttagaaa ttcagggata ttgggtcttt agccttatga atttgagctg     2640
   cttatttaat tggtgtaatt tactacatat tagtactata ttcgtaagga ttttttatta     2700
   accattacag attttacaaa cagctagtta tatggtaaac agattattat gcctttttgc     2760
   aattctgaat atgattctag tatttgtgta gatgtatttg gtactttttc ccctaattcc     2820
   aacactagtt tatatatata gcgaataaat ctagttgtat aaatttttaa atgccgtcag     2880
   tagaaagcac acaaggttat gattttttta attactggct tctgatttct ttcacttctg     2940
   atccttttcc tttttctcag atgtagctga gtcttgatca ttttaagaca acgatgggta     3000
   gaattttgag attaatgtta attttccctt tttgttaatt tcagtcccct ctcactatgc     3060
   ttttgtccag aaggatcaag aattctacca tcccttgggt ctttgtgtat aaacaatgtt     3120
   aaataaaggt agactcagtc tttaagatat tagacagttt ttttagtcca tgggattgta     3180
   aatataaaca ttaactttcc tataagaata ttttggcttt gtaatctata gcctcaaatt     3240
   ggtatttatt atggattcac tagacaaaca gctgtttcct tattgtcttt tttctttagt     3300
   gtttctgatt tgctatcagt agctgttttt aaagccgtcc aaggaaaata attatttaca     3360
   gtttttgaag tcacttttga gccctcatca agctctcatt gtgatgggag ggataccttt     3420
   ttgttgttaa aagcctatta ttgttaaagg ccttttatgg aaaccaactt ggaaaacaac     3480
   cttaaatgtg gatgtatcag atttggttta tccagccatg ggagagaaaa caaacctaag     3540
   tttactttac ttgtacatat acactacaat ggatagtata tttgctgtaa actacaatgt     3600
   aaaacctcaa taaaagtgcg ctgtacttct taatgtttat taaaagatgt atttttacaa     3660
   aaaaaaaaaa aaaaaaaaaa aaaa                                            3684
<210> 23
   <211> 244
   <212> PRT
   <213> Homo sapiens
<400> 23
   Met Gly Cys Arg Asp Val His Ala Ala Thr Val Leu Ser Phe Leu Cys
   1               5                   10                  15
   Gly Ile Ala Ser Val Ala Gly Leu Phe Ala Gly Thr Leu Leu Pro Asn
               20                  25                  30
   Trp Arg Lys Leu Arg Leu Ile Thr Phe Asn Arg Asn Glu Lys Asn Leu
               35                  40                  45
   Thr Val Tyr Thr Gly Leu Trp Val Lys Cys Ala Arg Tyr Asp Gly Ser
       50                  55                  60
   Ser Asp Cys Leu Met Tyr Asp Thr Thr Trp Tyr Ser Ser Val Asp Gln
   65                  70                  75                  80
   Leu Asp Leu Arg Val Leu Gln Phe Ala Leu Pro Leu Ser Met Leu Ile
                   85                  90                  95
   Ala Met Gly Ala Leu Leu Leu Cys Leu Ile Gly Met Cys Asn Thr Ala
               100                 105                 110
   Phe Arg Ser Ser Val Pro Asn Ile Lys Leu Ala Lys Cys Leu Val Asn
           115                 120                 125
   Ser Ala Gly Cys His Leu Val Ala Gly Leu Leu Phe Phe Leu Ala Gly
       130                 135                 140
   Thr Val Ser Leu Ser Pro Ser Ile Trp Val Ile Phe Tyr Asn Ile His
   145                 150                 155                 160
   Leu Asn Lys Lys Phe Glu Pro Val Phe Ser Phe Asp Tyr Ala Val Tyr
                   165                 170                 175
   Val Thr Ile Ala Ser Ala Gly Gly Leu Phe Met Thr Ser Leu Ile Leu
               180                 185                 190
   Phe Ile Trp Tyr Cys Thr Cys Lys Ser Leu Pro Ser Pro Phe Trp Gln
           195                 200                 205
   Pro Leu Tyr Ser His Pro Pro Ser Met His Thr Tyr Ser Gln Pro Tyr
       210                 215                 220
   Ser Ala Arg Ser Arg Leu Ser Ala Ile Glu Ile Asp Ile Pro Val Val
   225                 230                 235                 240
   Ser His Thr Thr
<210> 24
   <211> 5229
   <212> DNA
   <213> Homo sapiens
<400> 24
   agaaagctgc ggcgcgagtc cgcggggccg acctcggaga cgcagctggg gccgggcgcg 60
   gcttggcggg agggtctgca gcgccgaggg aggctgctgg tgcgtgagga agagagctag    120
   agactggaca ggggaggcag agcagcgtca gagccgcgca ggggacggga gtgagagcag    180
   gagcgacgca gagcggccgt cgccgtgccc gggtctcagg gcgcctggct gaagtgagca    240
   tggcttcagt ggcctgggcc gtcctcaagg tgctgctgct tctccccact cagacttgga    300
   gccccgtggg agcaggaaat ccacctgact gtgatgcccc actggcctct gccttgccta    360
   ggtcatcctt cagcagctcc tcagagctgt ccagcagcca cggcccgggg ttttcaaggc    420
   ttaatcgaag agatggagct ggtggctgga ctccacttgt gtcaaataaa taccaatggc    480
   tgcaaattga ccttggagag agaatggagg tcactgctgt cgccacccaa ggaggatatg    540
   ggagctctga ctgggtgacc agctacctcc tgatgttcag tgatggtggg agaaactgga    600
   agcagtatcg ccgagaagaa agcatctggg gttttccagg aaacacaaac gcagacagtg    660
   tggtgcacta cagactccag cctccctttg aagccaggtt cctgcgcttt ctccctttag    720
   cctggaaccc taggggcagg attgggatgc ggatcgaagt gtacggatgt gcatataaat    780
   ctgaggtggt ttattttgat ggacaaagtg ctctgctgta tagacttgat aaaaaacctt    840
   taaaaccaat aagagacgtt atttctttga aatttaaagc catgcagagc aatggaattc    900
   tacttcacag agaaggacaa catggaaatc acattactct ggaattaatt aaaggaaagc    960
   ttgtcttttt tcttaattca ggcaatgcta agctgccttc cactattgct cctgtgaccc   1020
   tcaccctggg cagcctgctg gacgaccagc actggcattc cgtcctcatc gagctcctcg   1080
   acacgcaggt caacttcacc gtggacaaac acactcatca tttccaagca aagggagatt   1140
   ccagttactt ggatcttaat tttgagatca gctttggggg aattccgaca cccggaagat   1200
   cgcgggcatt cagacgtaaa agctttcatg ggtgtttaga aaatctttat tataatggag   1260
   tggatgttac cgaattagcc aagaaacaca aaccacagat cctcatgatg ggaaatgtgt   1320
   ccttctcatg tccacagcca cagactgtcc ctgtgacttt tctgagctcc aggagttatc   1380
   tggctctgcc aggcaactct ggggaggaca aagtgtctgt cacttttcaa tttcgaacgt   1440
   ggaacagagc aggacatttg cttttcggcg aacttcgacg tggttcaggg agtttcgtcc   1500
   tctttcttaa ggatggcaag ctcaaactga gtctcttcca gccgggacag tcaccaagga   1560
   atgtcacagc aggtgctgga ttaaacgatg ggcagtggca ctctgtgtcc ttctctgcca   1620
   agtggagcca tatgaatgtg gtggtggacg atgacacagc tgttcagccc ctggtggctg   1680
   tgctcattga ttcaggtgac acctattatt ttggaggctg cctggacaac agctctggct   1740
   ctggatgtaa aagccccctg ggagggtttc agggctgcct aaggctcatc accattggtg   1800
   acaaagcggt ggatcccatc ttagtacagc agggggcgct ggggagtttc agggacctcc   1860
   agatagactc ctgcggcatc acagacaggt gcttgcccag ctactgtgag catgggggcg   1920
   agtgttccca gtcgtgggac accttctcct gtgactgtct aggcacaggc tatacgggcg   1980
   agacctgcca ttcctctctc tacgagcagt cttgtgaagc ccacaagcac cgagggaacc 2040
   cgtctgggct ttactatatt gatgcagatg gaagtggccc cctgggacca tttcttgtgt 2100
   actgcaatat gacagcagac gccgcgtgga cggtggtgca gcacggtggc cccgacgcgg 2160
   tgaccctccg aggtgccccc agcgggcacc cgcgctcggc tgtgtccttc gcgtacgcag 2220
   cgggcgcggg gcagctgcgg tccgcggtga acctggcgga gcgctgcgag cagcggctgg 2280
   ctctgcgctg cgggacggcg cggcgcccgg actcacgaga tggaacccca ctgagctggt 2340
   gggttggaag aaccaatgaa acacacactt cctggggagg ttctctgcct gatgctcaaa 2400
   agtgtacttg tggattagag gggaactgca ttgattctca gtattactgc aactgtgatg 2460
   ctggccggaa tgaatggact agtgacacaa tagtcctttc ccaaaaggag cacctgccag 2520
   tcactcagat tgtgatgaca gacgcaggcc gaccacattc cgaagcagct tatacactgg 2580
   ggccactgct ctgccgcgga gatcagtcat tctggaattc agcttccttc aacactgaga 2640
   cttcatacct tcatttccct gctttccacg gagaactcac tgctgacgtg tgcttctttt 2700
   ttaagaccac agtttcctcc ggggtgttta tggagaacct ggggatcaca gatttcatca 2760
   ggattgagct gcgtgctccc acagaagtga ccttttcctt cgatgtgggg aatggacctt 2820
   gtgaggtcac ggtgcagtca cccactccct ttaatgacaa tcagtggcac cacgtgaggg 2880
   cagagagaaa tgttaaagga gcgtctcttc aagttgatca gcttcctcag aagatgcagc 2940
   ctgcccctgc tgatgggcac gttcgtttac agctcaacag ccagctcttc attggtggaa 3000
   cggccaccag acagagaggc tttctaggat gcattcggtc tctgcagttg aacggggtgg 3060
   ccctggatct ggaagaaaga gccacagtga cgccaggagt ggagccaggg tgtgcaggac 3120
   actgcagcac ctatggacac ttgtgtcgca atggagggag atgcagagag aaacgcaggg 3180
   gggtcacctg tgactgtgcc ttctcagcct atgatgggcc gttctgctcc aatgagattt 3240
   ccgcatattt tgcaactggc tcctcaatga cataccattt tcaagaacat tacactttaa 3300
   gtgaaaactc cagctctctc gtttcttcat tacacagaga tgtaacattg accagagaaa 3360
   tgatcacact gagcttccga accacacgaa ctccgagctt attgctgtat gtgagctctt 3420
   tctatgagga atacctttca gttatcctcg ccaacaatgg aagtttgcag attaggtaca 3480
   agctagatag acatcaaaat cctgatgcat ttacctttga ttttaaaaac atggctgatg 3540
   ggcaacttca ccaagtgaag attaacagag aagaagctgt ggtcatggta gaggttaacc 3600
   agagcacaaa gaaacaagtc atcttgtcct cagggacaga attcaacgcc gtcaaatctc 3660
   tcatattggg aaaggtttta gaggctgccg gcgcggaccc ggacacaagg cgggcggcga 3720
   ctagtggctt cactggctgc ctctcggcgg tgcgcttcgg ccgcgctgct cccctgaagg 3780
   cggcgctgcg ccccagcggc ccctcccggg tcaccgtccg cggccacgtg gcccctatgg 3840
   cccgctgcgc agcgggggcg gcgtccggct ccccggcgcg ggaactggct ccccgactcg 3900
   cggggggcgc aggtcgttct ggaccagcgg atgagggaga gcccttggtt aatgcagaca 3960
   gaagagactc tgctgtcatc ggaggtgtga tagcagtggt gatatttatt ttgctttgca 4020
   tcactgccat agccatacgc atctatcaac agagaaagtt acgcaaagaa aatgagtcaa 4080
   aagtctcaaa aaaagaagag tgctaggaca gctctaaaca gtgagctcga tgtgcaaaac 4140
   gcagtccatg aaaaccagaa agagcgagtc ttctgattgg cagctgtggc tgtctctatc 4200
   atcgtgactg tggacttccc tgctgttgcc atcagggtgc acacaagcag gtgcagtgct 4260
   gtcacctggc tgaagacctg cagcctcgga gcctctggga ggtccctttc tccctcggtg 4320
   aaacacagtc ctccacatca atttccaaac aatgaattag gtatggccat tcatcactgt 4380
   tcagtagttt ccccgtccaa aggctctctt ccaaaactgc agtttgatct gtgttaataa 4440
   ttgtggggtt ttagatgaga aaatggctat aaagctgtgg ccctacttta ttttttaaaa 4500
   atgacagaac ttttgttcag atgtaaaaga caaaattgca ctttaatgtt ttttgttact 4560
   tgaaaacata tctgggatcc ctttttttgg tcctctgctg atatttataa aacaagaaat 4620
   gcttcttgga ctaccttcac tggcatttcc atagtcctgg aatccagagc caagtggcct 4680
   atctaaaatt cacagccctt ttattctcct gtgtgatggt taatacaaca cagttgaagc 4740
   ctggaaacac taccattatt tttggtgtat tgctttttct aattgactgt ttttaatgat 4800
   tttgatacat tttaatgttg aaattaatat tgaatgttag ctatgaaatt ttagtattga 4860
   attttataat ggaacagaac attggtaggt aacaagatgc aagaggatgt caatacaaga 4920
   ttgtctgcct gtttttcttt gtaatttgta attacagttt ttgtaacttg tgattatgtt 4980
   tttaactaaa tttaccacca gatacaaaca atacttctta cacagagtta tcctttattt 5040
   atatcattaa gacgtgaatg aaacatcatc ctaacttact tccccaagat attgagaggt 5100
   catatctgtt tttctttatc attcatttct ttttctaaaa gttgttactg atatgctttt 5160
   gatttcctat gactctatta tgttgtacag aacatctttt caatttatta aaaaaatagc 5220
   ttaactgaa 5229
<210> 25
   <211> 1288
   <212> PRT
   <213> Homo sapiens
<400> 25
   Met Ala Ser Val Ala Trp Ala Val Leu Lys Val Leu Leu Leu Leu Pro
   1               5                   10                  15
   Thr Gln Thr Trp Ser Pro Val Gly Ala Gly Asn Pro Pro Asp Cys Asp
               20                  25                  30
   Ala Pro Leu Ala Ser Ala Leu Pro Arg Ser Ser Phe Ser Ser Ser Ser
               35                  40                  45
   Glu Leu Ser Ser Ser His Gly Pro Gly Phe Ser Arg Leu Asn Arg Arg
           50                  55                  60
   Asp Gly Ala Gly Gly Trp Thr Pro Leu Val Ser Asn Lys Tyr Gln Trp
   65                  70                  75                  80
   Leu Gln Ile Asp Leu Gly Glu Arg Met Glu Val Thr Ala Val Ala Thr
                   85                  90                  95
   Gln Gly Gly Tyr Gly Ser Ser Asp Trp Val Thr Ser Tyr Leu Leu Met
               100                 105                 110
   Phe Ser Asp Gly Gly Arg Asn Trp Lys Gln Tyr Arg Arg Glu Glu Ser
           115                 120                 125
   Ile Trp Gly Phe Pro Gly Asn Thr Asn Ala Asp Ser Val Val His Tyr
       130                 135                 140
   Arg Leu Gln Pro Pro Phe Glu Ala Arg Phe Leu Arg Phe Leu Pro Leu
   145                 150                 155                 160
   Ala Trp Asn Pro Arg Gly Arg Ile Gly Met Arg Ile Glu Val Tyr Gly
                   165                 170                 175
   Cys Ala Tyr Lys Ser Glu Val Val Tyr Phe Asp Gly Gln Ser Ala Leu
               180                 185                 190
   Leu Tyr Arg Leu Asp Lys Lys Pro Leu Lys Pro Ile Arg Asp Val Ile
           195                 200                 205
   Ser Leu Lys Phe Lys Ala Met Gln Ser Asn Gly Ile Leu Leu His Arg
       210                 215                 220
   Glu Gly Gln His Gly Asn His Ile Thr Leu Glu Leu Ile Lys Gly Lys
   225                 230                 235                 240
   Leu Val Phe Phe Leu Asn Ser Gly Asn Ala Lys Leu Pro Ser Thr Ile
                   245                 250                 255
   Ala Pro Val Thr Leu Thr Leu Gly Ser Leu Leu Asp Asp Gln His Trp
               260                 265                 270
   His Ser Val Leu Ile Glu Leu Leu Asp Thr Gln Val Asn Phe Thr Val
           275                 280                 285
   Asp Lys His Thr His His Phe Gln Ala Lys Gly Asp Ser Ser Tyr Leu
   290 295 300
   Asp Leu Asn Phe Glu Ile Ser Phe Gly Gly Ile Pro Thr Pro Gly Arg
   305 310 315 320
   Ser Arg Ala Phe Arg Arg Lys Ser Phe His Gly Cys Leu Glu Asn Leu
   325 330 335
   Tyr Tyr Asn Gly Val Asp Val Thr Glu Leu Ala Lys Lys His Lys Pro
   340 345 350
   Gln Ile Leu Met Met Gly Asn Val Ser Phe Ser Cys Pro Gln Pro Gln
   355 360 365
   Thr Val Pro Val Thr Phe Leu Ser Ser Arg Ser Tyr Leu Ala Leu Pro
   370 375 380
   Gly Asn Ser Gly Glu Asp Lys Val Ser Val Thr Phe Gln Phe Arg Thr
   385 390 395 400
   Trp Asn Arg Ala Gly His Leu Leu Phe Gly Glu Leu Arg Arg Gly Ser
   405 410 415
   Gly Ser Phe Val Leu Phe Leu Lys Asp Gly Lys Leu Lys Leu Ser Leu
   420 425 430
   Phe Gln Pro Gly Gln Ser Pro Arg Asn Val Thr Ala Gly Ala Gly Leu
   435 440 445
   Asn Asp Gly Gln Trp His Ser Val Ser Phe Ser Ala Lys Trp Ser His
   450 455 460
   Met Asn Val Val Val Asp Asp Asp Thr Ala Val Gln Pro Leu Val Ala
   465 470 475 480
   Val Leu Ile Asp Ser Gly Asp Thr Tyr Tyr Phe Gly Gly Cys Leu Asp
   485 490 495
   Asn Ser Ser Gly Ser Gly Cys Lys Ser Pro Leu Gly Gly Phe Gln Gly
   500 505 510
   Cys Leu Arg Leu Ile Thr Ile Gly Asp Lys Ala Val Asp Pro Ile Leu
   515 520 525
   Val Gln Gln Gly Ala Leu Gly Ser Phe Arg Asp Leu Gln Ile Asp Ser
   530 535 540
   Cys Gly Ile Thr Asp Arg Cys Leu Pro Ser Tyr Cys Glu His Gly Gly
   545 550 555 560
   Glu Cys Ser Gln Ser Trp Asp Thr Phe Ser Cys Asp Cys Leu Gly Thr
   565 570 575
   Gly Tyr Thr Gly Glu Thr Cys His Ser Ser Leu Tyr Glu Gln Ser Cys
   580 585 590
   Glu Ala His Lys His Arg Gly Asn Pro Ser Gly Leu Tyr Tyr Ile Asp
   595 600 605
   Ala Asp Gly Ser Gly Pro Leu Gly Pro Phe Leu Val Tyr Cys Asn Met
   610 615 620
   Thr Ala Asp Ala Ala Trp Thr Val Val Gln His Gly Gly Pro Asp Ala
   625 630 635 640
   Val Thr Leu Arg Gly Ala Pro Ser Gly His Pro Arg Ser Ala Val Ser
   645 650 655
   Phe Ala Tyr Ala Ala Gly Ala Gly Gln Leu Arg Ser Ala Val Asn Leu
   660 665 670
   Ala Glu Arg Cys Glu Gln Arg Leu Ala Leu Arg Cys Gly Thr Ala Arg
   675 680 685
   Arg Pro Asp Ser Arg Asp Gly Thr Pro Leu Ser Trp Trp Val Gly Arg
   690 695 700
   Thr Asn Glu Thr His Thr Ser Trp Gly Gly Ser Leu Pro Asp Ala Gln
   705 710 715 720
   Lys Cys Thr Cys Gly Leu Glu Gly Asn Cys Ile Asp Ser Gln Tyr Tyr
   725 730 735
   Cys Asn Cys Asp Ala Gly Arg Asn Glu Trp Thr Ser Asp Thr Ile Val
   740 745 750
   Leu Ser Gln Lys Glu His Leu Pro Val Thr Gln Ile Val Met Thr Asp
   755 760 765
   Ala Gly Arg Pro His Ser Glu Ala Ala Tyr Thr Leu Gly Pro Leu Leu
   770 775 780
   Cys Arg Gly Asp Gln Ser Phe Trp Asn Ser Ala Ser Phe Asn Thr Glu
   785                 790                 795                 800
   Thr Ser Tyr Leu His Phe Pro Ala Phe His Gly Glu Leu Thr Ala Asp
                   805                 810                 815
   Val Cys Phe Phe Phe Lys Thr Thr Val Ser Ser Gly Val Phe Met Glu
               820                 825                 830
   Asn Leu Gly Ile Thr Asp Phe Ile Arg Ile Glu Leu Arg Ala Pro Thr
           835                 840                 845
   Glu Val Thr Phe Ser Phe Asp Val Gly Asn Gly Pro Cys Glu Val Thr
       850                 855                 860
   Val Gln Ser Pro Thr Pro Phe Asn Asp Asn Gln Trp His His Val Arg
   865                 870                 875                 880
   Ala Glu Arg Asn Val Lys Gly Ala Ser Leu Gln Val Asp Gln Leu Pro
                   885                 890                 895
   Gln Lys Met Gln Pro Ala Pro Ala Asp Gly His Val Arg Leu Gln Leu
               900                 905                 910
   Asn Ser Gln Leu Phe Ile Gly Gly Thr Ala Thr Arg Gln Arg Gly Phe
           915                 920                 925
   Leu Gly Cys Ile Arg Ser Leu Gln Leu Asn Gly Val Ala Leu Asp Leu
       930                 935                 940
   Glu Glu Arg Ala Thr Val Thr Pro Gly Val Glu Pro Gly Cys Ala Gly
   945                 950                 955                 960
   His Cys Ser Thr Tyr Gly His Leu Cys Arg Asn Gly Gly Arg Cys Arg
                   965                 970                 975
   Glu Lys Arg Arg Gly Val Thr Cys Asp Cys Ala Phe Ser Ala Tyr Asp
               980                 985                 990
   Gly Pro Phe Cys Ser Asn Glu Ile  Ser Ala Tyr Phe Ala  Thr Gly Ser
           995                 1000                 1005
   Ser Met  Thr Tyr His Phe Gln  Glu His Tyr Thr Leu  Ser Glu Asn
       1010                 1015                 1020
   Ser Ser  Ser Leu Val Ser Ser  Leu His Arg Asp Val  Thr Leu Thr
       1025                 1030                 1035
   Arg Glu  Met Ile Thr Leu Ser  Phe Arg Thr Thr Arg  Thr Pro Ser
       1040                 1045                 1050
   Leu Leu  Leu Tyr Val Ser Ser  Phe Tyr Glu Glu Tyr  Leu Ser Val
       1055                 1060                 1065
   Ile Leu  Ala Asn Asn Gly Ser  Leu Gln Ile Arg Tyr  Lys Leu Asp
       1070                 1075                 1080
   Arg His  Gln Asn Pro Asp Ala  Phe Thr Phe Asp Phe  Lys Asn Met
       1085                 1090                 1095
   Ala Asp  Gly Gln Leu His Gln  Val Lys Ile Asn Arg  Glu Glu Ala
       1100                 1105                 1110
   Val Val  Met Val Glu Val Asn  Gln Ser Thr Lys Lys  Gln Val Ile
       1115                 1120                 1125
   Leu Ser  Ser Gly Thr Glu Phe  Asn Ala Val Lys Ser  Leu Ile Leu
       1130                 1135                 1140
   Gly Lys  Val Leu Glu Ala Ala  Gly Ala Asp Pro Asp  Thr Arg Arg
       1145                 1150                 1155
   Ala Ala  Thr Ser Gly Phe Thr  Gly Cys Leu Ser Ala  Val Arg Phe
       1160                 1165                 1170
   Gly Arg  Ala Ala Pro Leu Lys  Ala Ala Leu Arg Pro  Ser Gly Pro
       1175                 1180                 1185
   Ser Arg  Val Thr Val Arg Gly  His Val Ala Pro Met  Ala Arg Cys
       1190                 1195                 1200
   Ala Ala  Gly Ala Ala Ser Gly  Ser Pro Ala Arg Glu  Leu Ala Pro
       1205                 1210                 1215
   Arg Leu  Ala Gly Gly Ala Gly  Arg Ser Gly Pro Ala  Asp Glu Gly
       1220                 1225                 1230
   Glu Pro  Leu Val Asn Ala Asp  Arg Arg Asp Ser Ala  Val Ile Gly
       1235                 1240                 1245
   Gly Val  Ile Ala Val Val Ile  Phe Ile Leu Leu Cys  Ile Thr Ala
       1250                 1255                 1260
   Ile Ala  Ile Arg Ile Tyr Gln  Gln Arg Lys Leu Arg  Lys Glu Asn
       1265                 1270                 1275
   Glu Ser  Lys Val Ser Lys Lys  Glu Glu Cys
       1280                1285
<210> 26
   <211> 6379
   <212> DNA
   <213> Homo sapiens
<400> 26
   agagggacgg aggaagctgc cggtataacg ggaggagagc gccaggcgga gctggggcgt      60
   ccctcccgct cgcttcttga ctcgcgttgg tgccggccgc ctcccgcgcc tagtgtccgg     120
   gacgcgcctg aacctgccgc ctccgtgcct ggggcggcgc cgcgcggccc ccaggctccc     180
   gcggggccga cctcggagac gcagctgggg ccgggcgcgg cttggcggga gggtctgcag     240
   cgccgaggga ggctgccagt gcgtgaggaa gagagctaga gactggacag gggagacaga     300
   gcagcgtcgg agccgcgcag gggacgggag tgagagcggg agtgagagca ggaacgacgc     360
   agagcggccg tcgccgtgcc cgggtctcag ggcgcctggc tgaagtgagc atggcttcag     420
   tggcctgggc cgtcctcaag gtgctgctgc ttctccccac tcagacttgg agccccgtag     480
   gagcaggaaa tccacctgac tgtgattccc cactggcctc tgccttgcct aggtcatcct     540
   tcagcagctc ctcagagctg tccagcagcc acggcccggg gttttcaagg cttaatcgaa     600
   gagatggagc tggtggctgg accccacttg tgtcaaataa ataccaatgg ctgcaaattg     660
   accttggaga gagaatggag gtcactgctg tcgccaccca aggaggatat gggagctctg     720
   actgggtgac cagctacctc ctgatgttca gtgatggtgg gagaaactgg aagcagtatc     780
   gccgagaaga aagcatctgg ggttttccag gaaacacaaa cgcagacagt gtggtgcact     840
   acagactcca gcctcccttt gaagccaggt tcctgcgctt tctcccttta gcctggaacc     900
   ccaggggcag gattgggatg cggatcgaag tgtacggatg tgcatataaa tctgaggtgg     960
   tttattttga tggacaaagt gctctgctgt atacacttga taaaaaacct ttaaaaccaa    1020
   taagagatgt tatttctttg aaatttaaag ccatgcagag caatggaatt ctacttcaca    1080
   gagaaggaca acatggaaat cacattactc tggaattaat taaaggaaag cttgtctttt    1140
   ttcttaattc aggcaatgct aagctgcctt ctactattgc tcctgtgacc ctcaccctgg    1200
   gcagcctgct ggatgaccag cactggcatt ccgtcctcat cgagctcctc gacacgcagg    1260
   tcaacttcac cgtggacaaa cacactcatc atttccaagc aaagggagat tccagtaact    1320
   tggatcttaa ttttgagatc agctttgggg gaattctgtc acccggaaga tcacgggcat    1380
   tcacacgtaa aagctttcat gggtgtttag aaaatcttta ttataatgga gtggatgtta    1440
   ccgaattagc caagaaacac aaaccacaga tcctcatgat gggaaatgtg tccttctcat    1500
   gtccacagcc acagactgtc cctgtgactt ttctgagctc caggagttat ctggctctgc    1560
   caggcaactc tggggaggac aaagtgtctg tcacttttca atttcgaacg tggaacagag 1620
   caggacattt gcttttcggc gaacttcaac gtggttcagg gagtttcgtc ctctttctta 1680
   aggatggcaa gctcaaactg agtctcttcc aggcgggaca gtcaccaagg aatgtcacag 1740
   caggtgctgg attaaacgat gggcagtggc actctgtatc cttctctgcc aagtggagcc 1800
   atatgaatgt ggtggtggat gatgacacag ctgttcagcc cctggtggct gtgctcattg 1860
   attcaggtga cacctattat tttggaggct gcctgggcaa cagctctggc tctggatgta 1920
   aaagccccct gggagggttt cagggctgcc taaggctcat caccattggt gacaaagcgg 1980
   tggatcccat cttagtacag cagggggcgc tggggagttt cagggacctc cagatagact 2040
   cctgcggcat cacagacagg tgcttgccca gctactgtga gcatgggggc gagtgttccc 2100
   agtcgtggga caccttctcc tgtgactgtc taggcacagg ctatacgggc gagacctgcc 2160
   attcctctct ctacgagcag tcttgtgaag cccacaagca ccgagggaac ccatccgggc 2220
   tttactatat tgatgcagat ggaagtggcc ccctgggacc atttcttgtg tactgcaata 2280
   tgacagcaga ctccgcgtgg acggtggtgc ggcacggtgg ccccgacgcg gtgaccctcc 2340
   gaggtgcccc cagcgggcac ccgctctcgg ctgtgtcctt cgcgtacgca gcgggcgcgg 2400
   ggcagctgcg ggccgcggtg aacctggcgg agcgctgcga gcagcggctg gctctgcgct 2460
   gcgggacagc gcggcgcccg gactcacgag atggaacccc actgagctgg tgggttggaa 2520
   gaaccaatga aacacacact tcctggggag gttctctgcc tgatgctcaa aagtgtactt 2580
   gtggattaga ggggaactgc attgattctc agtattactg caactgtgat gctggccaga 2640
   atgaatggac tagtgacaca atagtccttt cccaaaagga gcacctccca gtcactcaga 2700
   ttgtgatgac agacacaggc caaccacatt ccgaagcaga ttatacactg gggccactgc 2760
   tctgccgcgg agataagtca ttttggaatt cagcttcctt caacactgag acttcatacc 2820
   ttcatttccc tgctttccac ggagaactca ctgctgacgt gtgcttcttt tttaagacca 2880
   cagtttcctc tggggtgttt atggagaacc tggggatcac agacttcatc aggattgagc 2940
   tgcgggctcc cacagaagtg acattttcct tcgatgtggg gaatggacct tgtgaggtca 3000
   cggtgcagtc acccactccc tttaatgaca atcagtggca ccacgtgagg gcagagagaa 3060
   atgttaaagg agcgtctctt caagttgatc agcttcctca gaagatgcag cctgcccctg 3120
   ctgatgggca cgttcgttta cagctcaaca gccagctctt cattggtgga acggccacca 3180
   gacagagagg ctttctagga tgcattcggt ctctgcagtt gaacggggtg gccctggatc 3240
   tggaagaaag agccacagtg acgccaggag tggagccagg gtgtgcagga cactgcagca 3300
   cctatggaca cttgtgtcgc aatggaggga gatgcagaga gaaacgcagg ggggtcacct 3360
   gtgactgtgc cttctcagcc tatgatggac cgttctgctc caatgagatt tccgcatatt 3420
   tcgcaactgg ctcctcaatg acataccatt ttcaagaaca ttacacttta agtgaaaact 3480
   ccagctctct cgtttcttca ttacacagag atgtaacatt gaccagagaa atgatcacac 3540
   tgagcttccg aaccacacga actccgagct tattgctgta tgtgagctct ttctatgagg 3600
   aatacctttc agttatcctc gccaacaatg gaagtttgca gattaggtac aagctagata 3660
   gacatcaaaa tcctgatgca tttacctttg attttaaaaa catggctgat gggcaacttc 3720
   accaagtgaa gattaacaga gaagaagccg tggtcatggt agaggttaac cagagcgcaa 3780
   agaaacaagt catcttgtcc tcagggacag aattcaacgc cgtcaaatct ctcatattgg 3840
   gaaaggtttt agaggctgcc ggcgcggacc cggacacaag gcgggcggcg actagtggct 3900
   tcactggctg cctctcggcg gtgcgcttcg gctgcgctgc tcccctgaag gcggcgctgc 3960
   gccccagcgg cccctcccgg gtcaccgtcc gcggccacgt ggcccctatg gcccgctgcg 4020
   cggcgggggc ggcgtccggc tccccggcgc gggaactggc tccccgactc gcggggggcg 4080
   caggtcgttc tggaccagtg gatgagggag agcccttggt taatgcagac agaagagact 4140
   ctgctgtcat cggaggtgtg atagcagtgg agatatttat tttgctttgc atcactgcca 4200
   tagccatacg catctatcaa cagagaaagt tacgcaaaga aaatgagtca aaagtctcaa 4260
   aaaaagaaga gtgctaggac agctctaaac agtgagctcg atgtgcaaaa cgcagtccat 4320
   gaaaaccaga aagagtgagt cttctgattg gcagctgtgg ctgtctctat catcgtgact 4380
   gtggacttcc ctgctgttgc catcagggtg cacacagcag gtgcagtgct gtcacctggc 4440
   tgaagatctg cagcctcaga gcctctggga ggtccctttc tccctcggtg aaacacagtc 4500
   ctccacatca atttccaaac aatgaattag gtatggccat tcatcactgt tcagtagttt 4560
   ccccgtccaa aggctctctt ccaaaactgc agtttgatct gtgttaataa ttgtggggtt 4620
   ttagatgaga aaatgactat aaagctgtgg ccctacttta ttttttaaaa atgacagaac 4680
   ttttgttcag atgtaaaaga caaaattgca ctttaatgtt ttttgttact tgaaaacata 4740
   tctgggatcc ctttttttgg tcctctgctg atattataaa acaagaaatg cttcttggac 4800
   taccttcact ggcatttcca tagtcctgga atccagagcc aagtggccta tctaaaattc 4860
   acagcccttt tattctcctg tgtgatggtt aatacaacac agttgaagcc tggaaacact 4920
   accattattt ttggtgtatt gctttttcta attgactgtt tttaatgatt ttgatacatt 4980
   ttaatgttga aattaatatt gaatgttagc tatgaaattt tagtattgaa ttttataatg 5040
   gaacagaaca ttggtaggta acaagatgca agaggatgtc aatacaagat tgtctgcctg 5100
   tttttctttg taatttgtaa ttacagtttt tgtaacttgt gattatgttt ttaactaaat 5160
   ttaccaccag atacaaacac tacttcttac acagagttat cctttattta tatcattaag 5220
   acgtgaatga aacatcatcc taacttactt ccccaagata ttgagaggtc atatctgttt 5280
   ttcatcattc atttcttttt ctaaaagttg ttactgatat gcttttgatt tcctatgact 5340
   ctattatgtt gtacagaaca tcttttcaat ttattaaaaa aaatagctta actgaagatc     5400
   actaattcct tttctaaatt ttgaactgct ctaggcataa atatcattgt gtattatcct     5460
   ttcatctaat cacttttgga atgtaaactg aaggctgttc actccaagtt aagttaacat     5520
   ttacatgatg tttattaaat ctgccagtct gatcaaaggt ttatgaccat ggggccaaat     5580
   tcccatagat tacccaaaat tgaatatgtg cagttaactg gaacatgtct cattaaggtt     5640
   tacatacaca ctaagacagc aaaggattgg aattaacttt atggagacag ttccagggac     5700
   taatattgaa gcagtggtta tttgataatg catggaacca ccatttggat tcaataaacc     5760
   aattacataa aagcaatgat gctacgctct tcatattcca gataccaact gcctaccaag     5820
   gactaatgag atggtatatt tcttccacat cagggcaagc tgaaaatata ttgattcagc     5880
   gttactgtta tgtaaatgaa tttccatctt ctcatatgtg ttatttttga aagaatgaat     5940
   acactctctg tcttaaatcc gctcttaaca aatgatctct tctcagaata ctacatctgt     6000
   attgagagta cagacagccc attggtcact gcaccttttg caagacagcg aaacggatgc     6060
   atcaaattgc attgatacat catcaccatc aatcttttgc actgattttt agacttaatc     6120
   ttcttatttg aaaataaact catttttaaa taaaataaaa aatattttag ggaaagctct     6180
   ataaaattaa aattacagaa ttatatgctt tttaaaattt atgcaaaaat gtgcagacaa     6240
   atgtattctg tattggttta tgtatcactt tgcccttgta tattttgaga ttattaagat     6300
   ttgttgactt ttttggtttg atatttatca tctgttaaat ttttcctttt aaattattaa     6360
   atgcaaagtg caagaaaaa                                                  6379
<210> 27
   <211> 1288
   <212> PRT
   <213> Homo sapiens
<400> 27
   Met Ala Ser Val Ala Trp Ala Val Leu Lys Val Leu Leu Leu Leu Pro
   1               5                   10                  15
   Thr Gln Thr Trp Ser Pro Val Gly Ala Gly Asn Pro Pro Asp Cys Asp
               20                  25                  30
   Ser Pro Leu Ala Ser Ala Leu Pro Arg Ser Ser Phe Ser Ser Ser Ser
           35                  40                  45
   Glu Leu Ser Ser Ser His Gly Pro Gly Phe Ser Arg Leu Asn Arg Arg
       50                  55                  60
   Asp Gly Ala Gly Gly Trp Thr Pro Leu Val Ser Asn Lys Tyr Gln Trp
   65                  70                  75                  80
   Leu Gln Ile Asp Leu Gly Glu Arg Met Glu Val Thr Ala Val Ala Thr
                   85                  90                  95
   Gln Gly Gly Tyr Gly Ser Ser Asp Trp Val Thr Ser Tyr Leu Leu Met
               100                 105                 110
   Phe Ser Asp Gly Gly Arg Asn Trp Lys Gln Tyr Arg Arg Glu Glu Ser
           115                 120                 125
   Ile Trp Gly Phe Pro Gly Asn Thr Asn Ala Asp Ser Val Val His Tyr
       130                 135                 140
   Arg Leu Gln Pro Pro Phe Glu Ala Arg Phe Leu Arg Phe Leu Pro Leu
   145                 150                 155                 160
   Ala Trp Asn Pro Arg Gly Arg Ile Gly Met Arg Ile Glu Val Tyr Gly
                   165                 170                 175
   Cys Ala Tyr Lys Ser Glu Val Val Tyr Phe Asp Gly Gln Ser Ala Leu
               180                 185                 190
   Leu Tyr Thr Leu Asp Lys Lys Pro Leu Lys Pro Ile Arg Asp Val Ile
           195                 200                 205
   Ser Leu Lys Phe Lys Ala Met Gln Ser Asn Gly Ile Leu Leu His Arg
       210                 215                 220
   Glu Gly Gln His Gly Asn His Ile Thr Leu Glu Leu Ile Lys Gly Lys
   225                 230                 235                 240
   Leu Val Phe Phe Leu Asn Ser Gly Asn Ala Lys Leu Pro Ser Thr Ile
                   245                 250                 255
   Ala Pro Val Thr Leu Thr Leu Gly Ser Leu Leu Asp Asp Gln His Trp
               260                 265                 270
   His Ser Val Leu Ile Glu Leu Leu Asp Thr Gln Val Asn Phe Thr Val
           275                 280                 285
   Asp Lys His Thr His His Phe Gln Ala Lys Gly Asp Ser Ser Asn Leu
       290                 295                 300
   Asp Leu Asn Phe Glu Ile Ser Phe Gly Gly Ile Leu Ser Pro Gly Arg
   305                 310                 315                 320
   Ser Arg Ala Phe Thr Arg Lys Ser Phe His Gly Cys Leu Glu Asn Leu
                   325                 330                 335
   Tyr Tyr Asn Gly Val Asp Val Thr Glu Leu Ala Lys Lys His Lys Pro
               340                 345                 350
   Gln Ile Leu Met Met Gly Asn Val Ser Phe Ser Cys Pro Gln Pro Gln
           355                 360                 365
   Thr Val Pro Val Thr Phe Leu Ser Ser Arg Ser Tyr Leu Ala Leu Pro
       370                 375                 380
   Gly Asn Ser Gly Glu Asp Lys Val Ser Val Thr Phe Gln Phe Arg Thr
   385                 390                 395                 400
   Trp Asn Arg Ala Gly His Leu Leu Phe Gly Glu Leu Gln Arg Gly Ser
                   405                 410                 415
   Gly Ser Phe Val Leu Phe Leu Lys Asp Gly Lys Leu Lys Leu Ser Leu
               420                 425                 430
   Phe Gln Ala Gly Gln Ser Pro Arg Asn Val Thr Ala Gly Ala Gly Leu
           435                 440                 445
   Asn Asp Gly Gln Trp His Ser Val Ser Phe Ser Ala Lys Trp Ser His
       450                 455                 460
   Met Asn Val Val Val Asp Asp Asp Thr Ala Val Gln Pro Leu Val Ala
   465                 470                 475                 480
   Val Leu Ile Asp Ser Gly Asp Thr Tyr Tyr Phe Gly Gly Cys Leu Gly
                   485                 490                 495
   Asn Ser Ser Gly Ser Gly Cys Lys Ser Pro Leu Gly Gly Phe Gln Gly
               500                 505                 510
   Cys Leu Arg Leu Ile Thr Ile Gly Asp Lys Ala Val Asp Pro Ile Leu
           515                 520                 525
   Val Gln Gln Gly Ala Leu Gly Ser Phe Arg Asp Leu Gln Ile Asp Ser
       530                 535                 540
   Cys Gly Ile Thr Asp Arg Cys Leu Pro Ser Tyr Cys Glu His Gly Gly
   545                 550                 555                 560
   Glu Cys Ser Gln Ser Trp Asp Thr Phe Ser Cys Asp Cys Leu Gly Thr
                   565                 570                 575
   Gly Tyr Thr Gly Glu Thr Cys His Ser Ser Leu Tyr Glu Gln Ser Cys
               580                 585                 590
   Glu Ala His Lys His Arg Gly Asn Pro Ser Gly Leu Tyr Tyr Ile Asp
           595                 600                 605
   Ala Asp Gly Ser Gly Pro Leu Gly Pro Phe Leu Val Tyr Cys Asn Met
       610                 615                 620
   Thr Ala Asp Ser Ala Trp Thr Val Val Arg His Gly Gly Pro Asp Ala
   625                 630                 635                 640
   Val Thr Leu Arg Gly Ala Pro Ser Gly His Pro Leu Ser Ala Val Ser
                   645                 650                 655
   Phe Ala Tyr Ala Ala Gly Ala Gly Gln Leu Arg Ala Ala Val Asn Leu
               660                 665                 670
   Ala Glu Arg Cys Glu Gln Arg Leu Ala Leu Arg Cys Gly Thr Ala Arg
           675                 680                 685
   Arg Pro Asp Ser Arg Asp Gly Thr Pro Leu Ser Trp Trp Val Gly Arg
       690                 695                 700
   Thr Asn Glu Thr His Thr Ser Trp Gly Gly Ser Leu Pro Asp Ala Gln
   705                 710                 715                 720
   Lys Cys Thr Cys Gly Leu Glu Gly Asn Cys Ile Asp Ser Gln Tyr Tyr
                   725                 730                 735
   Cys Asn Cys Asp Ala Gly Gln Asn Glu Trp Thr Ser Asp Thr Ile Val
               740                 745                 750
   Leu Ser Gln Lys Glu His Leu Pro Val Thr Gln Ile Val Met Thr Asp
           755                 760                 765
   Thr Gly Gln Pro His Ser Glu Ala Asp Tyr Thr Leu Gly Pro Leu Leu
       770                 775                 780
   Cys Arg Gly Asp Lys Ser Phe Trp Asn Ser Ala Ser Phe Asn Thr Glu
   785                 790                 795                 800
   Thr Ser Tyr Leu His Phe Pro Ala Phe His Gly Glu Leu Thr Ala Asp
                   805                 810                 815
   Val Cys Phe Phe Phe Lys Thr Thr Val Ser Ser Gly Val Phe Met Glu
               820                 825                 830
   Asn Leu Gly Ile Thr Asp Phe Ile Arg Ile Glu Leu Arg Ala Pro Thr
   835 840 845
   Glu Val Thr Phe Ser Phe Asp Val Gly Asn Gly Pro Cys Glu Val Thr
   850 855 860
   Val Gln Ser Pro Thr Pro Phe Asn Asp Asn Gln Trp His His Val Arg
   865 870 875 880
   Ala Glu Arg Asn Val Lys Gly Ala Ser Leu Gln Val Asp Gln Leu Pro
   885 890 895
   Gln Lys Met Gln Pro Ala Pro Ala Asp Gly His Val Arg Leu Gln Leu
   900 905 910
   Asn Ser Gln Leu Phe Ile Gly Gly Thr Ala Thr Arg Gln Arg Gly Phe
   915 920 925
   Leu Gly Cys Ile Arg Ser Leu Gln Leu Asn Gly Val Ala Leu Asp Leu
   930 935 940
   Glu Glu Arg Ala Thr Val Thr Pro Gly Val Glu Pro Gly Cys Ala Gly
   945 950 955 960
   His Cys Ser Thr Tyr Gly His Leu Cys Arg Asn Gly Gly Arg Cys Arg
   965 970 975
   Glu Lys Arg Arg Gly Val Thr Cys Asp Cys Ala Phe Ser Ala Tyr Asp
   980 985 990
   Gly Pro Phe Cys Ser Asn Glu Ile Ser Ala Tyr Phe Ala Thr Gly Ser
   995 1000 1005
   Ser Met Thr Tyr His Phe Gln Glu His Tyr Thr Leu Ser Glu Asn
   1010 1015 1020
   Ser Ser Ser Leu Val Ser Ser Leu His Arg Asp Val Thr Leu Thr
   1025 1030 1035
   Arg Glu Met Ile Thr Leu Ser Phe Arg Thr Thr Arg Thr Pro Ser
   1040 1045 1050
   Leu Leu Leu Tyr Val Ser Ser Phe Tyr Glu Glu Tyr Leu Ser Val
   1055 1060 1065
   Ile Leu Ala Asn Asn Gly Ser Leu Gln Ile Arg Tyr Lys Leu Asp
   1070 1075 1080
   Arg His Gln Asn Pro Asp Ala Phe Thr Phe Asp Phe Lys Asn Met
       1085                1090                1095
   Ala Asp Gly Gln Leu His Gln Val Lys Ile Asn Arg Glu Glu Ala
       1100                1105                1110
   Val Val Met Val Glu Val Asn Gln Ser Ala Lys Lys Gln Val Ile
       1115                1120                1125
   Leu Ser Ser Gly Thr Glu Phe Asn Ala Val Lys Ser Leu Ile Leu
       1130                1135                1140
   Gly Lys Val Leu Glu Ala Ala Gly Ala Asp Pro Asp Thr Arg Arg
       1145                1150                1155
   Ala Ala Thr Ser Gly Phe Thr Gly Cys Leu Ser Ala Val Arg Phe
       1160                1165                1170
   Gly Cys Ala Ala Pro Leu Lys Ala Ala Leu Arg Pro Ser Gly Pro
       1175                1180                1185
   Ser Arg Val Thr Val Arg Gly His Val Ala Pro Met Ala Arg Cys
       1190                1195                1200
   Ala Ala Gly Ala Ala Ser Gly Ser Pro Ala Arg Glu Leu Ala Pro
       1205                1210                1215
   Arg Leu Ala Gly Gly Ala Gly Arg Ser Gly Pro Val Asp Glu Gly
       1220                1225                1230
   Glu Pro Leu Val Asn Ala Asp Arg Arg Asp Ser Ala Val Ile Gly
       1235                1240                1245
   Gly Val Ile Ala Val Glu Ile Phe Ile Leu Leu Cys Ile Thr Ala
       1250                1255                1260
   Ile Ala Ile Arg Ile Tyr Gln Gln Arg Lys Leu Arg Lys Glu Asn
       1265                1270                1275
   Glu Ser Lys Val Ser Lys Lys Glu Glu Cys
       1280                1285
<210> 28
   <211> 3319
   <212> DNA
   <213> Homo sapiens
<400> 28
   ggaatggagg ccaacgtcaa agccagcctg ttccccagag tgcctggcta gcctggagca      60
   agggcccgca gccctgcctg tgtgcagagt ggcgtgcctg tttggatgtg ctggaggagg     120
   aggaggagga agcagagcca ccataccagg gccgtgcggg ctgtgcgggc tgtagggcgt     180
   acacacacac acatacacac acacacacac acacacacag gcacacagat gcaagctgtg     240
   tgaatcaccc agcctgtgag tcagcgccag gattcctgtc ggggaatgag tgagaccagg     300
   ctgaggctgt ctgtccccag accccagagc acgtccggca ccaccatgac tgggctgttg     360
   aagaggaaat ttgaccagct ggatgaggac aactcctcgg tctcctcctc ctcctcttcc     420
   tctgggtgcc agtctcgctc ctgctcccca agctcttctg tctcccgtgc ctgggactca     480
   gaggaggaag gcccctggga tcagatgccc ctgcctgacc gtgacttctg cggccccaga     540
   agtttcaccc ccctgtctat cctgaagcgg gctcgccggg agcgcccagg ccgtgtagcc     600
   tttgatggga tcaccgtctt ctacttcccc cgctgccagg gcttcaccag tgtgcccagc     660
   cgtggtggct gtactctggg tatggccctt cgccacagtg cttgccgtcg cttctctttg     720
   gctgagtttg cgcaggagca agcccgtgca cggcacgaga agctccgcca gcgcttgaaa     780
   gaggagaagt tggagatgct gcagtggaag ctttcggcag ctggggtacc ccaggcagag     840
   gcagggctgc cacctgtggt ggatgccatt gatgacgcct ctgtggagga ggacttggca     900
   gtcgctgtgg caggtggccg gttggaagaa gtgagcttcc tacagcccta cccagcccgg     960
   cgacgtcgag ctctgctgag ggcttcaggt gtgcgaagga tcgatcggga ggagaagcgg    1020
   gagctgcagg cactgcgcca atcccgggag gattgtggct gtcactgcga taggatctgc    1080
   gaccctgaga cctgcagctg cagcctggca ggcatcaagt gccagatgga ccacacagca    1140
   ttcccctgtg gctgctgcag ggagggctgt gagaacccca tgggccgtgt ggaatttaat    1200
   caggcaagag ttcagaccca tttcatccac acactcaccc gcctgcagtt ggaacaggag    1260
   gctgagagct ttagggagct ggaggcccct gcccagggca gcccacccag ccctggtgag    1320
   gaggccctgg tccctacttt cccactggcc aagcccccca tgaacaatga gctgggagac    1380
   aacagctgca gcagcgacat gactgattct tctacagcat cttcatcagc atcgggcact    1440
   agtgaggctc ctgactgccc cacccaccca ggcctgcctg gccctggctt ccagcctggc    1500
   gttgatgatg acagcctggc acgcatcttg agtttcagtg actctgactt cggtggggag    1560
   gaggaggaag aggaggaagg gagcgtgggg aacctggaca acctcagctg cttccatcca    1620
   gctgacatct ttggtactag tgaccctggt ggcctggcca gctggaccca cagctattct    1680
   ggctgtagct tcacatcagg cgtcctggat gagaatgcca acctggatgc cagctgcttc    1740
   ctaaatggtg gccttgaagg gtcaagggaa ggcagccttc ctggcacctc agtgccaccc    1800
   agcatggacg ctggccggag tagctcagtg gatctcagct tgtcttcttg tgactccttt    1860
   gagttactcc aggctctgcc agattatagt ctggggcctc actacacatc acagaaggtg 1920
   tctgacagcc tggacaacat cgaggcacct cacttccccc tgcctggcct gtctccacct 1980
   ggggatgcca gcagttgctt cctggagtcc ctcatgggct tctccgagcc agccgccgaa 2040
   gccctagatc cctttattga cagccagttt gaggacactg tcccagcatc tctaatggag 2100
   cctgtgccgg tgtgaggacc aggatgtctt ttcccagccc caagagacct gttgctgctt 2160
   tcttgtaatt atggggctcc ccagagtctg cgtaacagtc tcccactggc tggctcaccc 2220
   acaggtgcca tgtgcacact cctggttttc aaacaattct ctggatttat ttatttgttt 2280
   taacttttct gtgctgaaga gaggactggg gggagggggc ttcccctttc agctgcccgg 2340
   ccccccacac ccacagcttg ctcttctatc tccacaacgt gagcctggaa gaggagaaaa 2400
   tgtggctcct ctggagcttg gcagaccact tttcggtctt tgcgtgatgt tccttagccc 2460
   aaagacggtg agacagggct gaaatcaggt ggcttctgcc accctgagcc ctagacccat 2520
   gggtggctaa atccactgga ctgtgaagac tataatttat ttccataatt tatttggaga 2580
   ttgaggaggc tttggttgca cttctttggc tggtgggtaa tgccaggggt ggggtgggca 2640
   caggccctca agagcccctt ttgccttgta gtcctacacc ttgccctgcc tgggctttgg 2700
   tgcagactag gtgtggattt gagctctgtg atctatgtct gctgcctggc tcctagatgg 2760
   ctctgtgggc aggtgctggc caaggacatc atctaggcag ggggagagcc tgggctgaac 2820
   agctgtgacc aaaactccct tctgccccac cctgccccct ccacttcctg ccctctgttc 2880
   catcttcccc cttcccaaag gccacagcct ttattccagg cccagggatg taggaggggg 2940
   aaggaggaaa caggaagccc agagagggca aagggcctac ctcggggcgc gaaccatgcc 3000
   ccagactatt atctcagggc tttctgggca ctgcacttca gcgtggccca cctgcccatg 3060
   ccctgaggcc agttggcgag gggtggctcc tgagggtttt tatacccttt gtttgctaat 3120
   gtttaatttt gcatcataat ttctacattg tccctgagtg tcagaactat aatttattcc 3180
   atttctctct gtgtctgtgc caagaaacgc aggctctggg cctgcccctt gcccaggagg 3240
   ccttgccagc ctgtgtgctt gtgggaacac cttgtacctg agcttacagg taccaataaa 3300
   gaggctttat ttttagcaa 3319
<210> 29
   <211> 589
   <212> PRT
   <213> Homo sapiens
<400> 29
   Met Thr Gly Leu Leu Lys Arg Lys Phe Asp Gln Leu Asp Glu Asp Asn
   1               5                   10                  15
   Ser Ser Val Ser Ser Ser Ser Ser Ser Ser Gly Cys Gln Ser Arg Ser
               20                  25                  30
   Cys Ser Pro Ser Ser Ser Val Ser Arg Ala Trp Asp Ser Glu Glu Glu
           35                  40                  45
   Gly Pro Trp Asp Gln Met Pro Leu Pro Asp Arg Asp Phe Cys Gly Pro
       50                  55                  60
   Arg Ser Phe Thr Pro Leu Ser Ile Leu Lys Arg Ala Arg Arg Glu Arg
   65                  70                  75                  80
   Pro Gly Arg Val Ala Phe Asp Gly Ile Thr Val Phe Tyr Phe Pro Arg
                   85                  90                  95
   Cys Gln Gly Phe Thr Ser Val Pro Ser Arg Gly Gly Cys Thr Leu Gly
               100                 105                 110
   Met Ala Leu Arg His Ser Ala Cys Arg Arg Phe Ser Leu Ala Glu Phe
           115                 120                 125
   Ala Gln Glu Gln Ala Arg Ala Arg His Glu Lys Leu Arg Gln Arg Leu
       130                 135                 140
   Lys Glu Glu Lys Leu Glu Met Leu Gln Trp Lys Leu Ser Ala Ala Gly
   145                 150                 155                 160
   Val Pro Gln Ala Glu Ala Gly Leu Pro Pro Val Val Asp Ala Ile Asp
                   165                 170                 175
   Asp Ala Ser Val Glu Glu Asp Leu Ala Val Ala Val Ala Gly Gly Arg
               180                 185                 190
   Leu Glu Glu Val Ser Phe Leu Gln Pro Tyr Pro Ala Arg Arg Arg Arg
           195                 200                 205
   Ala Leu Leu Arg Ala Ser Gly Val Arg Arg Ile Asp Arg Glu Glu Lys
       210                 215                 220
   Arg Glu Leu Gln Ala Leu Arg Gln Ser Arg Glu Asp Cys Gly Cys His
   225                 230                 235                 240
   Cys Asp Arg Ile Cys Asp Pro Glu Thr Cys Ser Cys Ser Leu Ala Gly
                   245                 250                 255
   Ile Lys Cys Gln Met Asp His Thr Ala Phe Pro Cys Gly Cys Cys Arg
               260                 265                 270
   Glu Gly Cys Glu Asn Pro Met Gly Arg Val Glu Phe Asn Gln Ala Arg
   275 280 285
   Val Gln Thr His Phe Ile His Thr Leu Thr Arg Leu Gln Leu Glu Gln
   290 295 300
   Glu Ala Glu Ser Phe Arg Glu Leu Glu Ala Pro Ala Gln Gly Ser Pro
   305 310 315 320
   Pro Ser Pro Gly Glu Glu Ala Leu Val Pro Thr Phe Pro Leu Ala Lys
   325 330 335
   Pro Pro Met Asn Asn Glu Leu Gly Asp Asn Ser Cys Ser Ser Asp Met
   340 345 350
   Thr Asp Ser Ser Thr Ala Ser Ser Ser Ala Ser Gly Thr Ser Glu Ala
   355 360 365
   Pro Asp Cys Pro Thr His Pro Gly Leu Pro Gly Pro Gly Phe Gln Pro
   370 375 380
   Gly Val Asp Asp Asp Ser Leu Ala Arg Ile Leu Ser Phe Ser Asp Ser
   385 390 395 400
   Asp Phe Gly Gly Glu Glu Glu Glu Glu Glu Glu Gly Ser Val Gly Asn
   405 410 415
   Leu Asp Asn Leu Ser Cys Phe His Pro Ala Asp Ile Phe Gly Thr Ser
   420 425 430
   Asp Pro Gly Gly Leu Ala Ser Trp Thr His Ser Tyr Ser Gly Cys Ser
   435 440 445
   Phe Thr Ser Gly Val Leu Asp Glu Asn Ala Asn Leu Asp Ala Ser Cys
   450 455 460
   Phe Leu Asn Gly Gly Leu Glu Gly Ser Arg Glu Gly Ser Leu Pro Gly
   465 470 475 480
   Thr Ser Val Pro Pro Ser Met Asp Ala Gly Arg Ser Ser Ser Val Asp
   485 490 495
   Leu Ser Leu Ser Ser Cys Asp Ser Phe Glu Leu Leu Gln Ala Leu Pro
   500 505 510
   Asp Tyr Ser Leu Gly Pro His Tyr Thr Ser Gln Lys Val Ser Asp Ser
   515 520 525
   Leu Asp Asn Ile Glu Ala Pro His Phe Pro Leu Pro Gly Leu Ser Pro
       530                 535                 540
   Pro Gly Asp Ala Ser Ser Cys Phe Leu Glu Ser Leu Met Gly Phe Ser
   545                 550                 555                 560
   Glu Pro Ala Ala Glu Ala Leu Asp Pro Phe Ile Asp Ser Gln Phe Glu
                   565                 570                 575
   Asp Thr Val Pro Ala Ser Leu Met Glu Pro Val Pro Val
               580                 585
<210> 30
   <211> 569
   <212> DNA
   <213> Homo sapiens
<400> 30
   ttctttacca aatcaatctc ttgcttttta acattttatt tacttttcct tgaagactaa      60
   ttaagaattc aggcaggtga tgaatggttt tcaataccag aagcacagga aggggaaaga     120
   gttccaggaa cgtctgcttc ttgagatccc cacatctgtg aactggagag agaaaggcta     180
   catgagtcct gtgaaggatc aggttaaaac ctgtaggtac aatcccaagt attctgttgc     240
   taatgacact ggctttgtgg acataccttc atgggagaag gacctggcga aggcagtggc     300
   aactgtgggg cccatctctg ttgctgttga tgcaagccat gtctccttcc agttctataa     360
   aaaaagctgg ggtaaaaact ggggcatgga tggctacata aagatggcca aagaccggag     420
   gaacaactgt ggaatcacca cagcagccag ctacccaact gtgtgagctg atggatggtg     480
   atgaggaagg actcgactga ggatggcaca tccagaggag gaactgatct tcaatctacc     540
   agcccctgct gtgtgaaatg cacacttca                                       569
<210> 31
   <211> 1718
   <212> **DNA**
   <213> Homo sapiens
<400> 31
   gagggtgcat aagttctcta gtagggtgat gatataaaaa gccaccggag cactccataa      60
   ggcacaaact ttcagagaca gcagagcaca caagcttcta ggacaagagc caggaagaaa     120
   ccaccggaag gaaccatctc actgtgtgta aacatgactt ccaagctggc cgtggctctc     180
   ttggcagcct tcctgatttc tgcagctctg tgtgaaggtg cagttttgcc aaggagtgct     240
   aaagaactta gatgtcagtg cataaagaca tactccaaac ctttccaccc caaatttatc     300
   aaagaactga gagtgattga gagtggacca cactgcgcca acacagaaat tattgtaaag     360
   ctttctgatg gaagagagct ctgtctggac cccaaggaaa actgggtgca gagggttgtg     420
   gagaagtttt tgaagagggc tgagaattca taaaaaaatt cattctctgt ggtatccaag     480
   aatcagtgaa gatgccagtg aaacttcaag caaatctact tcaacacttc atgtattgtg     540
   tgggtctgtt gtagggttgc cagatgcaat acaagattcc tggttaaatt tgaatttcag     600
   taaacaatga atagtttttc attgtaccat gaaatatcca gaacatactt atatgtaaag     660
   tattatttat ttgaatctac aaaaaacaac aaataatttt taaatataag gattttccta     720
   gatattgcac gggagaatat acaaatagca aaattgaggc caagggccaa gagaatatcc     780
   gaactttaat ttcaggaatt gaatgggttt gctagaatgt gatatttgaa gcatcacata     840
   aaaatgatgg gacaataaat tttgccataa agtcaaattt agctggaaat cctggatttt     900
   tttctgttaa atctggcaac cctagtctgc tagccaggat ccacaagtcc ttgttccact     960
   gtgccttggt ttctccttta tttctaagtg gaaaaagtat tagccaccat cttacctcac    1020
   agtgatgttg tgaggacatg tggaagcact ttaagttttt tcatcataac ataaattatt    1080
   ttcaagtgta acttattaac ctatttatta tttatgtatt tatttaagca tcaaatattt    1140
   gtgcaagaat ttggaaaaat agaagatgaa tcattgattg aatagttata aagatgttat    1200
   agtaaattta ttttatttta gatattaaat gatgttttat tagataaatt tcaatcaggg    1260
   tttttagatt aaacaaacaa acaattgggt acccagttaa attttcattt cagataaaca    1320
   acaaataatt ttttagtata agtacattat tgtttatctg aaattttaat tgaactaaca    1380
   atcctagttt gatactccca gtcttgtcat tgccagctgt gttggtagtg ctgtgttgaa    1440
   ttacggaata atgagttaga actattaaaa cagccaaaac tccacagtca atattagtaa    1500
   tttcttgctg gttgaaactt gtttattatg tacaaataga ttcttataat attatttaaa    1560
   tgactgcatt tttaaataca aggctttata tttttaactt taagatgttt ttatgtgctc    1620
   tccaaatttt ttttactgtt tctgattgta tggaaatata aaagtaaata tgaaacattt    1680
   aaaatataat ttgttgtcaa agtaaaaaaa aaaaaaaa                            1718
<210> 32
   <211> 99
   <212> PRT
   <213> Homo sapiens
<400> 32
   Met Thr Ser Lys Leu Ala Val Ala Leu Leu Ala Ala Phe Leu Ile Ser
   1               5                   10                  15
   Ala Ala Leu Cys Glu Gly Ala Val Leu Pro Arg Ser Ala Lys Glu Leu
               20                  25                  30
   Arg Cys Gln Cys Ile Lys Thr Tyr Ser Lys Pro Phe His Pro Lys Phe
           35                  40                  45
   Ile Lys Glu Leu Arg Val Ile Glu Ser Gly Pro His Cys Ala Asn Thr
       50                  55                  60
   Glu Ile Ile Val Lys Leu Ser Asp Gly Arg Glu Leu Cys Leu Asp Pro
   65                  70                  75                  80
   Lys Glu Asn Trp Val Gln Arg Val Val Glu Lys Phe Leu Lys Arg Ala
                   85                  90                  95
   Glu Asn Ser
<210> 33
   <211> 978
   <212> DNA
   <213> Homo sapiens
<400> 33
   tggctctgac cgcgctggcc tgggccggag agcccaggag gcgtgtctca gagaaaagat        60
   ataagcggcc cccggacgct aaagcggtgc cagcggcgga gtctccaact gggagagctg       120
   cagctgccga gaggaggaga acgctgaggt cggtcggacc aacggacgcg ctgaccgctg       180
   ccaactgcag ctcgcgctgc ctcctgctcg cgccgtgcca ctaaggtcat tcccgcctcc       240
   gagagcccag agccgagatg gaaacggtcc aggagctgat ccccctggcc aaggagatga       300
   tggcccagaa gcgcaagggg aagatggtga agctgtacgt gctgggcagc gtgctggccc       360
   tcttcggcgt ggtgctcggc ctgatggaga ctgtgtgcag ccccttcacg gccgccagac      420
   gtctgcggga ccaggaggca gccgtggcgg agctgcaggc cgccctggag cgacaggctc       480
   tccagaagca agccctgcag gagaaaggca agcagcagga cacggtcctc ggcggccggg       540
   ccctgtccaa ccggcagcac gcctcctagg aactgtggga gaccagcgga gtgggaggga       600
   gacgcagtag acagagacag accgagagag gaatggagag acagaggggg cgcgcgcaca       660
   ggagcctgac tccgctggga gagtgcagga gcacgtgctg ttttttattt ggacttaact       720
   tcagagaaac cgctgacatc tagaactgac ctaccacaag catccaccaa aggagtttgg       780
   gattgagttt tgctgctgtg cagcactgca ttgtcatgac atttccaaca ctgtgtgaat       840
   tatctaaatg cgtctaccat tttgcactag ggaggaagga taaatgcttt ttatgttatt       900
   attattaatt attacaatga ccaccatttt gcattttgaa ataaaaaaac tttttatacc       960
   ataaaaaaaa aaaaaaaa                                                     978
<210> 34
   <211> 103
   <212> PRT
   <213> Homo sapiens
<400> 34
   Met Glu Thr Val Gln Glu Leu Ile Pro Leu Ala Lys Glu Met Met Ala
   1               5                   10                  15
   Gln Lys Arg Lys Gly Lys Met Val Lys Leu Tyr Val Leu Gly Ser Val
               20                  25                  30
   Leu Ala Leu Phe Gly Val Val Leu Gly Leu Met Glu Thr Val Cys Ser
           35                  40                  45
   Pro Phe Thr Ala Ala Arg Arg Leu Arg Asp Gln Glu Ala Ala Val Ala
       50                  55                  60
   Glu Leu Gln Ala Ala Leu Glu Arg Gln Ala Leu Gln Lys Gln Ala Leu
   65                  70                  75                  80
   Gln Glu Lys Gly Lys Gln Gln Asp Thr Val Leu Gly Gly Arg Ala Leu
                   85                  90                  95
   Ser Asn Arg Gln His Ala Ser
               100
<210> 35
   <211> 3436
   <212> DNA
   <213> Homo sapiens
<400> 35
   tcaatgcact gttcaaggat gtttgagaga ttttctgtct tgcctgaata caaaacactg      60
   tagggaacct ctttgtgctt gactttgcac acacacggga tcttcagggt gggtacattt     120
   aaaactgaca agtattgcca aattgccccc caacgtagtt tttacacccc acctactact     180
   ctcagtgaaa aatcaggccc tttttatttt aagggctaaa ttgctaatcc ctgtgtgaaa     240
   atgggtgtta acaaaataaa aaataaggaa aatatgcagg cttacccaat tttgttatct     300
   atacatttta cagtctctaa tacttagcat agctcctttg ggtgtctgcc caccaccctt     360
   ctttctgaac gtggggttcc ctgggctttc cttgtttgac ttcagacctg gatctaatag     420
   ggatctggag gtagaatcaa gacgaatgtt ccctttggcc ttaggtgagt cctttgcatc     480
   tctgaggccc tgttgatgac atccagttct ctagacctct gcccaaacca gagcaatccc     540
   tcctcaaata tgctaagcag aaatgcagac aatataagac acactatatt tatattttag     600
   gagtttacat taccttccct tctgtttgga aaccaacatc tctcaaaatt aggtatagtt     660
   aacacagaat accccaaaat accaccattc ttcataattc catttcctta tttctggatt     720
   ctttcaaggg taaaaacctt gtttcattca tctttggggc agcatttgcc tagtatgtgg     780
   taggcgccca gtagattttg gataaactga cctgattttc tgttaatatt ccaactggat      840
   tatttctttc tcctcttaca gaatgaagat attcaaatgt tattttaaac ataccctaca      900
   gcagaaagtt ttcatcctgt ttttaaccct atggctgctc tctttgttaa agcttctaaa      960
   tgtgagacga ctctttccgc aaaaagacat ttacttggtt gagtactccc taagtacctc     1020
   gccttttgta agaaacagat acactcatgt taaggatgaa gtcaggtatg aagttaactg     1080
   ttcgggtatc tatgaacagg agcctttgga aattggaaag agtctggaaa taagaagaag     1140
   ggacatcatt gacttggagg atgatgatgt tgtggcaatg accagtgatt gtgacattta     1200
   tcagactcta agaggttatg ctcaaaagct tgtctcaaag gaggagaaaa gcttcccaat     1260
   agcctattct ttggttgtcc acaaagatgc aattatggtt gaaaggctta tccatgctat     1320
   atacaaccag cacaatattt actgcatcca ttatgatcgt aaggcacctg ataccttcaa     1380
   agttgccatg aacaatttag ctaagtgctt ctccaatatt ttcattgctt ccaaattaga     1440
   ggctgtggaa tatgcccaca tttccagact ccaggctgat ttaaattgct tgtcggacct     1500
   tctgaagtct tcaatccagt ggaaatatgt tatcaacttg tgtgggcaag attttcccct     1560
   gaagtcaaat tttgaattgg tgtcagagtt gaaaaaactc aatggagcaa atatgttgga     1620
   gacggtgaaa cccccaaaca gtaaattgga aagattcact taccatcatg aacttagacg     1680
   ggtgccttat gaatatgtga agctaccaat aaggacaaac atctccaagg aagcaccccc     1740
   ccataacatt cagatatttg ttggcagtgc ttattttgtt ttaagtcaag catttgttaa     1800
   atatattttc aacaactcca tcgttcaaga cttttttgcc tggtctaaag acacatactc     1860
   tcctgatgag cacttttggg ctaccttgat tcgggttcca ggaatacctg gggagatttc     1920
   cagatcagcc caggatgtgt ctgatctgca gagtaagact cgccttgtca agtggaatta     1980
   ctatgaaggc tttttctatc ccagttgtac tggatctcac cttcgaagcg tgtgtattta     2040
   tggagctgca gaattaaggt ggcttatcaa agatggacat tggtttgcta ataaatttga     2100
   ttctaaggtg gaccctatct tgattaaatg cttggcagaa aagcttgaag aacagcagag     2160
   agactggatc actttgccct cagaaaagtt atttatggat agaaatctca ctaccacatc     2220
   atgatagtaa aatcaggatg gaaataagag ggtgcctgat aaatggagtc agtgtggaat     2280
   tgaataccat actatgccca atactgttta aactcagtcc tcccatattt taaaaggtgt     2340
   ccaaaattcc atacacaagg gaaagtgatc tagcctttga tgttattagc ctgcagttgg     2400
   ctaggttttt ttaatatttg tttttgcttg taatctcact gagccaaatc agagatctta     2460
   aacattcagt cagtcatcaa acattattga gcacctaact atatgacagg cactttttta     2520
   gagactgcgg cttatcctca tcatagcaac ctcggtatct ttaagttctc cacataacag     2580
   tcaggattct actgaagaag cttttgaagt ttgtggtaat cgtctgatca tataaaccac     2640
   ccatttcaga gtagtgttta agtactgtga ccaacactcc acttgtctct taactcagct     2700
   ttcaagacat ttcttaacca tcagagcaga ggaggaaaga ctcactacct cagaaaaatc     2760
   tcaaagaata gtccaatttc ctgcttgcca aagcataatc tgccttttgg tgcattactt     2820
   ggtcaattca gggttgagga gactgttggg gggcatttat aatgtatgaa agttaaggaa     2880
   gggggtgagg atgtgggttg ggacaggtag tacctaaaga ggagcgaagg gatttataca     2940
   acatttttat catgttacaa aacagtatca tgaatggcct cctttttagt tcaactgttt      3000
   ctttaaaatg catttactga ttaaaaataa gaactgacca atagctccaa gtgtcacaca     3060
   ccagaacatt aagcctaagt cctcaattca taagttatca ttctagataa gtttctttac     3120
   aatggaatac tttctgtgga atcattataa ttctgttgta tggaactatc aagctaagag     3180
   tcactaaact ttcttcaaaa ggttgtgtga aatatgacag ctttctaaat taatttgtat     3240
   agtcatttaa aattttttct tctctggcaa ctgtccaact ggaatccaga tttaaagtga     3300
   taaaagctct aagtttcttg cagtcttttt ctcagcttag ttccagagag aaaaaagcta     3360
   attttcctaa ggacacagca agaatattca ttaaggatat tttctaaaac ccacacttga     3420
   gaaaaccacc caatga                                                     3436
<210> 36
   <211> 453
   <212> PRT
   <213> Homo sapiens
<400> 36
   Met Lys Ile Phe Lys Cys Tyr Phe Lys His Thr Leu Gln Gln Lys Val
   1               5                   10                  15
   Phe Ile Leu Phe Leu Thr Leu Trp Leu Leu Ser Leu Leu Lys Leu Leu
               20                  25                  30
   Asn Val Arg Arg Leu Phe Pro Gln Lys Asp Ile Tyr Leu Val Glu Tyr
           35                  40                  45
   Ser Leu Ser Thr Ser Pro Phe Val Arg Asn Arg Tyr Thr His Val Lys
       50                  55                  60
   Asp Glu Val Arg Tyr Glu Val Asn Cys Ser Gly Ile Tyr Glu Gln Glu
   65                  70                  75                  80
   Pro Leu Glu Ile Gly Lys Ser Leu Glu Ile Arg Arg Arg Asp Ile Ile
                   85                  90                  95
   Asp Leu Glu Asp Asp Asp Val Val Ala Met Thr Ser Asp Cys Asp Ile
               100                 105                 110
   Tyr Gln Thr Leu Arg Gly Tyr Ala Gln Lys Leu Val Ser Lys Glu Glu
   115 120 125
   Lys Ser Phe Pro Ile Ala Tyr Ser Leu Val Val His Lys Asp Ala Ile
   130 135 140
   Met Val Glu Arg Leu Ile His Ala Ile Tyr Asn Gln His Asn Ile Tyr
   145 150 155 160
   Cys Ile His Tyr Asp Arg Lys Ala Pro Asp Thr Phe Lys Val Ala Met
   165 170 175
   Asn Asn Leu Ala Lys Cys Phe Ser Asn Ile Phe Ile Ala Ser Lys Leu
   180 185 190
   Glu Ala Val Glu Tyr Ala His Ile Ser Arg Leu Gln Ala Asp Leu Asn
   195 200 205
   Cys Leu Ser Asp Leu Leu Lys Ser Ser Ile Gln Trp Lys Tyr Val Ile
   210 215 220
   Asn Leu Cys Gly Gln Asp Phe Pro Leu Lys Ser Asn Phe Glu Leu Val
   225 230 235 240
   Ser Glu Leu Lys Lys Leu Asn Gly Ala Asn Met Leu Glu Thr Val Lys
   245 250 255
   Pro Pro Asn Ser Lys Leu Glu Arg Phe Thr Tyr His His Glu Leu Arg
   260 265 270
   Arg Val Pro Tyr Glu Tyr Val Lys Leu Pro Ile Arg Thr Asn Ile Ser
   275 280 285
   Lys Glu Ala Pro Pro His Asn Ile Gln Ile Phe Val Gly Ser Ala Tyr
   290 295 300
   Phe Val Leu Ser Gln Ala Phe Val Lys Tyr Ile Phe Asn Asn Ser Ile
   305 310 315 320
   Val Gln Asp Phe Phe Ala Trp Ser Lys Asp Thr Tyr Ser Pro Asp Glu
   325 330 335
   His Phe Trp Ala Thr Leu Ile Arg Val Pro Gly Ile Pro Gly Glu Ile
   340 345 350
   Ser Arg Ser Ala Gln Asp Val Ser Asp Leu Gln Ser Lys Thr Arg Leu
   355 360 365
   Val Lys Trp Asn Tyr Tyr Glu Gly Phe Phe Tyr Pro Ser Cys Thr Gly
       370                 375                 380
   Ser His Leu Arg Ser Val Cys Ile Tyr Gly Ala Ala Glu Leu Arg Trp
   385                 390                 395                 400
   Leu Ile Lys Asp Gly His Trp Phe Ala Asn Lys Phe Asp Ser Lys Val
                   405                 410                 415
   Asp Pro Ile Leu Ile Lys Cys Leu Ala Glu Lys Leu Glu Glu Gln Gln
               420                 425                 430
   Arg Asp Trp Ile Thr Leu Pro Ser Glu Lys Leu Phe Met Asp Arg Asn
           435                 440                 445
   Leu Thr Thr Thr Ser
       450
<210> 37
   <211> 2178
   <212> DNA
   <213> Homo sapiens
<400> 37
   ccgcagcccg cccgagccgc gtcagggcag aggcggggtg gggcgcggtt aaaaggcgca      60
   gcaggtggga gccggggcct tcacccgaaa cccgacgaga gcccgacagc cggcggcgcc     120
   cgagcccgac ctgcctgccc agccggagcg aagggcgccg ccccgcgcag agcccgcgcc     180
   agggccgccg gccgcagagc agttaaaacg tgcaggcacc agaaggcact tcctgtcggt     240
   gaagaagacc tgtctccggt gtcacgggca tcctgtgttt tgcaaacggg gctgacctcc     300
   cttcctgggg agcaggaagg gtcagggaag gaaaagaagt acagaagatc tggctaaaca     360
   atttctgtat ggcgaaagaa aaattctaac ttgtacgccc tcttcatgca tctttaattc     420
   aatttgaata ttccaggcga catcctcact gaccgagcaa agattgacat tcgtatcatc     480
   actgtgcacc attggcttct aggcactcca gtggggtagg agaaggaggt ctgaaaccct     540
   cgcagaggga tcttgccctc attctttggg tctgaaacac tggcagtcgt tggaaacagg     600
   actcagggat aaaccagcgc aatggattgg gggacgctgc acactttcat cgggggtgtc    660
   aacaaacact ccaccagcat cgggaaggtg tggatcacag tcatctttat tttccgagtc     720
   atgatcctcg tggtggctgc ccaggaagtg tggggtgacg agcaagagga cttcgtctgc     780
   aacacactgc aaccgggatg caaaaatgtg tgctatgacc actttttccc ggtgtcccac     840
   atccggctgt gggccctcca gctgatcttc gtctccaccc cagcgctgct ggtggccatg     900
   catgtggcct actacaggca cgaaaccact cgcaagttca ggcgaggaga gaagaggaat     960
   gatttcaaag acatagagga cattaaaaag cagaaggttc ggatagaggg gtcgctgtgg      1020
   tggacgtaca ccagcagcat ctttttccga atcatctttg aagcagcctt tatgtatgtg      1080
   ttttacttcc tttacaatgg gtaccacctg ccctgggtgt tgaaatgtgg gattgacccc      1140
   tgccccaacc ttgttgactg ctttatttct aggccaacag agaagaccgt gtttaccatt      1200
   tttatgattt ctgcgtctgt gatttgcatg ctgcttaacg tggcagagtt gtgctacctg      1260
   ctgctgaaag tgtgttttag gagatcaaag agagcacaga cgcaaaaaaa tcaccccaat      1320
   catgccctaa aggagagtaa gcagaatgaa atgaatgagc tgatttcaga tagtggtcaa      1380
   aatgcaatca caggtttccc aagctaaaca tttcaaggta aaatgtagct gcgtcataag      1440
   gagacttctg tcttctccag aaggcaatac caacctgaaa gttccttctg tagcctgaag      1500
   agtttgtaaa tgactttcat aataaataga cacttgagtt aactttttgt aggatacttg       1560
   ctccattcat acacaacgta atcaaatatg tggtccatct ctgaaaacaa gagactgctt      1620
   gacaaaggag cattgcagtc actttgacag gttcctttta agtggactct ctgacaaagt      1680
   gggtactttc tgaaaattta tataactgtt gttgataagg aacatttatc caggaattga      1740
   tacgtttatt aggaaaagat atttttatag gcttggatgt ttttagttct gactttgaat      1800
   ttatataaag tatttttata atgactggtc ttccttacct ggaaaaacat gcgatgttag      1860
   ttttagaatt acaccacaag tatctaaatt tggaacttac aaagggtcta tcttgtaaat      1920
   attgttttgc attgtctgtt ggcaaatttg tgaactgtca tgatacgctt aaggtggaaa      1980
   gtgttcattg cacaatatat ttttactgct ttctgaatgt agacggaaca gtgtggaagc      2040
   agaaggcttt tttaactcat ccgtttgcca atcattgcaa acaactgaaa tgtggatgtg      2100
   attgcctcaa taaagctcgt ccccattgct taagccttca aaaaaaaaaa aaaaaaaaaa      2160
   aaaaaaaaaa aaaaaaaa                                                    2178
<210> 38
   <211> 261
   <212> **PRT**
   <213> Homo sapiens
<400> 38
<210> 39
   <211> 444
   <212> DNA
   <213> Homo sapiens
<400> 39
   atggacacac tttgctacac actcctgctg ctgaccaccc cttcctgtga gtgctgtggt       60
   cagggacttc ctcagaagtg aaacatcagt tgtctccttt gtgggcttca tcttcttatg      120
   tcttctccac aggggtcttg tcccaggtca ccttgaagga gtctggtcct gtgctggtga      180
   aacccacaga gaccctcacg ctgacctgca ccgtctctgg gttctcactc agcaatgcta      240
   gaatgggtgt gagctggatc cgtcagcccc cagggaaggc cctggagtgg cttgcacaca      300
   ttttttcgaa tgacgaaaaa tcctacagca catctctgaa gagcaggctc accatctcca      360
   aggacacctc caaaagccag gtggtcctta ccatgaccaa catggaccct gtggacacag      420
   ccacatatta ctgtgcacgg atac                                              444
<210> 40
   <211> 119
   <212> PRT
   <213> Homo sapiens
<400> 40
   Met Asp Thr Leu Cys Tyr Thr Leu Leu Leu Leu Thr Thr Pro Ser Trp
   1               5                   10                  15
   Val Leu Ser Gln Val Thr Leu Lys Glu Ser Gly Pro Val Leu Val Lys
               20                  25                  30
   Pro Thr Glu Thr Leu Thr Leu Thr Cys Thr Val Ser Gly Phe Ser Leu
           35                  40                  45
   Ser Asn Ala Arg Met Gly Val Ser Trp Ile Arg Gln Pro Pro Gly Lys
       50                  55                  60
   Ala Leu Glu Trp Leu Ala His Ile Phe Ser Asn Asp Glu Lys Ser Tyr
   65                  70                  75                  80
   Ser Thr Ser Leu Lys Ser Arg Leu Thr Ile Ser Lys Asp Thr Ser Lys
                   85                  90                  95
   Ser Gln Val Val Leu Thr Met Thr Asn Met Asp Pro Val Asp Thr Ala
               100                 105                 110
   Thr Tyr Tyr Cys Ala Arg Ile
           115
<210> 41
   <211> 1201
   <212> **DNA**
   <213> Homo sapiens
<400> 41
   agttagccca gggaggaagg ggacagaatg acagcatggt tatatcaatg gtccaattct        60
   tggttccaac ccttgattgt ccttcgagag caccacccat tccttacaac acatctctcc       120
   gcttcatttt gggagttttg aagttattaa agttattgat ttcttttcct tacaaccaaa       180
   ataatctaaa ttaagattgt ggccaagttt ggattaaaac ctgcctcccg gctccaagac       240
   ccatgttttt ctatcatgct gactggcagc aaagacgtct tcccatggat ttttcagaac       300
   tctgttcttt aaatgtgacc ttagatgcac cagactcatg ccatccaaag gttggaggta       360
   ctgcccagtt ttagcaatga aagtcccacg tcacgggaaa cctctgagtc ctggacaaat       420
   caagatgaca ttttctatgc atatgcaagc atgtcaccag gagcagagca cagagggaca       480
   acacagcttt tgcgcttcca gctggctccc atcaagaagt tagaagggag cctgcaaaca       540
   catttcctgc tatcatctct ccaccccaga atgactttcc ctggtagagc tgggggaggg       600
   gaggcgggca gcaggccccc caggaggccc tgggcaggaa tattaatact ccaacttccc       660
   agcacaagag gagggaggag gagcggccac ggcgcagtcc ggtcctgggg gccctggaag       720
   gtggttgctg agcagcccgt gggaggcacg gatccgccag ctcacggcgg caggggccgc       780
   ccaagtccta atgaaaacac ctagctcctg gcagggcgca gccgacacat cgccctgcct       840
   tctcggccac ctgggagagg atgggcgccg cacgcggata cccaacctgc cgggttgtgc       900
   tcgcagtgac atcgcttttc tcccggcaag tgcatccatc tcgctgggtc tggatagata       960
   acacaatctc tctgcccaca ccccatttgc tgggagacag cctgggcttc tccatttttt      1020
   atgggctcac ttagcttgga ggaaatccag aggtcaaagc cggcgcagac gctccagtca      1080
   ccttgcccgt ccgggcgcgc agccattccg aaacgggctc acccacgggg ccctaaatta      1140
   cggcgattac actgcaattc gcagcgcgat attactttga tacactacct gctgcagctt      1200
   g                                                                      1201
<210> 42
   <211> 4526
   <212> DNA
   <213> Homo sapiens
<400> 42
   ggaagacaac ctaggcaata ccattctgaa caattgctct ttcaagaggc ttagttgtga      60
   gtggtgcagc aagatgagtg gaagcaagct tcttttcttt ttccttttct tccttccttt     120
   cttaatgggg aagatttaaa tgtgttcata gtcctggggg aaggtgcctg gggagagaga     180
   ggttgaagga gaagaaaagc gagagcagtg tttctgcaga aatagaatgg gactgagaac     240
   cagacactga tgggggcttg catttggaaa caaggaaaat aagcaagggt ggagttgtag     300
   taccattttg tattatatgt ctcataaccc tagtatctga actctctgtg atcagacttt     360
   gctgcctgtg gacttttcta cgtattgatt ttagtgtctt gttccttgtg tattatgtga     420
   tttttttaaa gctgtaaact gcttactttc cttggaaatt tgtgggaatt ctttgagtcc     480
   ttagttgaga ttttattcct ccagagagga ttttctttgc cttctgcaaa tcatctcaag     540
   gcagtaccat agtgttaatt cagacaacaa atccatttga aggctggctt gtggtgacaa 600
   ggtctcaggg gagattttcc ccactccaac accaagtcca aaacgtatac atttctttgt 660
   cgtcttttgc atgatgggtt tatttcttgt ctactcttcc cttgagggtg tatcctttgg 720
   gaggaggtac caggtttggg tgaggtctcc taatacactg tctatattaa tggcatttta 780
   ggctttactt cacaaacccc aagcggttgc agctgtcgaa accgaaaccc agggcaaaag 840
   gtggctttgg tgcctgctta cttcgagttc ttgctttcac ttcattttgg cctttgtaga 900
   ttctcttcct tttgtgctag ctcaacgatg tggttttatt tttacattct tacatatttt 960
   atccagggtc ttagtttcag tcgagaaggt agttcaggat atcaaatctg ccctactgct 1020
   agaaaaggaa gcccggtgtg cctgctatcg taactttggt tttgataagc aaacacgagc 1080
   agtttttgac tgctgggttt acagcatagg aaccctgttg ctatgcagct gttgttggaa 1140
   aatagtctgt gggctcagag tttctaaacc aaccgtctat tggatgccca gagaaggaag 1200
   gaaagcgagg gccctggctt gctgattaca aaccatctcc atggtcacag gttgctgggt 1260
   gctcctttta cgatgttttc ccagcagcat gcaggcagca gtagcttcct ttgatcattt 1320
   ttcagtttct gttatttgct agtgagagaa agtttatacc tgcttgtggg gacatgagtg 1380
   aagaaagaga ggggactttg aaacaagaga agagagcaaa ctcaaaaggt ttttcatggc 1440
   agcatgtgac aagatgatgc tgaaaaattt atatggaaat tcaaaggatc aagaatagct 1500
   gaaacatcac tgaagaagaa caaagtgggc tgaactcgct ctactagata tcaagattta 1560
   taagcaacta cagtatttag gacagtgtag tatcggcact gggagagaaa aaaaaaaaaa 1620
   aaaactaaaa ggaaaaagag cccagagact gacttttgtg tatgtggaca gtttgtttat 1680
   gatgaaagta ccactgctat gcactgtggg aaaatgtcct caaagaatga cactagggca 1740
   actgggtatc cccatgtaaa aacaattaac cctaatctca catcacacac aaaaatcaac 1800
   tccatttggg gtatatactt aaatggaagg taaaactatt aatataaagt ttatagagaa 1860
   tattgtttat tgcttttttg gccccgattc ttctctcctc cctgtgccca tgccctttcc 1920
   caggtgactt tgcagttcct ctcataaaag gggcagatta tgtttttcct ccctggactc 1980
   tatgtttggc ttaagtcctt tggtggaagt gacagtgtgc cattctgggc ctaggcctca 2040
   agggaccttg aacatctctg cttgccctct tccacttatg ccatccccat gagaaggata 2100
   tgcctgatcc agcccactga ccttgacagg gagatgagag acacatgggt agagttctct 2160
   ggtgaagctg ccccagccaa acccagcctg gagccaagcc cagccaagat aaagtgagcc 2220
   catcctggac cagctgaccc aggcacctga atgagaacaa aataattttt gttttccatc 2280
   attgagcttt gggtacattt gttaggcagc aataaactaa catcagaagg taatatagga 2340
   aaatatctat atgacttcca gataggcaaa tgtgttttaa gctagtcaca aaagtacctg 2400
   ctcaccataa agaaaaagag tttttatgtt aaaattaaga atcctcattt atcagaaaat 2460
   actgttaaga gagtaaagag gcttgccata aaatggaaga tgatatttgc aaccattcaa 2520
   caaaaaacca gtatcgagaa tattttaaaa attcctacac agaattaaga aaagacagat 2580
   tattttacat tttatataaa agtccatcca ccttttatat gtacctgttg cacataaaaa 2640
   tgagcaaaaa gacttgaaag agtattttgc aaaagagtaa atccacctgg acaataaaca 2700
   tacgataatg ttctgaatcc cagaaaccag ggaaatgtag attaaagtca acaacaaagt 2760
   ggtagcttag accccaagat taaaacagtg tcagtgagaa aataccagtt acagagatag 2820
   tcaggaacct ttaagaaaca agatgctgga tgttacattg aaaaatatgt gctgagctgt 2880
   gctgctctgt aactacctgc cctgttgtga atctgaccca caatgcctgt gagagtggtg 2940
   ttttctctgg aggattcaaa ggaaaggaca gttgccttct tccttgtgtt gctacagggc 3000
   agagagaata ctgcaggagg tgaggggcag aacagagttt ataacctcaa ctgcagtgtg 3060
   aaaatgctgg ttacctccac catgtcttac ggtgtagcag ccatatgttt tgagttgggt 3120
   agatcccaac caccttcagg gataagccct gattgcccta agccattcat cataatccca 3180
   tttccctggc cttggatggc tatataacct acattgattc aatcagactg aggttctgga 3240
   ctcttgctca gtattaaaca catgtatgac ctagaagtgc tgcagccatt ttgctactac 3300
   gaggaaccca gtctgaagac aaattgtgta caagggaagg ggcagagcca agagaattat 3360
   ggaatgaaat ccagtttgat tagatcatgc ctgaaatttt ctaattacta cacctttcag 3420
   ttatatgaac caatgaattt cctttatttt tcaagccagt ttgagttggc tttctgctag 3480
   ttgcaataga agcatctgta gaaattagag caacaggtga tgaggaaaga tgtactgaaa 3540
   tttccgcagg atctggtgat gaggagaagc acaagttata ccaccaaata tggaatggag 3600
   gcttaatagg tttaaaaata atattaaggg ggaagtttga ctgaggaact ctggcaaatg 3660
   ccacttgact gacacatatg atatctagta gcttagtgaa gtttagggat ggttccttga 3720
   gagaatcaac tagaaagcct tttaaaactc ctttgaagag aatataagct cttgaaaaga 3780
   tcccaaagat caatcatttc atcaaacagc tggggaaact gaggcccaaa gaggttatgt 3840
   tttagttagt acttaagatg gggccaggtc ctgggtgtcc agatttctca tgagggttct 3900
   atcaactcat gaaagctcat catcttagat gtgtataatg aaaaactttc attttcatct 3960
   actcacattg aatctcattg actctatttt ttcttcctca cccagctcta aagatctttt 4020
   aggaatttag cagaaaatag gtaactgtca gtcaaattta ccatcacttg ctgatgtggg 4080
   gaattcctgg ggtgccccat gtccagatca gttatgtaag ccctgagtga gattcatctg 4140
   tcacaggctc attgcctcct tagctcctgg tggtttgaag cttcccttgg atttggcatc 4200
   ttgtcctggg ttcttgctcc tactctcctt ggggttcagt tctctctcct ggattttgag 4260
   cacagtggca ccaactccct tattcctggt gcaggtggag ccggctctgg ctgtggtcgt 4320
   ggattctgcc atgatctctc caggtcctac ctgagaattt gatagtcctt gacttttctc 4380
   tttttacccc caacatggct ttgtcaagcc ctgggattct atctgatgtc taccaccttt 4440
   cttgtcttta aaccactctt tcaatcccca cctcattatc ttttgcctgg accattacaa 4500
   tagttactaa aggccctaaa ccaaaa 4526
<210> 43
   <211> 103
   <212> DNA
   <213> Homo sapiens
<400> 43
   gagtcagaat tctcatcagg ctgtgatgct cagttgtgtg tagattgaaa gccctaattt 60
   tacacacaac tgaggatcat agcctgatgg ttcctttttg ttt 103
<210> 44
   <211> 80
   <212> DNA
   <213> Homo sapiens
<400> 44
   ggtacttgaa gagtggttat ccctgctgtg ttcgcttaat ttatgacgaa tcatacaggg 60
   acatccagtt tttcagtatc 80
<210> 45
   <211> 94
   <212> DNA
   <213> Homo sapiens
<400> 45
   actgatatat ttgtcttatt tgagagctga ggagtatttt tatgcaatct gaatgatctc 60
   agctgtctga aaatgtcttc aattttaaag gctt 94
<210> 46
   <211> 1036
   <212> DNA
   <213> Homo sapiens
<400> 46
   cccaaaccca ttccacctta ctaccaaaca accttaacca aaccatttac tcaaataaag      60
   tataggcgat agaaattgta aaccggcgca atagatatag taccgcaagg gaaagatgaa     120
   aaattataac caagcataat atagcaagga ctaacccctg taccttctgc ataatgaatt     180
   aactagaaat aactttgcaa agagaaccaa agctaagacc cccgaaacca gacgagctac     240
   ctaagaacag ctaaaagagc atacccgtct atgtagcaaa atagtgggaa gattcatagg     300
   tagaggcgac aaacctaccg agtctggtga tagctggttg tccaagatag aatcttagtt     360
   caactttaaa tttacccaca gaaccctcca aatccccctg taaatttaac tgttagtcca     420
   aagaggaaca gctctttgga cactaggaaa aaaccttgta aagagagtaa aaaatttaat     480
   acccatagta ggcctaaaag cagccaccaa ttaagaaagc gttcaagctc aacacctatc     540
   atccgaaaaa tcccaaacat atgactgaac tccttgcacc caattggacc aatctattac     600
   cttatagaag aactaacgtt ggtataagta acatgaaagc attctcctcc acataagcct     660
   acatcagacc aaaatattaa actgacaatt aacagcctaa tatctacaac caaccaacaa     720
   gccattatta cccccactgt taacccaaca caggcatgcc cacaaggaaa ggttaaaaaa     780
   agtaaaagga actcggcaaa ccttacccca cctgtttacc aaaaacatca cctctagcat     840
   tatcagtatt agaggcaccc ctgcccggtg acatatgttt aacggccgcg gtaccctaac     900
   cgtgcaaagg tagcataatc acttgttcct taaataggga cttgtatgaa tggctccacg     960
   agggttcagc tgtctcttac tttcaaccag tgaaattgac ctgcccgtga agagactttt    1020
   aagttcagtt ttttaa                                                    1036
<210> 47
   <211> 28
   <212> PRT
   <213> Homo sapiens
<400> 47
   Met Ala Pro Arg Gly Phe Ser Cys Leu Leu Leu Ser Thr Ser Glu Ile
   1               5                   10                  15
   Asp Leu Pro Val Lys Arg Leu Leu Ser Ser Val Phe
               20                  25
<210> 48
   <211> 1019
   <212> DNA
   <213> Homo sapiens
<400> 48
   ggttgagccg ggtagggaaa gcagcctaaa gcccgggaca ggcacacagg cccaggtgtg        60
   taggccacag cagccgcagt cctgaaaggc tgcaacaccc agacctccag gagagaccag       120
   gcccaggatg cctcgcctgt tcttgttcca cctgctagaa ttctgtttac tactgaacca       180
   attttccaga gcagtcgcgg ccaaatggaa ggacgatgtt attaaattat gcggccgcga       240
   attagttcgc gcgcagattg ccatttgcgg catgagcacc tggagcaaaa ggtctctgag       300
   ccaggaagat gctcctcaga cacctagacc agtggcagaa attgtaccat ccttcatcaa       360
   caaagataca gaaactataa ttatcatgtt ggaattcatt gctaatttgc caccggagct       420
   gaaggcagcc ctatctgaga ggcaaccatc attaccagag ctacagcagt atgtacctgc       480
   attaaaggat tccaatctta gctttgaaga atttaagaaa cttattcgca ataggcaaag       540
   tgaagccgca gacagcaatc cttcagaatt aaaatactta ggcttggata ctcattctca       600
   aaaaaagaga cgaccctacg tggcactgtt tgagaaatgt tgcctaattg gttgtaccaa       660
   aaggtctctt gctaaatatt gctgagatga agctaattgt gcacatcttg tctaattttc       720
   acacatagtc ttgatgacat ttcactgatg cttctgtcag gtcccactaa ttattagaat       780
   ataagaaatc tttattaatg tttagatttt tcatttggtg tgtaagaaaa tattctttgt       840
   atgattgtag tttctgtaaa tgacactttc tatgctgaag tctttttgtc tttttattaa       900
   cagtataatt gtgttgattc tttttaatgc tgttaactta aaattacaat aaaacctttg       960
   ccactttaac catacataat aacaatacag tacctaacaa cagtaaaaaa aaaaaaaaa       1019
<210> 49
   <211> 185
   <212> PRT
   <213> Homo sapiens
<400> 49
   Met Pro Arg Leu Phe Leu Phe His Leu Leu Glu Phe Cys Leu Leu Leu
   1               5                   10                  15
   Asn Gln Phe Ser Arg Ala Val Ala Ala Lys Trp Lys Asp Asp Val Ile
               20                  25                  30
   Lys Leu Cys Gly Arg Glu Leu Val Arg Ala Gln Ile Ala Ile Cys Gly
           35                  40                  45
   Met Ser Thr Trp Ser Lys Arg Ser Leu Ser Gln Glu Asp Ala Pro Gln
       50                  55                  60
   Thr Pro Arg Pro Val Ala Glu Ile Val Pro Ser Phe Ile Asn Lys Asp
   65                  70                  75                  80
   Thr Glu Thr Ile Ile Ile Met Leu Glu Phe Ile Ala Asn Leu Pro Pro
                   85                  90                  95
   Glu Leu Lys Ala Ala Leu Ser Glu Arg Gln Pro Ser Leu Pro Glu Leu
               100                 105                 110
   Gln Gln Tyr Val Pro Ala Leu Lys Asp Ser Asn Leu Ser Phe Glu Glu
           115                 120                 125
   Phe Lys Lys Leu Ile Arg Asn Arg Gln Ser Glu Ala Ala Asp Ser Asn
       130                 135                 140
   Pro Ser Glu Leu Lys Tyr Leu Gly Leu Asp Thr His Ser Gln Lys Lys
   145                 150                 155                 160
   Arg Arg Pro Tyr Val Ala Leu Phe Glu Lys Cys Cys Leu Ile Gly Cys
                   165                 170                 175
   Thr Lys Arg Ser Leu Ala Lys Tyr Cys
               180                 185
<210> 50
   <211> 4796
   <212> DNA
   <213> Homo sapiens
<400> 50
   ctgcgcagtc tgaggcgtcc ctcgcgtcgg gtggaagagc ggctgcgcaa caagtgaacc      60
   tatggattaa ggtgtagccg ggcggcccgg aggttgagag tgcaagatta ttctgcgcct     120
   gcgcggcccg ggtaactttg ctggctgctc ctaggctccc ggctcgccgc catcttgtat     180
   tggggtttca ttgttcccgc tgggccgggc ggtttagtgt aattgccgcc ggaggaggag     240
   gcggagtaac ctctggtcag ccgagaaacc ccactatcct gtagccataa ccgcttaaac     300
   gatttgggag gtagtgaagg gcagggagct ggacctggag gcgccgccgc gacagcagca     360
   gccatggagg acgagatgcc caagactcta tacgtcggta acctttccag agatgtgaca     420
   gaagctctaa ttctgcaact ctttagccag attggacctt gtaaaaactg caaaatgatt     480
   atggatacag ctggaaatga tccctattgt tttgtggagt ttcatgagca tcgtcatgca     540
   gctgcagcat tagctgctat gaatggacgg aagataatgg gtaaggaagt caaagtgaat     600
   tgggcaacaa cccctagcag tcaaaagaaa gatacaagca gtagtaccgt tgtcagcaca     660
   cagcgttcac aagatcattt ccatgtcttt gttggtgatc tcagcccaga aattacaact     720
   gaagatataa aagctgcttt tgcaccattt ggaagaatat cagatgcccg agtggtaaaa     780
   gacatggcaa caggaaagtc taagggatat ggctttgtct cctttttcaa caaatgggat     840
   gctgaaaacg ccattcaaca gatgggtggc cagtggcttg gtggaagaca aatcagaact     900
   aactgggcaa cccgaaagcc tcccgctcca aagagtacat atgagtcaaa taccaaacag     960
   ctatcatatg atgaggttgt aaatcagtct agtccaagca actgtactgt atactgtgga    1020
   ggtgttactt ctgggctaac agaacaacta atgcgtcaga ctttttcacc atttggacaa    1080
   ataatggaaa ttcgagtctt tccagataaa ggatattcat ttgttcggtt caattcccat    1140
   gaaagtgcag cacatgcaat tgtttctgtt aatggtacta ccattgaagg tcatgttgtg    1200
   aaatgctatt ggggcaaaga aactcttgat atgataaatc ccgtgcaaca gaatcaaatt    1260
   ggatatcccc aaccttatgg ccagtggggc cagtggtatg gaaatgcaca acaaattggc    1320
   cagtatatgc ctaatggttg gcaagttcct gcatatggaa tgtatggcca ggcatggaac    1380
   cagcaaggat ttaatcagac acagtcttct gcaccatgga tgggaccaaa ttatggagtg    1440
   caaccgcctc aagggcaaaa tggcagcatg ttgcccaatc agccttctgg gtatcgagtg      1500
   gcagggtatg aaacccagtg aataaggact ccagaatcta aagccagtgg cttgaggcta 1560
   cagggagtgt agtaaagccg ttgtttactt aaagatttat caaatcagtc agtgcaaatg 1620
   tcagatacaa tgtatttatt taaaagattc atttttaatc atgaaattac ttatcatcca 1680
   cattgtttta aaaagaaaca agatgctgga tgtctgccaa tttttgcctt cattaccttt 1740
   tttgataaag tttctcagat ccttgtttca aacacaaatg cagggattgc tgccactttt 1800
   taactattaa gaggcagaaa attgcacaat attgaacttt tttccactga agtagtgtgc 1860
   agttctagtt tgcattcctg atatgattta aaacatgtaa tataaagatg ttaaaaaaaa 1920
   aaaccaaaac tgtgcagagt ctagaagttg tttgtcatct tcagcttgtg cacaattctg 1980
   ttttaggtta aaaaaaggca ttgtttgagc tgtcccatct ccactgttat ccctttgggg 2040
   ttttttaata taaattatta gtttacatca tttttgtatc tacatctttt ttcacaaatt 2100
   tgtcttgcct tattaaagtt ctgtaaaata tacttaaatg gaaaaaatga tgttcattta 2160
   gattgaaaac ttttctcaga tggattgata attgcattca tcttgtgttt tatatgagaa 2220
   ggtgcctcaa gaatttcctg ttggatttgt ttaaaaggat ttttatcttt cgtgataaac 2280
   tttgctgtgt accaggaact ataaaaacaa aaacttgtta ctaaagaaaa tatctgaaat 2340
   gtgataagtt cttatgccat gttaatttca tgtgtcaact tcaacattta catgtattat 2400
   ttcattatgt aaaatgtttt agcaatttaa tattttgcac agttagcaaa ctttgtatgt 2460
   catttccttc aaggcatcat gcagagttga catgagattt ataaggtttt aagttgtttg 2520
   catgtgaaaa tcaaatacat actttggtag tctttgaata caaagtcatc tgctcttgtt 2580
   tttcaagaat tttgagacac aaagttgtat gtaaaggaat atattaattt gccgttttct 2640
   aggtagattt gctcaaaaag agtgaatcaa cttaatatgt acaaatgata gctgtgaaac 2700
   tgtagaatat ctttgtgtca ggcttggagt tcattgtgac ctccaaattt tgcctgaagg 2760
   accagctggg caaagcatct tttaaatgtt cagaggccaa aagataaaca aaaaaaaaac 2820
   cttaaaatcc tacctcttta aacagccttc agataagaga atcctcagtg caatcattat 2880
   tttgattcgt ttggtacctg ttttcctgga gttcccgatt ttattatttt ggggtggctc 2940
   caagcattaa gaggtttaat ctttgatggc attgttctag ttttgaaatt tctagtatat 3000
   ttcagagtct cttagaagac ttgtgtggga agtttcactt tgttttcagt gaagatcaca 3060
   aacctccttc ttcctttact caagaggaaa ggtcccagta tacatatttg aatggttgat 3120
   ggttttcaag accttcaggg agctccctgc attttaccta gaaacagaaa aggcccgcaa 3180
   aatcttaagt ttcctggcct gcatttcccg ggtaggggca aatgactcca agctggtctc 3240
   taagccaata cccttataaa ccagagccca ggaaagacag ctcgagtgta taattctctg 3300
   gagctcaatt ctatgcagtt gtgctgatat ttcattaagt cactgtgtat ttttaagtgt 3360
   tgatacatta aaagtcgctt tatggaagat gagtaaattt tttaaatact tggaaatttt 3420
   atttccttgt taacttctac agatcagggc atgcaaccaa aagcagctta aatgaaatat 3480
   tttaaaataa aatatcagga agctattttt agatttcttc tggcttatgt ttctacttta 3540
   ggaccctcat tgttctctta ttaaaaaaaa ttatttcctg tgcatctcat ggactgcagg 3600
   gtaaattatt tgggcataaa taatttaaat agttttcttt cattttgact atctccagta 3660
   ataacagttt ttattatcca gcatattggc ttattgcaca aatcttaaaa tgtacattga 3720
   ctactttctg agaagaaagt ggtatcagta ctcatgatga aaaggttact actgaacaaa 3780
   ttcacatttc aggaacacct ctatctttgg tttaaatctt actcttagtt tttccgtcta 3840
   aaaatcatac tggtattagt atcaggtaag gaaattaaag tttttaaaat ggtttcattc 3900
   tctgcaatat gcaaaattta gattttactt tctggtactg taaagaacct gaagtgattt 3960
   acacttaatg ggtgattaat ccagtattct ttaccctgaa tgtttggata ttaaagttcc 4020
   tttatgtttt ctataacctg tgggatcttc ttgcagtgat tattgtgtgt gagatttttt 4080
   ttctttttgg tctatccata ttgttatatt cactcaggta tttttttttt aatcttattc 4140
   cagaatcagt ggtttatatt gggttactgt ttaacaccaa atggaattgg cattctgcag 4200
   atttaattaa ttatgaaacc agggtctcat tttccttgct gatacttgtt gaaaatgaga 4260
   ttcacattct agtctttatt ttcctcctgt tttgtccctg tgcttgtaca tcttcctttt 4320
   atttgtgtgt tatagttcta ttccatttga gaaggcagtt ggtaagaact agattgcatg 4380
   tacaaagaca ggtttactaa gtgctgtaca gtggtcctga ggttacagtt gaattagaaa 4440
   aacgaaatgt acttacagga aataagaaag caaacctttc aaatgagagt gatgatttct 4500
   ttaaaaaaaa tcagtttttt tctctcaaat aatgttcttt atttcacgaa atcgtcaatc 4560
   ttaagcatga gcagggataa acaactccta gaaggaactc aattcattct tcctggattt 4620
   tctctgttgt taaatcacaa aaatgatagt ccccaatcgt ttctttatag gaggttatta 4680
   catttcatta cagtcactgc attttgactg ttgtgtttag aatttgaatg tacatccaaa 4740
   atgatgagtt tcaatttaag agccttaata aaatgtgtga gtgtgtctca attgaa 4796
<210> 51
   <211> 386
   <212> PRT
   <213> Homo sapiens
<400> 51
<210> 52
   <211> 1001
   <212> DNA
   <213> Homo sapiens
<400> 52
   aatgggggtc agagctgcta tcctttggct agtaaaacaa gattacttgg tgaaattatg        60
   taaatctcct cgaaagaaat aagtttcaga gttgtctaga gaatatcact tggactgttc       120
   tcaggctttc cactacatct attgtaccac tatggtgccc tgaaaagagg ctttctcagg       180
   cttaattcca tggcttagtc tttattccag tatcaaaaag ggggaatccc agccagagct       240
   cccatgaggg aggatagctg catgctaacc acattaatct attatcaagg taactcggtc       300
   atttttgtca ggcagcacag tgctgtgatt tatagcacat tcatctttgg gagtgggaca       360
   agattatcag taaaacctgg taagtaggca atatgtcact aaagtaggag gcttaatgtg       420
   gctactgaga cccactaaac ttactgcagt atttggaagg cccaagtgtc aagaaattaa       480
   tggtttatgc agacttaagt ggattccaat gaaggaagaa atgttaaagt aatggcacag       540
   aggatagaag agctagctgt gaaaaaaaat agccatgtgg atgaaccaaa cgcaacagac       600
   tgaaagagcc tagagagttg atcctaaaga aaaaggcaga aagtctggct gagcacttcc       660
   agttctgctg acctggcttg attagtgaat ctgggccaca gtatctttat ctataaaatg       720
   aaaacattta atatcacctc agattttttg ttttaatttt gtgggtacat aataggtgta       780
   tatatttatt ttaaagataa gctaatatat gtgaaagtat tttgtaaatt cttaaatgct       840
   atccacatgt aaaatattat ttgtttatag aacaaaaaaa aactatgtca gatttagaat       900
   tcttttaaca tgagaatttg taggttctta gtgaaatagc catttacatg tgatctgtag       960
   ccctaggcat tggtaaaaag cactttctct tctactgaaa t                          1001
<210> 53
   <211> 17
   <212> PRT
   <213> Homo sapiens
<400> 53
   Ile Tyr Ser Thr Phe Ile Phe Gly Ser Gly Thr Arg Leu Ser Val Lys
   1               5                   10                  15
   Pro
<210> 54
   <211> 1001
   <212> DNA
   <213> Homo sapiens
<400> 54
   ggtttcaccg ttttagccgg gatggtctcg atctcctgac ctcgtgatcc gcccgcctcg      60
   gcctcccaaa gtgctgggat tacaggcgtg agccaccgcg cccggcctca tgatttttaa     120
   tagttagatt ttatgcaaaa tttcatttga gggtaaactt aaaggtgaac aagtttaatg     180
   gactttggcc atggcagaga aatggttagg tttggatata aggtggacct ctctaattat     240
   aagaaaaaaa aggaacatga gaattggtga acaagacctt ccctggagat cttcagaaac     300
   ctgaggtcac tcaggagaga aatgtgtgaa gttgagagct tacagtaaat ggtctctgaa     360
   gtttcccacc attccaaaga gtgcgtgagc ttaaaatttt tcgtgagttt agctaaatat     420
   gtgcttagtg ttagattagg tttcaaccaa gcaaaagaac cctgggagaa gtactctgct     480
   tggaaatgaa gcatcctttg gtttttgtgg tacaatagat cactgtgggt tttcagatgg     540
   ccagaagctg ctctttgcaa gggggaccat gttaaaggtg gatcttagta agtattatta     600
   ctaatgaatt cttaattgat tagtttttga gacaagtaat aagctttttt ggaaatctaa     660
   gtggactgcc aaatgttctc gatttatcca tattgcatgg ggagcagagc attcttcatg     720
   ccttctccat cctcacttct gccaggtagc agtttgcatt gttaacaggg aagcagaatg     780
   gaagcttaac agagaagcag tatggaagac gtctccttag cccagtggtc agttgcatca     840
   gagaagtctg ctctgaaatg cgaacacttt ctcttgcagc tggcagcccc ctgcaacctc     900
   agtgccctcc atggccaggg ccaaagcttc caggtgccca gataacatcc caaagtcact     960
   ctgagagaag agagaaaatc ggtcttactt taccttttct a                        1001
<210> 55
   <211> 19
   <212> PRT
   <213> Homo sapiens
<400> 55
   Phe Ser Asp Gly Gln Lys Leu Leu Phe Ala Arg Gly Thr Met Leu Lys
   1               5                   10                  15
   Val Asp Leu
<210> 56
   <211> 1001
   <212> DNA
   <213> Homo sapiens
<400> 56
   tgccaaccag agaagctggg ccctttgtag cacaagcctc aggagcagag aatggaggga      60
   cattctcaac tgagaggaga gggactagga ggctagatgg gaaatgaggt gactctacag     120
   aaaatcgaat atgggatggt ataaaaggat gagaaagatg gcagggggtc cacgatcgct     180
   cttgtattta gacctggggg atgcactgat agataccacc tggagagaga aagaggattt     240
   ctgtttccct ctgaagaaaa atgtggcaga aaaatcaaaa cagatcgcac atgtcaagcc     300
   tctcctaatt acagcctttc attctggttt ctctcagctg ttccagcagg aaatggtagg     360
   aaagggtcat tggtgccttt gatgttgtgg aagcattgca ggaaggagat ttgcaagaaa     420
   cggggcaggg gcttgcagta gagtctggag agtttagaat gatggttttt gcaatgactt     480
   agaacactgt gtatctaact ttggaaatga gaaattaacc tttgggactg gaacaagact     540
   caccatcata cccagtaagt tcttcatcct tggtcaggaa atcagcctgc ataagattct     600
   ggggatcata gtagagatgc aggcttagag aattttcatc tgtccttgtt cataactggt     660
   agagacaggt ggcagccaaa tatctgagtt ttccggggga actccagtcc tgactattta     720
   attcaaaact agaaaatatg attcacaatt ctgtttttgc tgctgccgtc aagtgctaat     780
   gtggcatgtg aagagaattc aggcagaatg actgcatctt cactttcatc ttgtgtgata     840
   attttagctt tgagtactgc aagtgtttgg gagaaatggt agaaacatag aatatcaaag     900
   agacaagcaa accaagagac ccagagaggc aggcagacgg aacgtcaggc agtataattt     960
   tacaaaacta attcagtgtt gtgtctctct ctgcaaatgg c                        1001
<210> 57
   <211> 20
   <212> PRT
   <213> Homo sapiens
<400> 57
   Ser Asn Phe Gly Asn Glu Lys Leu Thr Phe Gly Thr Gly Thr Arg Leu
   1               5                   10                  15
   Thr Ile Ile Pro
               20
<210> 58
   <211> 443
   <212> DNA
   <213> Homo sapiens
<400> 58
   atgaagccca ccctcatctc agtgcttgtg ataatattta tactcagtaa gtaaagcctc 60
   tttttgttct atttttctat ggtattctga tttagaataa ctttaatgaa tattatcccc 120
   agtctgtaag acttttcctg atccagaatt ggtgttatag gaggaacaag agcccagaga 180
   gtgactcagc ccgagaagct cctctctgtc tttaaagggg ccccagtgga gctgaagtgc 240
   aactattcct attctgggag tcctgaactc ttctggtatg tccagtactc cagacaacgc 300
   ctccagttac tcttgagaca catctctaga gagagcatca aaggcttcac tgctgacctt 360
   aacaaaggcg agacatcttt ccacctgaag aaaccatttg ctcaagagga agactcagcc 420
   atgtattact gtgctctaag tgg 443
<210> 59
   <211> 109
   <212> PRT
   <213> Homo sapiens
<400> 59
   Met Lys Pro Thr Leu Ile Ser Val Leu Val Ile Ile Phe Ile Leu Arg
   1 5 10 15
   Gly Thr Arg Ala Gln Arg Val Thr Gln Pro Glu Lys Leu Leu Ser Val
   20 25 30
   Phe Lys Gly Ala Pro Val Glu Leu Lys Cys Asn Tyr Ser Tyr Ser Gly
   35 40 45
   Ser Pro Glu Leu Phe Trp Tyr Val Gln Tyr Ser Arg Gln Arg Leu Gln
   50 55 60
   Leu Leu Leu Arg His Ile Ser Arg Glu Ser Ile Lys Gly Phe Thr Ala
   65 70 75 80
   Asp Leu Asn Lys Gly Glu Thr Ser Phe His Leu Lys Lys Pro Phe Ala
   85 90 95
   Gln Glu Glu Asp Ser Ala Met Tyr Tyr Cys Ala Leu Ser
   100 105
<210> 60
   <211> 460
   <212> DNA
   <213> Homo sapiens
<400> 60
   atgggctgca ggctcctctg ctgtgtggtc ttctgcctcc tccaagcagg tgagtcccgg        60
   gcccaggtga catgatccta ttggagtccc taagcctttt caccatgaca acaacagcag       120
   gccgtctcct aggatttgcc tgaattctgc ttctttcctt tgcaggtccc ttggacacag       180
   ctgtttccca gactccaaaa tacctggtca cacagatggg aaacgacaag tccattaaat       240
   gtgaacaaaa tctgggccat gatactatgt attggtataa acaggactct aagaaatttc       300
   tgaagataat gtttagctac aataataagg agctcattat aaatgaaaca gttccaaatc       360
   gcttctcacc taaatctcca gacaaagctc acttaaatct tcacatcaat tccctggagc       420
   ttggtgactc tgctgtgtat ttctgtgcca gcagccaaga                             460
<210> 61
   <211> 114
   <212> PRT
   <213> Homo sapiens
<400> 61
   Met Gly Cys Arg Leu Leu Cys Cys Val Val Phe Cys Leu Leu Gln Ala
   1               5                   10                  15
   Gly Pro Leu Asp Thr Ala Val Ser Gln Thr Pro Lys Tyr Leu Val Thr
               20                  25                  30
   Gln Met Gly Asn Asp Lys Ser Ile Lys Cys Glu Gln Asn Leu Gly His
           35                  40                  45
   Asp Thr Met Tyr Trp Tyr Lys Gln Asp Ser Lys Lys Phe Leu Lys Ile
       50                  55                  60
   Met Phe Ser Tyr Asn Asn Lys Glu Leu Ile Ile Asn Glu Thr Val Pro
   65                  70                  75                  80
   Asn Arg Phe Ser Pro Lys Ser Pro Asp Lys Ala His Leu Asn Leu His
                   85                  90                  95
   Ile Asn Ser Leu Glu Leu Gly Asp Ser Ala Val Tyr Phe Cys Ala Ser
               100                 105                 110
   Ser Gln